# EUROPÄISCHE PATENTSCHRIFT

(11) **EP 2 621 508 B1**
(45) Veröffentlichungstag und Bekanntmachung des Hinweises auf die Patenterteilung: **26.08.2015**
(21) Anmeldenummer: 11760500.6
(22) Anmeldetag: 26.09.2011
(51) Int. Cl.: A61K 38/08, C07K 1/107, C07K 7/06

(54) **N-CARBOXYALKYL-AURISTATINE UND IHRE VERWENDUNG**
N-CARBOXYALKYL AURISTATINS AND THE USE THEREOF
N-CARBOXYALKYL-AURISTATINES ET LEUR UTILISATION

(30) Priorität: 16.03.2011 EP 11158466; 29.09.2010 EP 10181966
(43) Veröffentlichungstag der Anmeldung: 07.08.2013
(73) Patentinhaber: Seattle Genetics, Inc., Bothell, WA 98021 (US)
(72) Erfinder: LERCHEN, Hans-Georg, 51375 Leverkusen (DE); EL SHEIKH, Sherif, 45219 Essen (DE); STELTE-LUDWIG, Beatrix, 42489 Wülfrath (DE); GOLFIER, Sven, 12247 Berlin (DE); SCHUHMACHER, Joachim, 42113 Wuppertal (DE); GNOTH, Mark, Jean, 40822 Mettmann (DE); KRENZ, Ursula, 42799 Leichlingen (DE)
(74) Vertreter: Hoffmann Eitle
(86) Internationale Anmeldenummer: PCT/EP2011/066658
(87) Internationale Veröffentlichungsnummer: WO 2012/041805

(56) Entgegenhaltungen:
- WO-A2-2007/008848
- WO-A2-2008/052187
- SVETLANA O. DORONINA ET AL: "Enhanced Activity of Monomethylauristatin F through Monoclonal Antibody Delivery: Effects of Linker Technology on Efficacy and Toxicity", BIOCONJUGATE CHEMISTRY, Bd. 17, Nr. 1, 1. Januar 2006 (2006-01-01) , Seiten 114-124, XP55009264, ISSN: 1043-1802, DOI: 10.1021/bc0502917
- KRISHNA MOHAN BAJJURI? ET AL: "The Legumain Protease-Activated Auristatin Prodrugs Suppress Tumor Growth and Metastasis without Toxicity", CHEMMEDCHEM, Bd. 6, Nr. 1, 3. Januar 2011 (2011-01-03), Seiten 54-59, XP55009266, ISSN: 1860-7179, DOI: 10.1002/cmdc.201000478

## Beschreibung

Die vorliegende Anmeldung betrifft neue, am N-Terminus mit einer Carboxyalkyl-Gruppe substituierte Derivate von Monomethylauristatin F, Verfahren zur Herstellung dieser Derivate, die Verwendung dieser Derivate zur Behandlung und/oder Prävention von Krankheiten sowie die Verwendung dieser Derivate zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Die meisten der heutzutage parenteral applizierten Chemotherapeutika sind oft nicht zielgerichtet auf das Tumorgewebe oder die Tumorzellen, sondern durch die systemische Gabe unspezifisch im Körper verteilt, d.h. auch an Orten, an denen eine Wirkstoffexposition unerwünscht ist, wie beispielsweise in gesunden Zellen, Geweben und Organen. Dies kann zu unerwünschten Nebenwirkungen bis hin zu gravierenden allgemein-toxischen Effekten führen, die dann den therapeutisch nutzbaren Dosisbereich des Wirkstoffs oftmals stark limitieren oder ein völliges Absetzen der Medikation erfordern.

Die verbesserte und selektive Verfügbarkeit dieser Chemotherapeutika in der Tumorzelle oder dem direkt umgebenden Gewebe und die damit verbundene Wirksteigerung einerseits und Minimierung toxischer Nebeneffekte andererseits steht daher seit mehreren Jahren im Fokus bei der Entwicklung neuer Chemotherapeutika. Viele Versuche wurden bislang unternommen, effiziente Methoden für die Wirkstoffeinbringung in die Zielzelle zu entwickeln. Die Optimierung der Assoziation zwischen Wirkstoff und intrazellulärem Ziel und die Minimierung der interzellulären Wirkstoffverteilung, beispielsweise zu Nachbarzellen, stellen jedoch nach wie vor eine schwierige Aufgabe dar.

Für die zielgerichtete Adressierung von Tumorgewebe und Tumorzellen sind beispielsweise monoklonale Antikörper geeignet. Die Bedeutung solcher Antikörper für die klinische Behandlung von Krebserkrankungen hat allgemein in den letzten Jahren erheblich zugenommen, basierend auf der Wirksamkeit solcher Agentien wie Trastuzumab (Herceptin), Rituximab (Rituxan), Cetuximab (Erbitux) und Bevacizumab (Avastin), welche inzwischen für die Therapie einzelner, spezifischer Tumorerkrankungen zugelassen sind [siehe z.B. G. P. Adams und L. M. Weiner, Nat. Biotechnol. 23, 1147-1157 (2005)]. In Folge hiervon ist auch das Interesse an so genannten Immunokonjugaten deutlich gestiegen, in welchen ein internalisierender, gegen ein Tumor-assoziiertes Antigen gerichteter Antikörper auf kovalente Weise über eine Verknüpfungseinheit ("linker") mit einem cytotoxisch wirkenden Agens verbunden ist, das nach Einschleusung und anschließender Spaltung des Konjugats innerhalb der Tumorzelle freigesetzt wird und dort seine Wirkung direkt und selektiv entfalten kann. Auf diesem Wege könnte die Schädigung von normalem Gewebe im Vergleich zu einer konventionellen Chemotherapie der Krebserkrankung in signifikant engeren Grenzen gehalten werden [siehe z.B. J. M. Lambert, Curr. Opin. Pharmacol. 5 543-549 (2005); A. M. Wu und P. D. Senter, Nat. Biotechnol. 23, 1137-1146 (2005); P. D. Senter, Curr. Opin. Chem. Biol. 13, 235-244 (2009); L. Ducry und B. Stump, Bioconjugate Chem. 21, 5-13 (2010)].

Statt Antikörpern können auch Liganden aus dem Wirkstoffbereich kleiner Moleküle verwendet werden, die selektiv an einen speziellen Zielort ("target"), wie beispielsweise an einen Rezeptor, binden [siehe z.B. E. Ruoslahti et al., Science 279, 377-380 (1998); D. Karkan et al., PLoS ONE 3 (6), e2469 (June 25, 2008)]. Bekannt sind auch Konjugate aus cytotoxischem Wirkstoff und adressierendem Ligand, die zwischen Ligand und Wirkstoff eine definierte Spaltstelle zur Freisetzung des Wirkstoffs aufweisen. Eine solche "Soll-Bruchstelle" kann beispielsweise in einer Peptidkette bestehen, die an einer bestimmten Stelle selektiv durch ein spezielles Enzym am Wirkort gespalten werden kann [siehe z.B. R. A. Firestone und L. A. Telan, US Patent Application US 2002/0147138]:

Auristatin E (AE) und Monomethylauristatin E (MMAE) sind synthetische Analoga der Dolastatine, einer speziellen Gruppe von linearen Pseudopeptiden, welche ursprünglich aus marinen Quellen isoliert wurden und die zum Teil sehr potente cytotoxische Aktivität gegenüber Tumorzellen aufweisen [für eine Übersicht siehe z.B. G. R. Pettit, Prog. Chem. Org. Nat. Prod. 70, 1-79 (1997); G. R. Pettit et al., Anti-Cancer Drug Design 10, 529-544 (1995); G. R. Pettit et al., Anti-Cancer Drug Design 13, 243-277 (1998)].

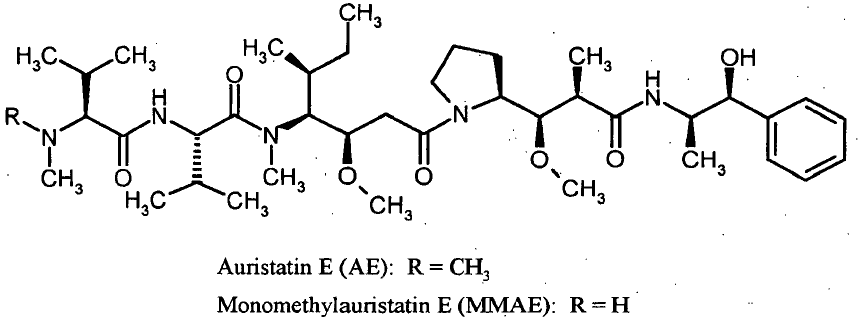

Allerdings besitzt MMAE den Nachteil einer vergleichsweise hohen systemischen Toxizität. Zudem ist diese Verbindung bei einer Anwendung in Form von Antikörper-Wirkstoff-Konjugaten (Immunokonjugaten) nicht kompatibel mit Verknüpfungseinheiten (Linkern) zwischen Antikörper und Wirkstoff, welche keine enzymatisch spaltbare Soll-Bruchstelle aufweisen [S. O. Doronina et al., Bioconjugate Chem. 17, 114-124 (2006)].

Monomethylauristatin F (MMAF) ist ein Auristatin-Derivat mit einer C-terminalen Phenylalanin-Einheit, das nur moderate anti-proliferative Wirkung im Vergleich zu MMAE aufweist. Dies ist sehr wahrscheinlich auf die freie Carboxylgruppe zurückzuführen, die aufgrund ihrer Polarität und Ladung die Zellgängigkeit dieser Verbindung negativ beeinflusst. In diesem Zusammenhang wurde der Methylester von MMAF (MMAF-OMe) als ein neutral geladenes, zellgängiges Prodrug-Derivat beschrieben, das im Vergleich zu MMAF eine um mehrere Größenordnungen *erhöhte in vitro-Cyto-*toxizität gegenüber verschiedenen Karzinoma-Zelllinien zeigt [S. O. Doronina et al., Bioconjugate --Chem. 17, 114-124- (2006)]. Es ist anzunehmen, dass diese Wirkung durch MMAF selbst hervorgerufen wird, das nach Aufnahme des Prodrugs in die Zellen schnell durch intrazelluläre Ester-Hydrolyse freigesetzt wird.

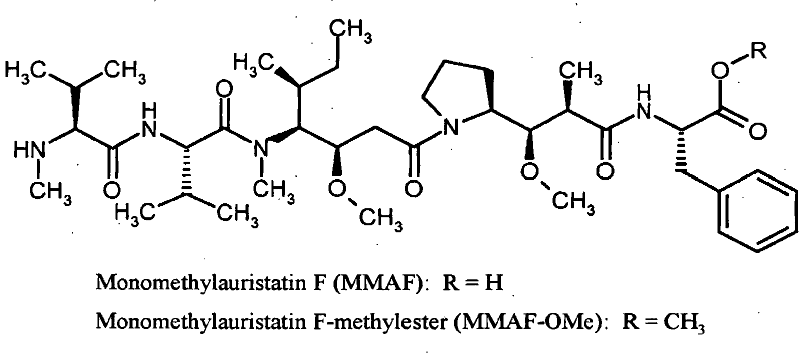

Wirkstoff Verbindungen auf Basis von einfachen Ester-Derivaten können generell jedoch dem Risiko einer chemischen Instabilität infolge einer ünspezifischen, vom vorgesehenen Wirkort unabhängigen Ester-Hydrolyse unterliegen, beispielsweise durch im Blutplasma vorhandene Esterasen; dies kann die Anwendbarkeit solcher Verbindungen in der Therapie deutlich einschränken. Darüber hinaus sind Auristatin-Derivate wie MMAE und MMAF auch Substrate für Transporterproteine, welche von vielen Tumorzellen exprimiert werden, was zu einer Resistenzentwicklung gegenüber diesen Wirkstoffen führen kann.

Aufgabe der vorliegenden Erfindung war es daher, ausgehend vom nur moderat wirksamen Mono-methylauristatin F (MMAF) neue Verbindungen zu identifizieren und für die Behandlung insbe-sondere von Krebserkrankungen bereitzustellen, die einerseits eine deutlich stärkere cytotoxische Aktivität in Ganzzell-Assays aufweisen und andererseits schwächer ausgeprägte Substrat-Eigen-schaften für Transporterproteine zeigen. Solche Substanzen könnten sich in besonderem Maße als Toxophore für die Verknüpfung mit Proteinen, wie beispielsweise Antikörpern, oder auch mit niedermolekularen Liganden zu anti-proliferativ wirkenden (Immuno-)Konjugaten eignen.

Monomethylauristatin F (MMAF) sowie verschiedene Ester- und Amid-Derivate hiervon wurden in WO 2005/081711-A2 offenbart. Weitere Auristatin-Analoga mit einer C-terminalen, amidisch substituierten Phenylalanin-Einheit sind in WO 01/18032-A2 beschrieben. In WO 02/088172-A2 und WO 2007/008603-A1 werden MMAF-Analoga beansprucht, welche Seitenketten-Modifikationen des Phenylalanins betreffen, und in WO 2007/008848-A2 solche, in denen die . Carboxylgruppe des Phenylalanins modifiziert ist. Weitere über den N- oder C-Terminus verknüpfte Auristatin-Konjugate sind unter anderem in WO 2004/010957-A2 und WO 2009/117531-A1 beschrieben [siehe auch S. O. Doronina et al., Bioconjugate Chem. 19, 1960-1963 (2008)].

Gegenstand der vorliegenden Erfindung sind nun Verbindungen der allgemeinen Formel (I) 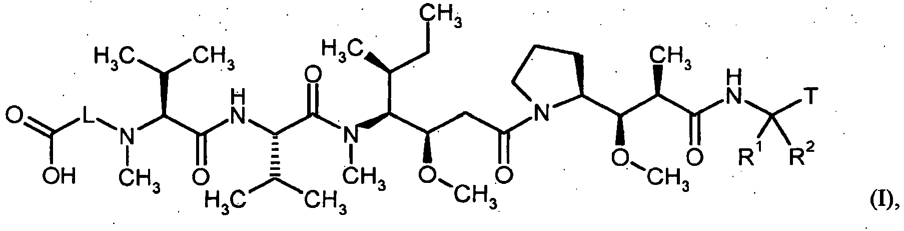 in welcher
- L: für geradkettiges (C₁-C₁₂)-Alkandiyl steht, das bis zu vierfach mit Methyl substituiert sein kann und in dem (a) zwei Kohlenstoffatome in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder (b) bis zu drei zueinander nicht benachbarte CH₂-Gruppen gegen -O- ausgetauscht sein können,
- R¹: für Wasserstoff oder Methyl steht,
- R²: für Isopropyl, Isobutyl, sec.-Butyl, *tert.-*Butyl*,* 1-Hydroxyethyl, Phenyl, Benzyl, 4-Hydroxy-benzyl, 1-Phenylethyl, Diphenylmethyl, 1H Imidazol-4-ylmethyl oder 1H Indol-3-ylmethyl steht,
oder
- R¹ und R²: zusammen mit dem Kohlenstoffatom, an das sie beide gebunden sind, eine 2-Phenylcyclopropan-1,1-diyl-Gruppe der Formel 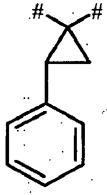 bilden, worin
# die Verknüpfungsstellen mit den übrigen Teilen des Moleküls kennzeichnet,
und.
- T: für eine Gruppe der Formel -C(=O)-OR³, -C(=O)-NR⁴R⁵, -C(=O)-NH-NH-R⁶ oder -CH₂-O-R⁷ steht, worin
R³ Wasserstoff, (C₁-C₆)-Alkyl oder (C₃-C₁₀)-Cycloalkyl bedeutet,
wobei (C₁-C₆)-Alkyl mit Phenyl, Naphthyl oder (C₃-C₁₀)-Cycloalkyl substituiert sein kann,
R⁴ Wasserstoff oder (C₁-C₆)-Alkyl bedeutet,
R⁵ Wasserstoff, (C₁-C₆)-Alkyl oder (C₃-C₁₀)-Cycloalkyl bedeutet, wobei (C₁-C₆)-Alkyl mit Phenyl substituiert sein kann,
oder
R⁴ und R⁵ miteinander verknüpft sind und zusammen mit dem Stickstoffatom, an das sie gebunden sind, einen 5- bis 7-gliedrigen, gesättigten Aza-Heterocyclus bilden, der ein weiteres Ring-Heteroatom aus der Reihe >N-H, >N-CH₃ oder -O- enthalten kann, das sich in 1,3- oder gegebenenfalls 1,4-Stellung in Relation zum erstgenannten Stickstoffatom befindet,
R⁶ (C₁-C₆)-Alkyl, (C₁-C₆)-Alkylcarbonyl, Phenyl oder Benzoyl bedeutet,
und
R⁷ (C₁-C₆)-Alkyl, das mit Phenyl substituiert sein kann, bedeutet,
wobei Phenyl seinerseits mit (C₁-C₆)-Alkoxycarbonyl oder Carboxyl substituiert sein kann,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure; Benzolsulfonsäure, Toluolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, *N,N-*Diisopropylethylamin, Monoethanolamin, Diethanolamin, Trisethanolamin, Dimethylaminoethanol, Diethylaminoethanol, Procain, Dicyclohexylamin, Dibehzylamin, *N*-Methylpiperidin, *N*-Methylmorpholin, Arginin, Lysin und 1,2-Ethylendiamin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, Isobutyl, sec.-Butyl, *tert.*-Butyl*,* n-Pentyl, 2-Pentyl, 3-Pentyl, Neopentyl, n-Hexyl, 2-Hexyl und 3-Hexyl.

(C₁-C₆)-Alkylcarbonyl und (C₁-C₄)-Alkylcarbonyl stehen im Rahmen der Erfindung für einen geradkettigen oder verzweigten Alkylrest mit 1 bis 6 bzw. 1 bis 4 Kohlenstoffatomen, der über eine Carbonyl-Gruppe [-C(=O)-] verknüpft ist. Bevorzugt ist eine geradkettige oder verzweigte Alkylcarbonyl-Gruppe mit 1 bis 4 Kohlenstoffatomen im Alkylrest. Beispielhaft und vorzugsweise seien genannt: Acetyl, Propionyl, n-Butyryl; iso-Butyryl, n-Pentanoyl, Pivaloyl, n-Hexanoyl und *n*-Heptanoyl.

(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy, *tert*.-Butoxy, *n*-Pentoxy und n-Hexoxy.

(C₁-C₆)-Alkoxycarbonyl und (C₁-C₄)-Alkoxycarbonyl stehen im Rahmen der Erfindung für einen geradkettigen oder verzweigten Alkoxyrest mit 1 bis 6 bzw. 1 bis 4 Kohlenstoffatomen, der über eine an das Sauerstoffatom gebundene Carbonyl-Gruppe [-C(=O)-] verknüpft ist. Bevorzugt ist eine geradkettige oder verzweigte Alkoxycarbonyl-Gruppe mit 1 bis 4 Kohlenstoffatomen im Alkoxyrest. Beispielhaft und vorzugsweise seien genannt: Methoxycarbonyl, Ethoxycarbonyl, n-Propoxycarbonyl, Isopropoxycarbonyl, n-Butoxycarbonyl, tert.-Butoxycarbonyl, n-Pentoxycarbonyl und *n*-Hexoxycarbonyl.

(C₁-C₁₂)-Alkandiyl, (C₁-C₈)-Alkandiyl und (C₁-C₆)-Alkandiyl stehen im Rahmen der Erfindung für einen geradkettigen, α,ω-divalenten Alkylrest mit 1 bis 12, 1 bis 8 bzw. 1 bis 6 Kohlenstoffatomen, Bevorzugt ist eine geradkettige Alkandiyl-Gruppe mit 1 bis 8, besonders bevorzugt mit 1 bis 6 Kohlenstoffatomen. Beispielhaft und vorzugsweise seien genannt: Methylen, Ethan-1,2-diyl (1,2-Ethylen), Propan-1,3-diyl (1,3-Propylen), Butan-1,4-diyl (1,4-Butylen), Pentan-1,5-diyl (1,5-Pentylen), Hexan-1,6-diyl (1,6-Hexylen), Heptan-1,7-diyl (1,7-Hexylen), Octan-1,8-diyl (1,8-Octylen), Nonan-1,9-diyl (1,9-Nonylen), Decan-1,10-diyl (1,10-Decylen), Undecan-1,11-diyl (1,11-Undecylen) und Dodecan-1,12-diyl (1,12-Dodecylen).

(C₃-C₆)-Cycloalkyl steht im Rahmen der Erfindung für eine monocyclische, gesättigte Cycloalkylgruppe mit 3 bis 6 Kohlenstoffatomen. Beispielhaft und vorzugsweise seien genannt: Cyclopropyl, Cyclobutyl, Cyclopentyl und Cyclohexyl.

(C₃-C₁₀)-Cycloalkyl steht im Rahmen der Erfindung für eine monocyclische oder gegebenenfalls bi-oder tricyclische, gesättigte Cycloalkylgruppe mit 3 bis 10 Kohlenstoffatomen. Beispielhaft und vorzugsweise seien genannt: Cyclopropyl, Cyclobutyl, Cyclopentyl, Cyclohexyl, Cycloheptyl, Cyclooctyl, Cyclononyl, Cyclodecyl, Hexahydroindanyl, Decalinyl, Bicyclo[2.1.1]hexyl, Bicyclo-[2.2.1]heptyl, Bicyclo[3.2.1]octyl, Bicyclo[2.2.2]octyl, Bicyclo[3.2.2]nonyl, Bicyclo[3.3.1]nonyl, Bicyclo[3.3.2]decyl, Bicyclo[4.3.1]decyl und Adamantyl.

Ein 5- bis 7-gliedriger Aza-Heterocyclus steht im Rahmen der Erfindung für einen monocyclischen, gesättigten Heterocyclus mit insgesamt 5 bis 7 Ringatomen, der ein Ring-Stickstoffatom enthält, über das er zugleich verknüpft ist, und der über dieses hinaus ein weiteres Ring-Heteroatom aus der Reihe >N-H, >N-CH₃ oder -O- enthalten kann, das sich in 1,3- oder gegebenenfalls 1,4-Stellung in Relation zum erstgenannten Ring-Stickstoffatom befindet. Beispielhaft und vorzugsweise seien genannt: Pyrrolidinyl, 1,3-Oxazolidinyl, Piperidinyl, Piperazinyl, N'-Methylpiperazinyl und Morpholinyl.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Bevorzugt im Rahmen der vorliegenden Erfindung sind Verbindungen der Formel (I), in welcher
- L: für geradkettiges (C₁-C₈)-Alkandiyl steht, in dem (a) zwei Kohlenstoffatome in 1,3- oder 1,4-Relation zueinander unter Einbezug des einen beziehungsweise der beiden zwischen ihnen liegenden Kohlenstoffatome zu einem Phenyl-Ring verbrückt sein können oder (b) bis zu zwei zueinander nicht benachbarte CH₂-Gruppen gegen -O- ausgetauscht sein können,
- R¹: für Wasserstoff steht,
- R²: für Benzyl, 4-Hydroxybenzyl, 1-Phenylethyl oder 1*H*-Indol-3-ylmethyl steht,
oder
- R¹ und R²: zusammen mit dem Kohlenstoffatom, an das sie beide gebunden sind, eine 2-Phenyl-cyclopropan-1,1-diyl-Gruppe der Formel 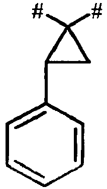 bilden, worin
# die Verknüpfungsstellen mit den übrigen Teilen des Moleküls kennzeichnet,
und
- T: für eine Gruppe der Formel -C(=O)-OR³, -C(=O)-NR⁴R⁵, -C(=O)-NH-NH-R⁶ oder -CH₂-O-R⁷ steht, worin
- R³: Wasserstoff oder (C₁-C₄)-Alkyl; das mit Phenyl, Naphthyl oder (C₃-C₁₀)-Cycloalkyl substituiert sein kann, bedeutet,
- R⁴: Wasserstoff oder Methyl bedeutet,
- R⁵: Wasserstoff oder (C₁-C₄)-Alkyl, das mit Phenyl substituiert sein kann, bedeutet,
oder
- R⁴ und R⁵: miteinander verknüpft sind und zusammen mit dem Stickstoffatom, an das sie gebunden sind, einen Piperidin- oder Morpholin-Ring bilden,
- R⁶: (C₁-C₄)-Alkylcarbonyl oder Benzoyl bedeutet,
und
- R⁷: (C₁-C₄)-Alkyl oder Benzyl, das in der Phenylgruppe mit (C₁-C₄)-Alkoxycarbonyl oder Carboxyl substituiert sein kann, bedeutet,
sowie ihre Salze, Solvate und Solvate der Salze.

Besonders bevorzugt im Rahmen der vorliegenden Erfindung sind Verbindungen der Formel (I), in welcher
- L: für geradkettiges (C₁-C₆)-Alkandiyl steht,
- R¹: für Wasserstoff steht,
- R²: für Benzyl, 1-Phenylethyl oder 1H-Indol-3-ylmethyl steht,
oder
- R¹ und R²: zusammen mit dem Kohlenstoffatom, an das sie beide gebunden sind, eine (1*S*,2*R*)-2-Phenylcyclopropan-1,1-diyl-Gruppe der Formel 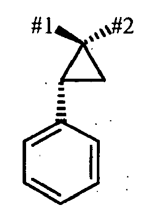 bilden, worin
# 1 die Verknüpfungsstelle mit dem angrenzenden Stickstoffatom
und
#2 die Verknüpfungsstelle mit der Gruppe T kennzeichnen,
und
- T: für eine Gruppe der Formel -C(=O)-OR³, -C(=O)-NR⁴R⁵, -C(=O)-NH-NH-R⁶ oder -CH₂-O-R⁷ steht, worin
R³ Wasserstoff, Methyl, Ethyl, n-Propyl, Benzyl oder Adamantylmethyl bedeutet,
R⁴ Wasserstoff oder Methyl bedeutet,
R⁵ Wasserstoff, Methyl, Ethyl, n-Propyl oder Benzyl bedeutet,
R⁶ Benzoyl bedeutet,
und
R⁷ Benzyl, das in der Phenylgruppe mit Methoxycarbonyl oder Carboxyl substituiert sein kann, bedeutet,
sowie ihre Salze, Solvate und Solvate der Salze.

Von besonderer Bedeutung im Rahmen der vorliegenden Erfindung sind Verbindungen der Formel (I-A) 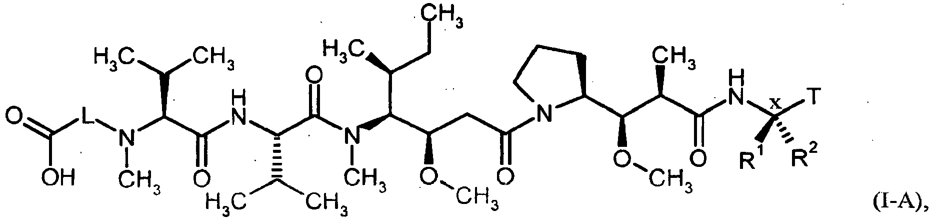 in welcher L, R¹ R² und T die oben definierten Bedeutungen haben und das die Reste R¹ und R² tragende C^{X}-Kohlenstoffatom die abgebildete Absolutkonfiguration aufweist,
sowie ihre Salze, Solvate und .Solvate der Salze.

Von besonderer Bedeutung im Rahmen der vorliegenden Erfindung sind auch Verbindungen der Formeln (I) und (I-A), in welchen
- L: für Propan-1,3-diyl steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt. Ganz besonders bevorzugt sind Kombinationen von zweien oder mehreren der oben genannten Vorzugsbereiche.

Weiterer Gegenstand der vorliegenden Erfindung ist ein Verfahren zur Herstellung der erfindungsgemäßen Verbindungen der Formel (I), dadurch gekennzeichnet, dass man eine Verbindung der Formel (II) 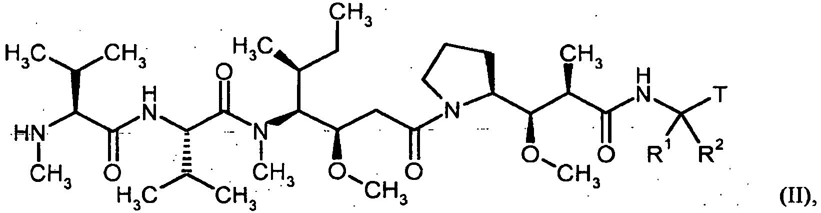 in welcher R¹, R² und T die oben angegebenen Bedeutungen haben,
in einem inerten Lösungsmittel entweder
[A] durch baseninduzierte Alkylierung mit einer Verbindung der Formel (III) 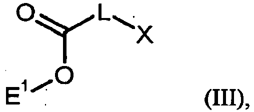 in welcher L die oben angegebene Bedeutung hat,
   - E¹: für Wasserstoff, (C₁-C₄)-Alkyl oder Benzyl steht,
   und
   - X: für eine Fluchtgruppe wie beispielsweise Chlorid, Bromid, Iodid, Mesylat, Triflat oder Tosylat steht,
   zu einer Verbindung der Formel (IV) 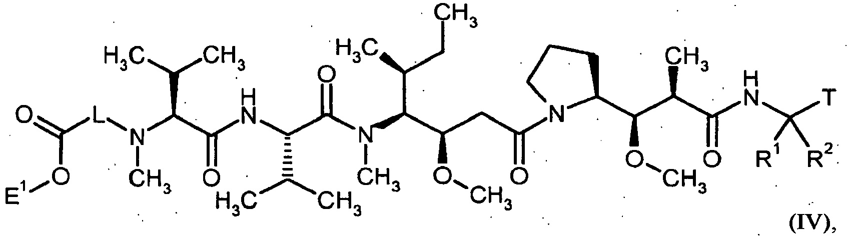 in welcher E¹, L, R¹, R² und T die oben angegebenen Bedeutungen haben,
   umsetzt und anschließend im Fall, dass E¹ für (C₁-C₄)-Alkyl oder Benzyl steht, diesen Ester-Rest, nach üblichen Methoden abspaltet, so dass ebenso wie im Fall, dass E¹ in (III) für Wasserstoff steht, die erfindungsgemäße Carbonsäure der Formel (I) 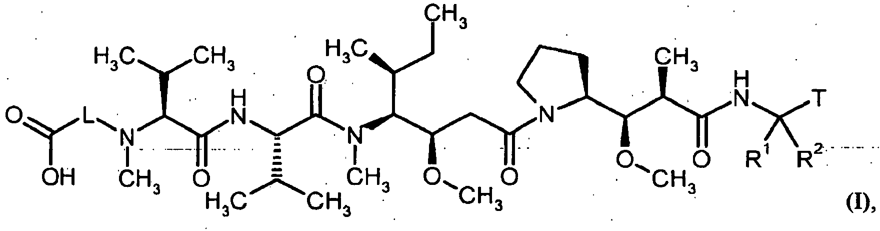 in welcher L, R¹, R² und T die oben angegebenen Bedeutungen haben,
   erhalten wird,
   oder
[B] durch Umsetzung mit einer Verbindung der Formel (V) 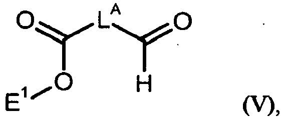 in welcher
   - E¹: für Wasserstoff, (C₁-C₄)-Alkyl oder Benzyl steht,
   und
   - L^{A}: die oben definierte Bedeutung von L hat, jedoch in der Alkyl-Kettenlänge um eine CH₂-Einheit verkürzt ist,
   in Gegenwart eines geeigneten Reduktionsmittels in eine Verbindung der Formel (VI) 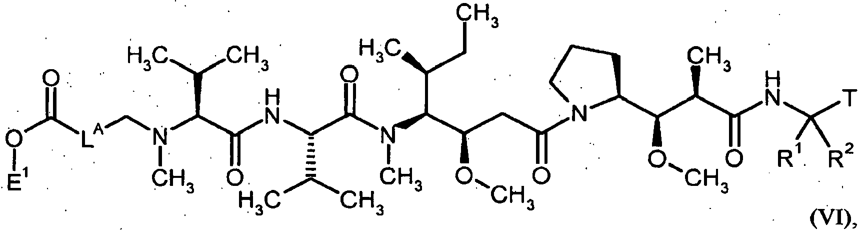 in welcher E¹, L^{A}, R¹, R² und T die oben angegebenen Bedeutungen haben,
   überführt und anschließend im Fall, dass E¹ für (C₁-C₄)-Alkyl oder Benzyl steht, diesen Ester-Rest nach üblichen Methoden abspaltet, so dass ebenso wie im Fall, dass E¹ in (V) für Wasserstoff steht, die erfindungsgemäße Carbonsäure der Formel (I-B) 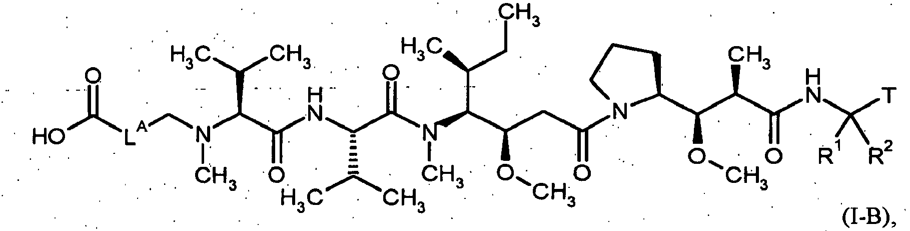 in welcher L^{A}, R¹, R² und T die oben angegebenen Bedeutungen haben,
   erhalten wird,
und die resultierenden Verbindungen der Formel (I) bzw. (I-B) gegebenenfalls in ihre Enantiomere und/oder Diastereomere trennt und/oder mit den entsprechenden (i) Lösungsmitteln und/oder (ii) Basen oder Säuren zu ihren Solvaten, Salzen und/oder Solvaten der Salze umsetzt.

Als inerte Lösungsmittel für die Umsetzung (II) + (III) → (IV) eignen sich beispielsweise Ether wie Diethylether, Diisopropylether, Methyl-tert.-butylether, Tetrahydrofuran, 1,4-Dioxan, 1,2-Dimethoxyethan oder Bis-(2-methoxyethyl)-ether, Kohlenwasserstoffe wie Benzol, Toluol, Xylol, Pentan, Hexan, Heptan, Cyclohexan oder Erdölfraktionen, oder dipolar-aprotische Lösungsmittel wie Aceton, Methylethylketon, Acetonitril, Dimethylsulfoxid (DMSO), *N,N-*Dimethylformamid (DMF), *N,N'*-Dimethylacetamid (DMA), *N,N'*-Dimethylpropylenharnstoff (DMPU), *N-*Methyl-pyrrolidinon (NMP) oder Pyridin. Ebenso ist es möglich, Gemische solcher Lösungsmittel einzusetzen. Bevorzugt wird Aceton oder *N,N-*Dimethylformamid verwendet.

Geeignete Basen für diese Alkylierungsreaktion sind insbesondere Alkalihydroxide wie beispielsweise Lithium-, Natrium- oder Kaliumhydroxid, Alkali- oder Erdalkalicarbonate wie Lithium-, Natrium-, Kalium-, Calcium- oder Cäsiumcarbonat, oder übliche organische Amine wie Triethylamin, *N*-Methylmorpholin, *N*-Methylpiperidin, *N,N-*Diisopropylethylamin, Pyridin oder 4*-N,N-*Dimethylaminopyridin. Bevorzugt wird Kalium- oder Cäsiumcarbonat verwendet. Gegebenenfalls ist der Zusatz eines Alkylierungskatalysators von Vorteil, wie beispielsweise Lithiumbromid oder -iodid, Natrium- oder Kaliumiodid, Tetra-n-butylammoniumbromid oder -iodid oder Benzyltriethyl-ammoniumbromid.

Die Reaktion (II) + (III) → (IV) wird im Allgemeinen in einem Temperaturbereich von -20°C bis +100°C, bevorzugt bei 0°C bis +50°C durchgeführt. Die Umsetzung kann bei normalem, bei erhöhtem oder bei erniedrigtem Druck erfolgen (z.B. von 0.5 bis 5 bar); in der Regel arbeitet man bei Normaldruck.

Die Umsetzung (II) + (V) → (VI) erfolgt in den für eine reduktive Aminierung üblichen, unter den Reaktionsbedingungen inerten Lösungsmitteln, gegebenenfalls in Gegenwart einer Säure und/ oder eines wasserentziehenden Mittels als Katalysator. Zu solchen Lösungsmitteln gehören beispielsweise Alkohole wie Methanol, Ethanol, *n*-Propanol, Isopropanol, n-Butanol oder tert.-Butanol, Ether wie Tetrahydrofuran, 1,4-Dioxan, 1,2-Dimethoxyethan oder Bis-(2-methoxyethyl)-ether, oder andere Solventien wie Dichlormethan, 1,2-Dichlorethan, N,N Dimethylformamid oder auch Wasser. Ebenso ist es möglich, Gemische dieser Lösungsmittel einzusetzen. Bevorzugt wird als Lösungsmittel ein 1,4-Dioxan/Wasser-Gemisch verwendet unter Zusatz von Essigsäure oder verdünnter Salzsäure als Katalysator.

Als Reduktionsmittel eignen sich für diese Reaktion insbesondere komplexe Borhydride, wie beispielsweise Natriumborhydrid, Natriumcyanoborhydrid, Natriumtriacetoxyborhydrid oder Tetra-n-butylammoniumborhydrid. Bevorzugt wird Natriumcyanoborhydrid eingesetzt.

Die Umsetzung (II) + (V) → (VI) erfolgt im Allgemeinen in einem Temperaturbereich von 0°C bis +120°C, bevorzugt bei +50°C bis +100°C. Die Reaktion kann bei normalem, bei erhöhtem oder bei erniedrigtem Druck durchgeführt werden (z.B. von 0.5 bis 5 bar); in der Regel arbeitet man bei Normaldruck.

Die Abspaltung eines Ester-Restes E¹ in den Verfahrensschritten (IV) → (I) und (VI) → (I-B) [E¹ = (C₁-C₄)-Alkyl oder Benzyl] wird nach üblichen Methoden durchgeführt, indem man den Ester in einem inerten Lösungsmittel mit einer Säure oder einer Base behandelt, wobei bei letzterer Variante das zunächst entstehende Carboxylat-Salz durch nachfolgende Zugabe einer Säure in die freie Carbonsäure überführt wird. Im Falle eines *tert*.-Butylesters geschieht die Spaltung bevorzugt mittels einer Säure. Bei einem Benzylester kann die Abspaltung auch durch Hydrogenolyse in Gegenwart eines geeigneten Palladium-Katalysators, wie beispielsweise Palladium auf Aktivkohle, erfolgen.

Der aus Verbindung (III) bzw. (V) stammende Ester-Rest E¹ wird hierbei so gewählt, dass die Bedingungen seiner Abspaltung kompatibel mit der jeweiligen Gruppe T in Verbindung (IV) und (VI) sind.

Als Base für die Ester-Hydrolyse sind die üblichen anorganischen Basen geeignet. Hierzu gehören insbesondere Alkali- oder Erdalkalihydroxide wie beispielsweise Lithium-, Natrium-, Kalium- oder Bariumhydroxid, oder Alkali- oder Erdalkalicarbonate wie Natrium-, Kalium- oder Calciumcarbonat. Bevorzugt sind Lithium-, Natrium-oder Kaliumhydroxid.

Als Säure eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle eines tert.-Butylesters und Salzsäure bei einem Methylester.

Als inerte Lösungsmittel eignen sich für diese Reaktionen Wasser oder die für eine Esterspaltung üblichen organischen Lösungsmittel. Hierzu gehören bevorzugt niedere Alkohole wie Methanol, Ethanol, n-Propanol oder Isopropanol, Ether wie Diethylether, Tetrahydrofuran, 1,4-Dioxan oder 1,2-Dimethoxyethan, oder andere Lösungsmittel wie Dichlormethan, Aceton, Methylethylketon, *N,N-*Dimethylformamid oder Dimethylsulfoxid. Ebenso ist es möglich, Gemische dieser Lösungsmittel einzusetzen. Im Falle einer basischen Ester-Hydrolyse werden bevorzugt Gemische von Wasser mit 1,4-Dioxan, Tetrahydrofuran, Methanol, Ethanol und/oder Dimethylformamid verwendet. Im Falle der Umsetzung mit Trifluoressigsäure wird bevorzugt Dichlormethan und im Falle der Umsetzung mit Chlorwasserstoff bevorzugt Tetrahydrofuran, Diethylether, 1,4-Dioxan oder Wasser eingesetzt.

Die Esterspaltung erfolgt im Allgemeinen in einem Temperaturbereich von -20°C bis +100°C, bevorzugt bei 0°C bis +50°C.

Die Verbindungen der Formel (II) können nach üblichen Methoden der Peptidchemie beispielsweise dadurch hergestellt werden, dass man eine Verbindung der Formel (VII) 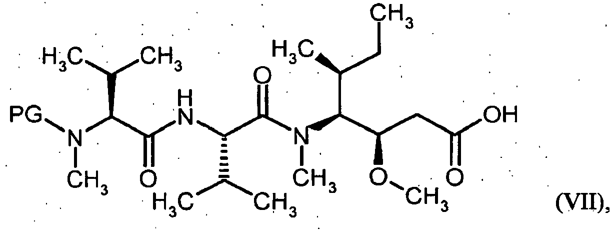 in welcher
- PG: für eine Amino-Schutzgruppe wie beispielsweise (9H-Fluoren-9-ylmethoxy)carbonyl, *tert.-*Butoxycarbonyl oder Benzyloxycarbonyl steht,
in einem inerten Lösungsmittel unter Aktivierung der Carboxyl-Funktion in (VII) entweder
[C] zunächst mit einer Verbindung der Formel (VIII) 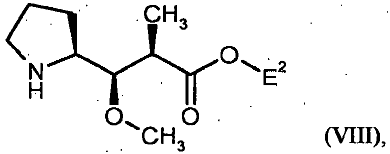 in welcher
   - E²: für Wasserstoff, (C₁-C₄)-Alkyl oder Benzyl steht,
   oder einem Salz dieser Verbindung zu einer Verbindung der Formel (IX) 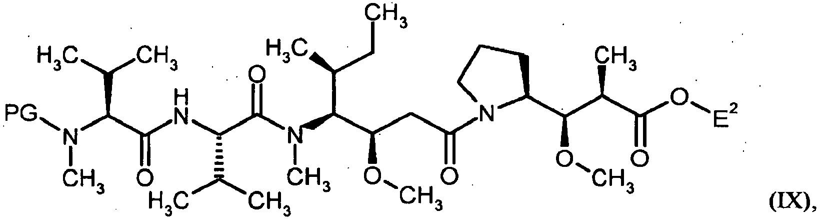 in welcher E² und PG die oben angegebenen Bedeutungen haben,
   kuppelt, anschließend im Fall, dass E² für (C₁-C₄)-Alkyl oder Benzyl steht, diesen Ester-Rest nach üblichen Methoden abspaltet und die resultierende Carbonsäure der Formel (X) 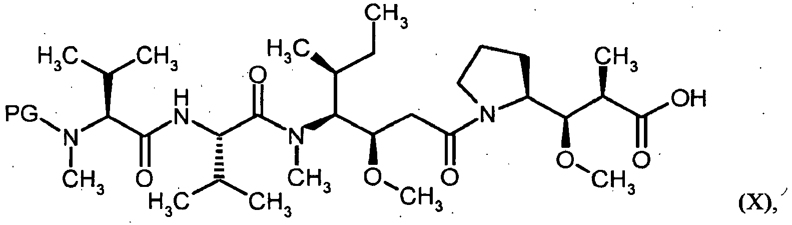 in welcher PG die oben angegebene Bedeutung hat,
   dann in einem inerten Lösungsmittel unter Aktivierung der Carboxyl-Funktion mit einer Verbindung der Formel (XI) 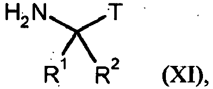 in welcher R¹, R² und T die oben angegebenen Bedeutungen haben,
   oder einem Salz dieser Verbindung zu einer Verbindung der Formel (XII) 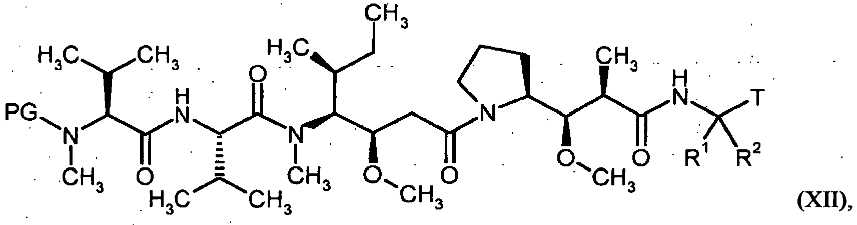 in welcher PG, R¹, R² und T die oben angegebenen Bedeutungen haben,
   kuppelt
   oder
[D] mit einer Verbindung der Formel (XIII) 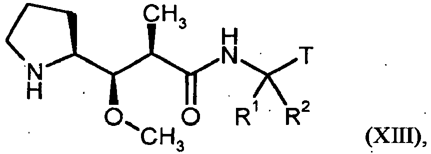 in welcher R¹, R² und T die oben angegebenen Bedeutungen haben,
   oder einem Salz dieser Verbindung gleichfalls zu der Verbindung der Formel (XII) 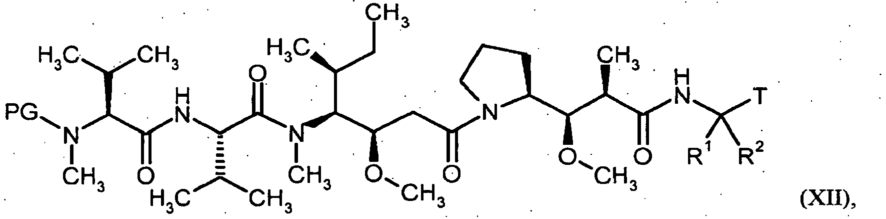 in welcher PG, R¹, R² und T die oben angegebenen Bedeutungen haben,
   kuppelt
und die Verbindung der Formel (XII) dann auf übliche Weise zu einer Verbindung der Formel (II) 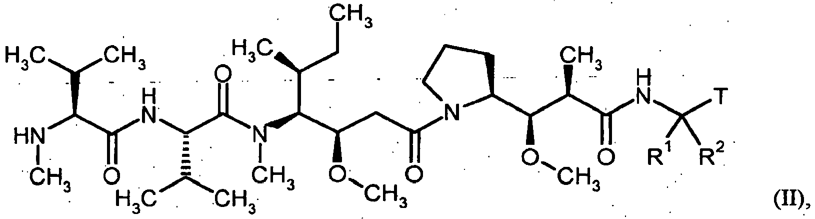 in welcher R¹, R² und T die oben angegebenen Bedeutungen haben,
entschützt.

Die oben beschriebenen Kupplungsreaktionen (Amid-Bildung aus jeweiliger Amin- und Carbonsäure-Komponente) werden nach allgemein üblichen Methoden der Peptidchemie durchgeführt [siehe z.B. M. Bodanszky, Principles of Peptide Synthesis, Springer-Verlag, Berlin, 1993; M. Bodanszky und A. Bodanszky, The Practice of Peptide Synthesis, Springer-Verlag, Berlin, 1984; H.-D. Jakubke und H. Jeschkeit, Aminosäuren, Peptide, Proteine, Verlag Chemie, Weinheim, 1982]. Inerte Lösungsmittel für die Kupplungsreaktionen (VII) + (VIII) → (IX), (X) + (XI) → (XII) und (VII) + (XIII) → (XII) sind beispielsweise Ether wie Diethylether, Diisopropylether, *tert.*-Butyl-methylether, Tetrahydrofuran, 1,4-Dioxan, 1,2-Dimethoxyethan oder Bis-(2-methoxyethyl)-ether, Kohlenwasserstoffe wie Benzol, Toluol; Xylol, Pentan, Hexan, Heptan, Cyclohexan oder Erdölfraktionen, Halogenkohlenwasserstoffe wie Dichlormethan, Trichlormethan, Tetrachlormethan, 1,2-Dichlorethan, Trichlorethylen oder Chlorbenzol, oder dipolar-aprotische Lösungsmittel wie Aceton, Methylethylketon, Acetonitril, Ethylacetat, Pyridin, Dimethylsulfoxid (DMSO), *N,N-*Dimethylformamid (DMF), *N,N-*Dimethylacetamid (DMA), *N,N*'-Dimethylpropylenharnstoff (DMPU) oder *N-*Methylpyrrolidinon (NMP). Ebenso ist es möglich, Gemische solcher Lösungsmittel zu verwenden. Bevorzugt wird *N,N*-Dimethylformamid eingesetzt.

Als Aktivierungs-/Kondensationsmittel für diese. Kupplungen eignen sich beispielsweise Carbodi-imide wie *N,N*'-Diethyl-, *N,N*'-Dipropyl-, *N,N*'-Diisopropyl-, *N,N*'-Dicyclohexylcarbodiimid (DCC) oder *N*-(3-Dimethylaminoisopropyl)-N'-ethylcarbodiimid-Hydrochlorid (EDC), Phosgen-Derivate wie *N,N*'-Carbonyldiimidazol (CDI) oder Isobutylchlorformiat, 1,2-Oxazolium-Verbindungen wie 2-Ethyl-5-phenyl-1,2-oxazolium-3-sulfat oder 2-*tert*.-Butyl-5-methylisoxazolium-perchlorat, Acyl-amino-Verbindungen wie 2-Ethoxy-1-ethoxycarbonyl-1,2-dihydrochinolin, α-Chlorenamine wie 1-Chlor-2-methyl-1-dimethylamino-1-propen, Phosphor-Verbindungen wie Propanphosphonsäureanhydrid, Cyanophosphonsäurediethylester, Bis-(2-oxo-3-oxazolidinyl)-phosphorylchlorid, Benzotriazol-1-yloxy-tris(dimethylamino)phosphonium-hexafluorophosphat oder Benzotriazol-1-yloxy-tris-(pyrrolidino)phosphonium-hexafluorophosphat (PyBOP), oder Uronium-Verbindungen wie *O*-(Benzotriazol-1-yl)-*N,N,N',N*'-tetramethyluronium-tetrafluoroborat (TBTU), *O*-(Benzotriazol-1-yl)-*N,N,N',N*'-tetramethyluronium-hexafluorophosphat (HBTU), 2-(2-Oxo-1-(2*H*)-pyridyl)-1,1,3,3-tetramethyluronium-tetrafluoroborat (TPTU), *O*-(7-Azabenzotriazol-1-yl)-*N,N,N',N*'-tetramethyl-uronium-hexafluorophosphat (HATU) oder *O-*(1*H-*6-Chlorbenzotriazol-1-yl)-1,1,3,3-tetramethyl-uronium-tetrafluoroborat (TCTU), gegebenenfalls in Kombination mit weiteren Hilfsstoffen wie 1-Hydroxybenzotriazol (HOBt) oder *N*-Hydroxysuccinimid (HOSu), sowie als Basen Alkalicarbonate, z.B. Natrium- oder Kaliumcarbonat, oder tertiäre Aminbasen wie Triethylamin, *N*-Methylmorpholin, N-Methylpiperidin, *N,N*-Diisopropylethylamin, Pyridin oder 4-*N,N*-Dimethylaminopyridin.

Im Rahmen der vorliegenden Erfindung wird als Aktivierungs-/Kondensationsmittel für solche Kupplungsreaktionen bevorzugt *N*-(3-Dimethylaminoisopropyl)-*N*'-ethylcarbodiimid-Hydrochlorid (EDC) in Kombination mit 1-Hydroxybenzotriazol (HOBt) und *N,N*-Diisopropylethylamin, oder *O-*(7-Azabenzotriazol-1-yl)-*N,N,N',N*'-tetramethyluronium-hexafluorophosphat (HATU) gleichfalls in Verbindung mit *N,N-*Diisopropylethylamin verwendet.

Die Kupplungsreaktionen (VII) + (VIII) → (IX), (X) + (XI) → (XII) und (VII) + (XIII) → (XII) werden in der Regel in einem Temperaturbereich von -20°C bis +60°C, bevorzugt bei 0°C bis +40°C durchgeführt. Die Umsetzungen können bei normalem, bei erhöhtem oder bei erniedrigtem Druck erfolgen (z.B. von 0.5 bis 5 bar); im Allgemeinen arbeitet man bei Normaldruck.

Gegebenenfalls vorhandene funktionelle Gruppen - wie insbesondere Amino-, Hydroxy- und Carboxylgruppen - können bei den zuvor beschriebenen Verfährensschritten, falls zweckmäßig oder erforderlich, auch in temporär geschützter Form vorliegen. Die Einführung und Entfernung solcher Schutzgruppen erfolgt hierbei nach üblichen, aus der Peptidchemie bekannten Methoden [siehe z.B. T.W. Greene und P.G.M. Wuts, Protective Groups in Organic Synthesis, Wiley, New York, 1999; M. Bodanszky und A. Bodanszky, The Practice of Peptide Synthesis, Springer-Verlag, Berlin, 1984]._{.} Bei Vorhandensein mehrerer geschützter Gruppen kann deren Wiederfreisetzung gege-benenfalls simultan in einer Eintopf-Reaktion oder auch in separaten Reaktionsschritten vor-genommen werden.

Als Amino-Schutzgruppe wird bevorzugt *tert*.-Butoxycarbonyl (Boc), Benzyloxycarbonyl (Z) oder (9*H*-Fluoren-9-ylmethoxy)carbonyl (Fmoc) verwendet; für eine Hydroxy- oder Carboxyl-Funktion wird vorzugsweise *tert.-*Butyl oder Benzyl als Schutzgruppe eingesetzt. Die Abspaltung einer *tert.-*Butyl- oder *tert*-Butoxycarbonyl-Gruppe geschieht üblicherweise durch Behandlung mit einer starken Säure, wie Chlorwasserstoff, Bromwasserstoff oder Trifluoressigsäure, in einem inerten Lösungsmittel wie Diethylether, 1,4-Dioxan, Dichlormethan oder Essigsäure; gegebenenfalls kann diese Reaktion auch ohne Zusatz eines inerten Lösungsmittels durchgeführt werden. Im Falle von Benzyl oder Benzyloxycarbonyl als Schutzgruppe werden diese bevorzugt durch Hydrogenolyse in Gegenwart eines geeigneten Palladium-Katalysators, wie beispielsweise Palladium auf Aktivkohle, entfernt. Die (9*H*-Fluoren-9-ylmethoxy)carbonyl-Gruppe wird im Allgemeinen mit Hilfe einer sekundären Aminbase wie Diethylamin oder Piperidin abgespalten.

Ein Ester-Rest E² in Verbindung (VIII) [E² = (C₁-C₄)-Alkyl oder Benzyl] wird hierbei so gewählt, dass die Bedingungen seiner Abspaltung kompatibel mit der jeweils eingesetzten Schutzgruppe PG aus Verbindung (VII) sind.

Die Verbindungen der Formel (VII) können auf analoge Weise beispielsweise dadurch hergestellt werden, dass man zunächst N-(Benzyloxycarbonyl)-L-Valin der Formel (XIV) 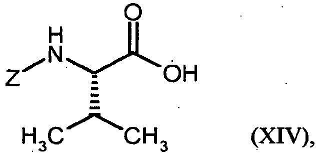 in welcher Z für die Benzyloxycarbonyl-Schutzgruppe steht,
mit Hilfe eines Kondensationsmittels mit einer Verbindung der Formel (XV) 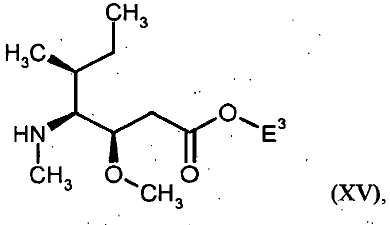 in welcher E³ für (C₁-C₄)-Alkyl steht,
oder einem Salz dieser Verbindung zu einer Verbindung der Formel (XVI) 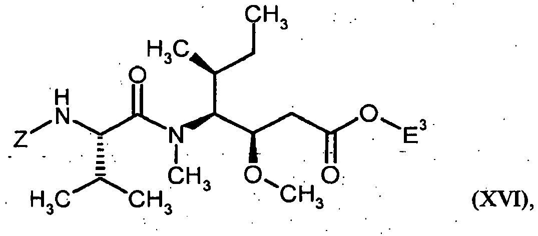 in welcher E³ und Z die oben angegebenen Bedeutungen haben,
kuppelt, diese nach hydrogenolytischer Entfernung der Z-Schutzgruppe dann in Gegenwart eines Kondensationsmittels mit *N*-geschütztem *N*-Methyl-L-valin der Formel (XVII) 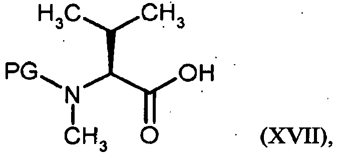 in welcher
PG für eine Amino-Schutzgruppe wie beispielsweise (9*H*-Fluoren-9-ylmethoxy)carbonyl, *tert.-*Butoxycarbonyl oder Benzyloxycarbonyl steht,
zu einer Verbindung der Formel (XVIII) 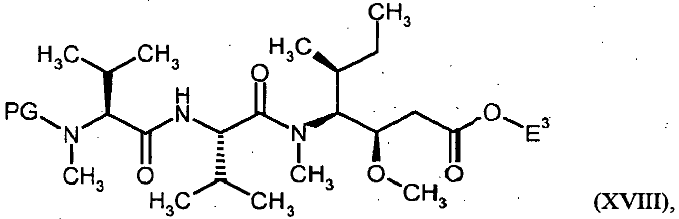 in welcher E³ und PG die oben angegebenen Bedeutungen haben,
kuppelt und abschließend die Ester-Gruppierung -C(O)O-E³ in (XVIII) nach üblichen Methoden in die freie Carbonsäure (VII) überführt.

Die Kupplungen (XIV) + (XV) → (XVI) und Z-entschütztes (XVI) + (XVII) → (XVIII) werden unter analogen Reaktionsbedingungen durchgeführt wie zuvor bei den in Verfahren [C] und [D] dargestellten Kupplungsschritten beschrieben.

Die Hydrolyse der Ester-Gruppe -C(O)O-E³ im Reaktionsschritt (XVIII) → (VII) erfolgt auf analoge Weise wie zuvor im Rahmen der Verfahrenssequenzen [A] und [B] für den Ester-Rest E¹ beschrieben. Die Alkylgruppe E³ in Verbindung (XV) wird hierbei so gewählt, dass die Bedingungen ihrer Abspaltung kompatibel mit der jeweils eingesetzten Schutzgruppe PG aus Verbindung (XVII) sind.

Die Verbindungen der Formel (XIII) ihrerseits sind durch Kupplung der oben beschriebenen Verbindung (XI) mit der Verbindung (XIX) 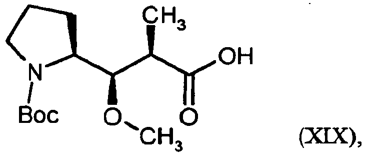 in welcher Boc für die tert.-Butoxycarbonyl-Schutzgruppe steht,
zu einer Verbindung der Formel (XX) 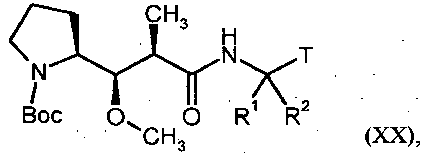 in welcher Boc, R¹, R² und T die oben angegebenen Bedeutungen haben,
und nachfolgende Abspaltung der Boc-Schutzgruppe zugänglich.

Die Kupplungsreaktion (XI) + (XIX) → (XX) wird wiederum unter analogen Bedingungen durchgeführt wie zuvor bei den in Verfahren [C] und [D] dargestellten Kupplungsschritten beschrieben.

Die Verbindungen der Formeln (III), (V), (VIII), (XI), (XIV), (XV), (XVII) und (XIX) einschießlich, wo zutreffend, chiraler oder diastereomerer Formen hiervon sind kommerziell erhältlich oder als solche in der Literatur beschrieben, oder sie können auf für den Fachmann offenkundigem Wege in Analogie zu in der Literatur publizierten Methoden hergestellt werden. Zahlreiche ausführliche Vorschriften sowie Literaturangaben zur Herstellung der Ausgangsmaterialien befinden sich auch im Experimentellen Teil im Abschnitt zur Herstellung der Ausgangsverbindungen und Intermediate.

Sollten entsprechende isomerenreine Ausgangsmaterialien nicht zur Verfügung stehen, so kann eine Trennung der erfindungsgemäßen Verbindungen in die korrespondierenden Enantiomere und/ oder Diastereomere zweckmäßigerweise auch bereits auf der Stufe der Verbindungen (II), (IV), (VI), (XI), (XII), (XIII) und (XX) erfolgen, welche dann in separierter Form gemäß den zuvor beschriebenen Reaktionsschritten weiter umgesetzt werden. Eine solche Auftrennung der Stereoisomeren läßt sich nach üblichen, dem Fachmann bekannten Methoden durchführen. Bevorzugt werden chromato- , graphische Verfahren an achiralen bzw. chiralen Trennphasen angewandt; im Falle von freien Carbonsäuren als Zwischenprodukten kann alternativ auch eine Trennung über diastereomere Salze mit Hilfe chiraler Basen erfolgen.

Die Herstellung der erfindungsgemäßen Verbindungen kann durch die folgenden Reaktionsschemata beispielhaft veranschaulicht werden:

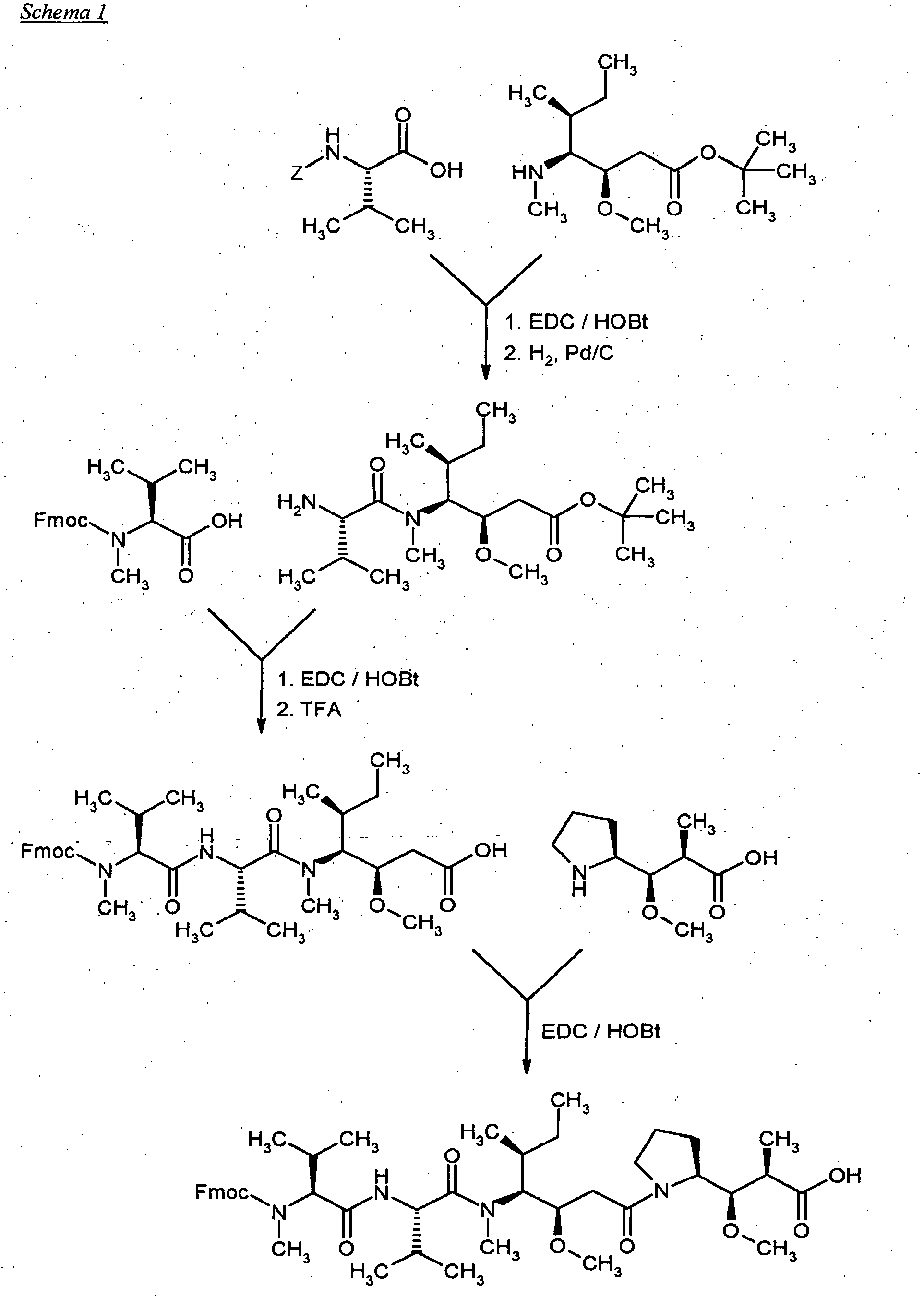

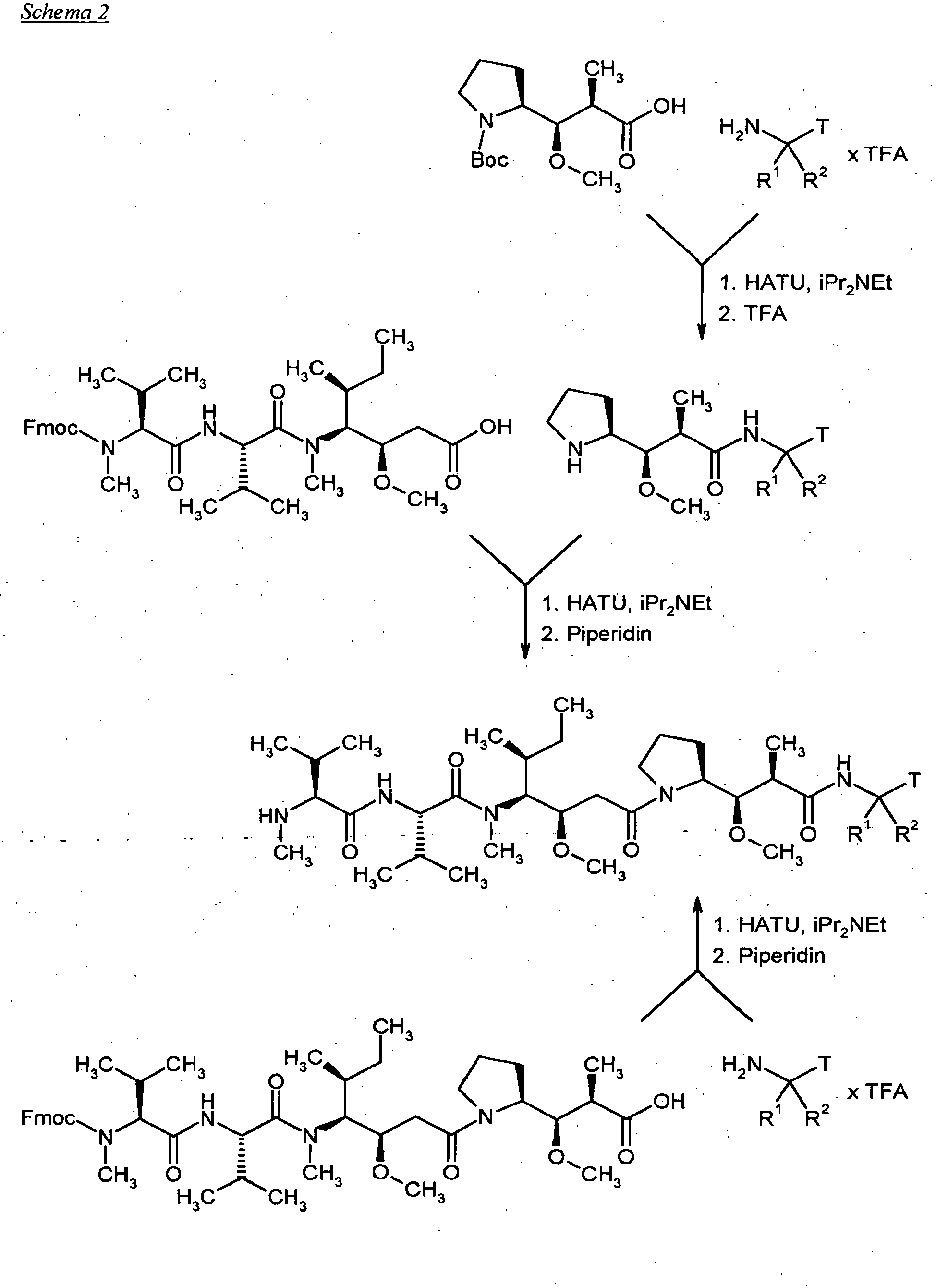

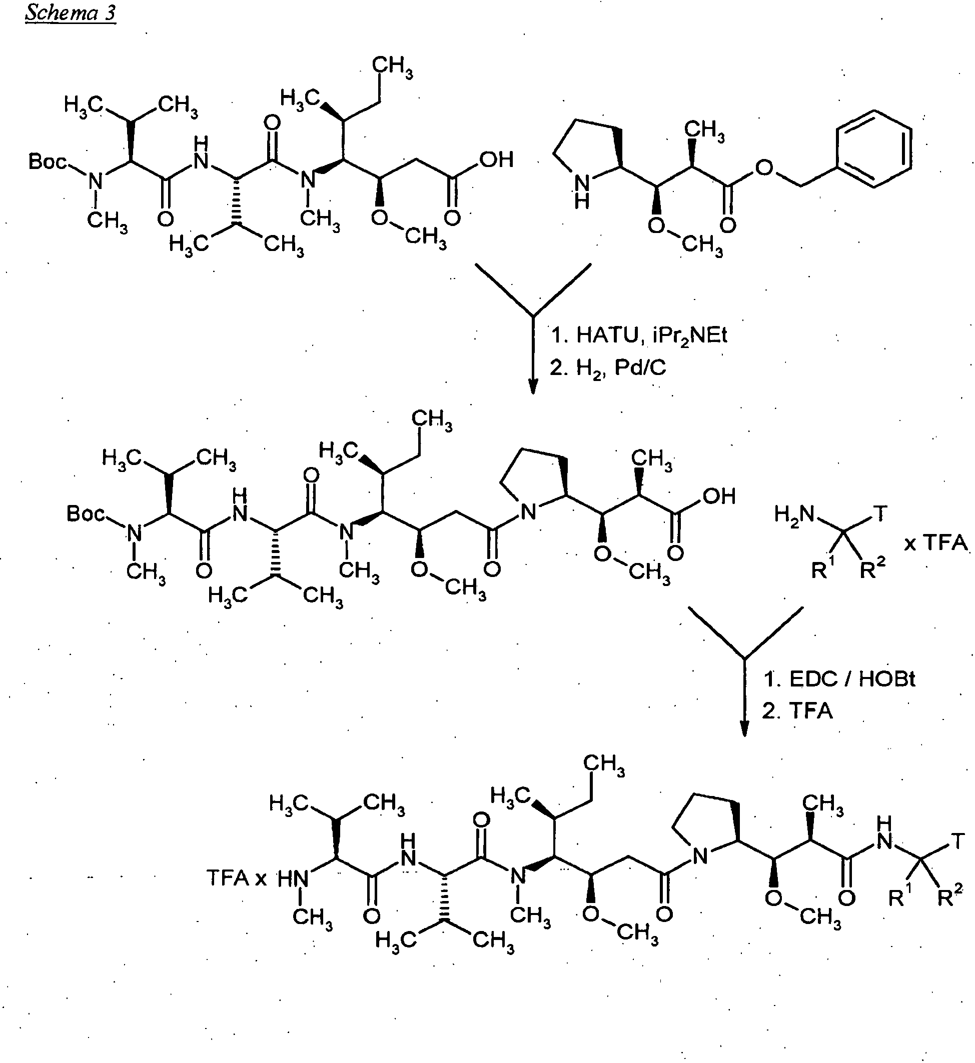

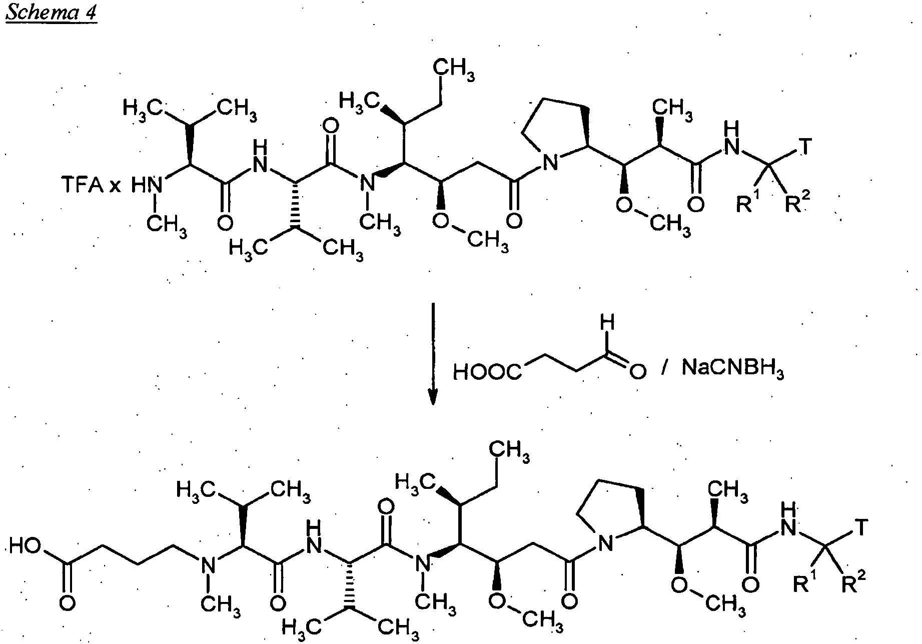

Die erfindungsgemäßen Verbindungen besitzen wertvolle pharmakologische Eigenschaften und können zur Vorbeugung und Behandlung von Erkrankungen bei Menschen und Tieren verwendet werden.

Im Vergleich zu anderen, aus dem Stand der Technik bekannten Auristatin-Derivaten hat die in den Verbindungen der vorliegenden Erfindung enthaltene N-terminale Carboxyalkyl-Gruppierung [HOOC-L- in Formel (I)] nicht die bloße Funktion eines Linkers für die potentielle Verknüpfung mit Antikörperproteinen oder anderen Liganden, sondern stellt vielmehr ein konstitutives Strukturelement für das überraschend vorteilhafte Eigenschaftsprofil dieser Verbindungen dar.

Die erfindungsgemäßen Verbindungen weisen im Vergleich beispielsweise zu Monomethylauristatin F (MMAF) eine deutlich stärkere cytotoxische Aktivität auf und haben andererseits ein verringertes Potential, gleichzeitig auch Substrate für zelluläre Transporterproteine zu sein.

Die erfindungsgemäßen Verbindungen sind daher in besonderem Maße zur Behandlung von hyperproliferativen Erkrankungen beim Menschen und bei Säugetieren allgemein geeignet. Die Verbindungen können einerseits die Zellproliferation und Zellteilung hemmen, blockieren, verringern oder senken und andererseits die Apoptose verstärken.

Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (Mammakarzinome einschließlich ductaler und lobulärer Formen, auch *in situ*), Atemwegstumore (kleinzelliges und nicht-kleinzelliges Karzinom, Bronchialkarzinome), Himtumore (z.B. des Hirnstamms und des Hypothalamus, Astrocytoma, Ependymoma, Glioblastoma, Gliome, Medulloblastoma, Meningiome sowie neuro-ektodermale und pineale Tumore), Tumore der Verdauungsorgane (Speiseröhren-, Magen-, Gallenblasen-, Dünndarm-, Dickdarm-, Rektum- und Analkarzinome), Lebertumore (u.a. hepatozelluläres Karzinom, Cholangiokarzinom und gemischthepatozelluläres Cholangiokarzinom), Tumore des Kopf- und Halsbereiches (Larynx-, Hypopharynx-, Nasopharynx-, Oropharynx-, Lippen- und Mundhöhlenkarzinome, orale Melanome), Hauttumore (Basaliome, Spinaliome, Plattenepithelkarzinome, Kaposi-Sarkom, maligne Melanome, nicht-melanomartiger Hautkrebs, Merkelzell-Hautkrebs, Mastzelltumore), Tumore des Stütz- und Bindegewebes (u.a. Weichteilsarkome, Osteosarkome, maligne fibröse Histiozytome, Chondrosarkome, Fibrosarkome, Hämangiosarkome, Leiomyosarkome, Liposarkome, Lymphosarkome und Rhabdomyosarkome), Tumore der Augen (u.a. intraokuläres Melanom und Retinoblastom), Tumore der endokrinen und exokrinen Drüsen (z.B. der thyroiden und parathyroiden Drüsen, Bauchspeicheldrüsen- und Speicheldrüsenkarzinome, Adenokarzinome), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostataund Hodenkarzinome des Mannes): Dazu gehören auch proliferative Erkrankungen des Blutes, des Lymphsystems und des Rückenmarks, in solider Form und als zirkulierende Zellen, wie Leukämien, Lymphome und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Diese gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Die Behandlung der zuvor genannten Krebserkrankungen mittels der erfindungsgemäßen Verbindungen umfasst sowohl eine Behandlung der soliden Tumore als auch eine Behandlung metastasierter oder zirkulierender Formen hiervon.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen- eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen.

Beispielsweise können die Verbindungen der vorliegenden Erfindung mit bekannten anti-hyperproliferativen, zytostatischen oder zytotoxischen Substanzen zur Behandlung von Krebserkrankungen kombiniert werden. Als geeignete Kombinationswirkstoffe seien beispielhaft genannt:
Aldesleukin, Alendronsäure, Alfaferon, Alitretinoin, Allopurinol, Aloprim, Aloxi, Altretamin, Aminoglutethimid, Amifostin, Amrubicin, Amsacrin, Anastrozol, Anzmet, Aranesp, Arglabin, Arsentrioxid, Aromasin, 5-Azacytidin, Azathioprin, BCG oder tice-BCG, Bestatin, Betamethason-Acetat, Betamethason-Natriumphosphat, Bexaroten, Bleomycin-Sulfat, Broxuridin, Bortezomib, Busulfan, Calcitonin, Campath, Capecitabin, Carboplatin, Casodex, Cefeson, Celmoleukin, Cerubidin, Chlorambucil, Cisplatin, Cladribin, Clodronsäure, Cyclophosphamid, Cytarabin, Dacarbazin, Dactinomycin, DaunoXome, Decadron, Decadron-Phosphat, Delestrogen, Denileukin Diftitox, Depomedrol, Deslorelin, Dexrazoxan, Diethylstilbestrol, Diflucan, Docetaxel, Doxifluridin, Doxorubicin, Dronabinol, DW-166HC, Eligard, Elitek, Ellence, Emend, Epirubicin, Epoetin-alfa, Epogen, Eptaplatin, Ergamisol, Estrace, Estradiol, Estramustin-Natriumphosphat, Ethinylestradiol, Ethyol, Etidronsäure, Etopophos, Etoposid, Fadrozol, Farston, Filgrastim, Finasterid, Fligrastim, Floxuridin, . Fluconazol, Fludarabin, 5-Fiuordeoxyuridin-Monophosphat, 5-Fluoruracil (5-FU), Fluoxymesteron, Flutamid, Formestan, Fosteabin, Fotemustin, Fulvestrant, Gammagard, Gemcitabin, Gemtuzumab, Gleevec, Gliadel, Goserelin, Granisetron-Hydrochlorid, Histrelin, Hycamtin, Hydrocorton, erythro-Hydroxynonyladenin, Hydroxyharnstoff, Ibritumomab Tiuxetan, Idarubicin, Ifosfamid, Interferon-alpha, Interferon-alpha-2, Interferon-alpha-2α, Interferon-alpha-2β, Interferon-alpha-nl, Interferon-alpha-n3, Interferon-beta, Interferon-gamma-1α, Interleukin-2, Intron A, Iressa, Irinotecan, Kytril, Lentinan-Sulfat, Letrozol, Leucovorin, Leuprolid, Leuprolid-Acetat, Levamisol, Levofolinsäure-Calciumsalz, Levothroid, Levoxyl, Lomustin, Lonidamin, Marinol, Mechlorethamin, Mecobalamin, Medroxyprogesteron-Acetat, Megestrol-Acetat, Melphalan, Menest, 6-Mercaptopurin, Mesna, Methotrexat, Metvix, Miltefosin, Minocyclin, Mitomycin C, Mitotan, Mitoxantron, Modrenal, Myocet, Nedaplatin, Neulasta, Neumega, Neupogen, Nilutamid, Nolvadex, NSC-631570, OCT-43, Octreotid, Ondansetron-Hydrochlorid, Orapred, Oxaliplatin, Paclitaxel, Pediapred, Pegaspargase, Pegasys, Pentostatin, Picibanil, Pilocarpin-Hydrochlorid, Pirarubicin, Plicamycin, Porfimer-Natrium, Prednimustin, Prednisolon, Prednison, Premarin, Procarbazin, Procrit, Raltitrexed, Rebif, Rhenium-186-Etidronat, Rituximab, Roferon-A, Romurtid, Salagen, Sandostatin, Sargramostim, Semustin, Sizofiran, Sobuzoxan, Solu-Medrol, Streptozocin, Strontium-89-chlorid, Synthroid, Tamoxifen, Tamsulosin, Tasonermin, Tastolacton, Taxoter, Teceleukin, Temozolomid, Teniposid, Testosteron-Propionat, Testred, Thioguanin, Thiotepa, Thyrotropin, Tiludronsäure, Topotecan, Toremifen, Tositumomab, Tastuzumab, Teosulfan, Tretinoin, Trexall, Trimethylmelamin, Trimetrexat, Triptorelin-Acetat, Triptorelin-Pamoat, UFT, Uridin, Valrubicin, Vesnarinon, Vinblastin, Vincristin, Vindesin, Vinorelbin, Virulizin, Zinecard, Zinostatin-Stimalamer, Zofran; ABI-007, Acolbifen, Actimmun, Affinitak, Aminopterin, Arzoxifen, Asoprisnil, Atamestan, Atrasentan, Avastin, BAY 43-9006 (Sorafenib), CCI-779, CDC-501, Celebrex, Cetuximab, Crisnatol, Cyproteron-Acetat, Decitabin, DN-101, Doxorubicin-MTC, dSLIM, Dutasterid, Edotecarin, Eflomithin, Exatecan, Fenretinid, Histamin-Dihydrochlorid, Histrelin-Hydrogel-Implant, Holmium-166-DOTMP, Ibandronsäure, Interferon-gamma, Intron-PEG, Ixabepilon, Keyhole Limpet-Hemocyanin, L-651582, Lanreotid, Lasofoxifen, Libra, Lonafarnib, Miproxifen, Minodronat, MS-209, liposomales MTP-PE, MX-6, Nafarelin, Nemorubicin, Neovastat, Nolatrexed, Oblimersen, Onko-TCS, Osidem, Paclitaxel-Polyglutamat, Pamidronat-Dinatrium, PN-401, QS-21, Quazepam, R-1549, Raloxifen, Ranpimas, 13-cis-Retinsäure, Satraplatin, Seocalcitol, T-138067, Tarceva, Taxoprexin, Thymosin-alpha-1, Tiazofurin, Tipifamib, Tirapazamin, TLK-286, Toremifen, TransMID-107R, Valspodar, Vapreotid, Vatalanib, Verteporfin, Vinflunin, Z-100, Zoledronsäure, sowie Kombinationen hiervon.

In einer bevorzugten Ausführungsform können die Verbindungen der vorliegenden Erfindung mit anti-hyperproliferativen Agentien kombiniert werden, welche beispielhaft - ohne dass diese Aufzählung abschließend wäre - sein können:
Aminoglutethimid, L-Asparaginase, Azathioprin, 5-Azacytidin, Bleomycin, Busulfan, Carboplatin, Carmustin, Chlorambucil, Cisplatin, Colaspase, Cyclophosphamid, Cytarabin, Dacarbazin, Dactinomycin, Daunorubicin, Diethylstilbestrol, 2',2'-Difluordeoxycytidin, Docetaxel, Doxorubicin (Adriamycin), Epirubicin, Epothilon und seine Derivate, erythro-Hydroxynonyladenin, Ethinylestradiol, Etoposid, Fludarabin-Phosphat, 5-Fluordeoxyuridin, 5-Fluordeoxyuridin-Monophosphat, 5-Fluoruracil, Fluoxymesteron, Flutamid, Hexamethylmelamin, Hydroxyhamstoff, Hydroxyprogesteron-Caproat, Idarubicin, Ifosfamid, Interferon, Irinotecan, Leucovorin, Lomustin, Mechlorethamin, Medroxyprogesteron-Acetat, Megestrol-Acetat, Melphalan, 6-Mercaptopurin, Mesna, Methotrexat, Mitomycin C, Mitotan, Mitoxantron, Paclitaxel, Pentostatin, N-Phosphonoacetyl-L-aspartat (PALA), Plicamycin, Prednisolon, Prednison, Procarbazin, Raloxifen, Semustin, Streptozocin, Tamoxifen, Teniposid, Testosteron-Propionat, Thioguanin, Thiotepa, Topotecan, Trimethylmelamin, Uridin, Vinblastin, Vincristin, Vindesin und Vinorelbin.

In viel versprechender Weise lassen sich die erfindungsgemäßen Verbindungen auch mit biologischen Therapeutika wie Antikörpern (z.B. Avastin, Rituxan, Erbitux, Herceptin) kombinieren. Die erfindungsgemäßen Verbindungen können auch in Kombination mit gegen die Angiogenese gerichteten Therapien positive Effekte erzielen, wie zum Beispiel mit Avastin, Axitinib, Recentin, Regorafenib, Sorafenib oder Sunitinib. Kombinationen mit Inhibitoren des Proteasoms und von mTOR sowie Kombinationen mit Antihormonen und steroidalen metabolischen Enzyminhibitoren sind wegen ihres günstigen Nebenwirkungsprofils ebenfalls besonders geeignet.

Generell können mit der Kombination von Verbindungen der vorliegenden Erfindung mit anderen, zytostatisch öder zytotoxisch wirksamen Agentien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfin-dungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindung-gemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (bei-spielsweise mikrokristalline Cellulose, Lactose, Mannitol), Lösungsmittel (z.B. flüssige Polyethylenglycole), Emulgatoren und. Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- abs.: absolut
- Ac: Acetyl
- aq.: wässrig, wässrige Lösung
- Boc: *tert*.-Butoxycarbonyl
- br.: breit (bei NMR)
- Bsp.: Beispiel
- *ca.*: *circa,* ungefähr
- CI: chemische Ionisation (bei MS)
- d: Dublett (bei NMR)
- d: Tag(e)
- DC: Dünnschichtchromatographie
- DCI: direkte chemische Ionisation (bei MS)
- dd: Dublett von Dublett (bei NMR)
- DMAP: 4-*N,N*-Dimethylaminopyridin
- DME: 1,2-Dimethoxyethan
- DMF: *N,N*-Dimethylformamid
- DMSO: Dimethylsulfoxid
- DPBS: Dulbecco's Phosphat-gepufferte Salz-Lösung
- dt: Dublett von Triplett (bei NMR)
- d. Th.: der Theorie (bei chemischer Ausbeute)
- EDC: *N*'-(3-Dimethylaminopropyl)-*N*-ethylcarbodiimid-Hydrochlorid
- EI: Elektronenstoß-Ionisation (bei MS)
- eq.: Äquivalent(e)
- ESI: Elektrospray-Ionisation (bei MS)
- FCS: fötales Kälberserum
- Fmoc: (9*H*-Fluoren-9-ylmethoxy)carbonyl
- ges.: gesättigt
- GTP: Guanosin-5'-triphosphat
- h: Stunde(n)
- HATU: *O*-(7-Azabenzotriazol-1-yl)-*N,N,N',N*'-tetramethyluronium-hexafluorophosphat
- HEPES: 4-(2-Hydroxyethyl)piperazin-1-ethansulfonsäure
- HOAc: Essigsäure
- HOBt: 1-Hydroxy-1*H*-benzotriazol-Hydrat
- HOSu: *N-*Hydroxysuccinimid
- HPLC: Hochdruck-, Hochldistungsflüssigchromatographie
- konz.: konzentriert
- LC-MS: Flüssigchromatographie-gekoppelte Massenspektrometrie
- m: Multiplett (bei NMR)
- min: Minute(n)
- MS: Massenspektrometrie
- MTT: 3-(4,5-Dimethylthiazol-2-yl)-2,5-diphenyl-2*H*-tetrazoliumbromid
- NMM: *N*-Methylmorpholin
- NMP: *N-*Methyl-2-pyrrolidinon
- NMR: Kernresonanzspektrometrie
- PBS: Phosphat-gepufferte Salz-Lösung
- Pd/C: Palladium auf Aktivkohle
- quant.: quantitativ (bei Ausbeute)
- quart: Quartett (bei NMR)
- quint: Quintett (bei NMR)
- R_{f}: Retentionsindex (bei DC)
- RT: Raumtemperatur
- Rₜ: Retentionszeit (bei HPLC)
- s: Singulett (bei NMR)
- t: Triplett (bei NMR)
- *tert.*: tertiär
- TFA: Trifluoressigsäure
- THF: Tetrahydrofuran
- UV: Ultraviolett-Spektrometrie
- v/v: Volumen zu Volumen-Verhältnis (einer Lösung)
- Z: Benzyloxycarbonyl
- zus.: zusammen

### HPLC- und LC-MS-Methoden:

### Methode 1 (LC-MS):

Instrument: Waters Acquity SQD UPLC System; Säule: Waters Acquity UPLC HSS T3 1.8µ 50 mm x 1 mm; Eluent A: 1 1 Wasser + 0.25 ml 99%-ige Ameisensäure, Eluent B: 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 2 (LC-MS):

Instrument: Micromass QuattroPremier mit Waters UPLC Acquity; Säule: Thermo Hypersil GOLD 1.9µ 50 mm x 1 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 3 (LC-MS):

Instrument: Micromass Quattro Micro MS mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Methode 4 (LC-MS):

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 5 (HPLC):

Gerät: HP 1090 Serie II; Säule: Merck Chromolith SpeedROD RP-18e, 50 mm x 4.6 mm; Vorsäule: Merck Chromolith Guard Cartridge Kit RP-18e, 5 mm x 4.6 mm; Injektionsvolumen: 5 µl; Eluent A: 70% HClO₄ in Wasser (4 ml/Liter), Eluent B: Acetonitril; Gradient: 0.00 min 20% B → 0.50 min 20%. B → 3.00 min 90% B → 3.50 min 90% B → 3.51 min 20% B → 4.00 min 20% B; Fluss: 5 ml/min; Säulentemperatur: 40°C.

### Methode 6 (HPLC):

Gerät: Waters 2695 mit DAD 996; Säule: Merck Chromolith SpeedROD RP-18e, 50 mm x 4.6 mm; Vorsäule: Merck Chromolith Guard Cartridge Kit RP-18e, 5 mm x 4.6 mm; Eluent A: 70% HClO₄ in Wasser (4 ml/Liter), Eluent B: Acetonitril; Gradient: 0.00 min 5% B → 0.50 min 5% B → 3.00 min 95% B → 4.00 min 95% B; Fluss: 5 ml/min.

### Methode 7 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.1 min 100% A (Fluss 2.5 ml/min); Ofen: 55°C; Fluss: 2 ml/min; UV-Detektion: 210 nm.

### Methode 8 (LC-MS):

Gerätetyp MS: Waters ZQ; Gerätetyp HPLC: Agilent 1100 Series; UV DAD; Säule: Thermo Hypersil GOLD 3µ 20 mm x 4 mm; Eluent A: 1 wasser + 0.5 ml 50%-ige Ameisensäure, Eluent B: 1 1 Acetonitril + 0.5 ml 50%-ige Ameisensäure; Gradient: 0.0 min 100% A → 2.0 min 60% A → 2.3 min 40% A → 3.0 min 20% A → 4.0 min 10% A → 4.2 min 100% A (Fluss 2.5 ml/min); Ofen: 55°C; Fluss: 2 ml/min; UV-Detektion: 210 nm.

### Methode 9 (LC-MS):

Instrument: Waters Acquity SQD UPLC System; Säule: Waters Acquity UPLC HSS T3 1.8µ 50 mm x 1 mm; Eluent A: 1 1 Wasser + 0.25 ml 99%-ige Ameisensäure, Eluent B: 1 Acetonitril + 0.25 ml 99%-ige Ameisensäure; Gradient: 0.0 min 95% A → 6.0 min 5% A → 7.5 min 5% A; Offen: 50°C; Fluss: 0.35 ml/min; UV-Detektion: 210-400 nm.

### Methode 10 (HPLC):

Gerät: Agilent 1200 Series; Säule: Agilent Eclipse XDB-C18 5µ 4.6 mm x 150 mm; Vorsäule: Phenomenex KrudKatcher Disposable Pre-Column; Injektionsvolumen: 5 µl; Eluent A: 1 1 Wasser + 0.01% Trifluoressigsäure; Eluent B: 1 1 Acetonitril + 0.01% Trifluoressigsäure; Gradient: 0.00 min 10% B → 1.00 min 10% B → 1.50 min 90% B → 5.5 min 10% B; Fluss: 2 ml/min; Säulentemperatur: 30°C.

### Methode 11 (LC-MS):

Instrument: Waters Acquity SQD UPLC System; Säule: Waters Acquity UPLC HSS T3 1.8µ 30 mm x 2 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.60 ml/min; Ofen: 50°C; UV-Detektion: 208-400 nm.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Ausgangsverbindung 1

### (2R,3R)-3-[(2S)-1-(tert-Butoxycarbonyl)pyrrolidin-2-yl]-3-methoxy-2-methylpropansäure (Boc-Dolaproin) Dicyclohexylamin-Salz

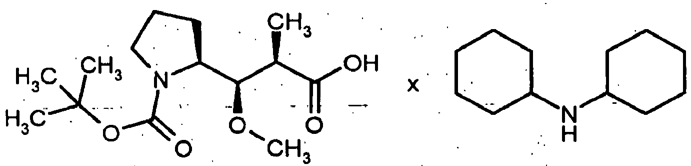

Die Titelverbindung kann auf verschiedenen Wegen nach Literaturvorschriften hergestellt werden, siehe z.B. Pettit et al., Synthesis 1996, 719; Shioiri et al., Tetrahedron Lett. 1991, 32, 931; Shioiri et al., Tetrahedron 1993, 49, 1913; Koga et al., Tetrahedron Lett. 1991, 32, 2395; Vidal et al., Tetrahedron 2004, 60, 9715; Poncet et al., Tetrahedron 1994, 50, 5345. Sie wurde hier entsprechend der Vorschrift von Shioiri et al. (Tetrahedron Lett. 1991, 32, 931) synthetisiert.

### Ausgangsverbindung 2

### tert.-Butyl-(3R,4S,5S)-3-methoxy-5-methyl-4-(methylamino)heptanoat-Hydrochlorid (Dolaisoleucin-OtBu x HCl)

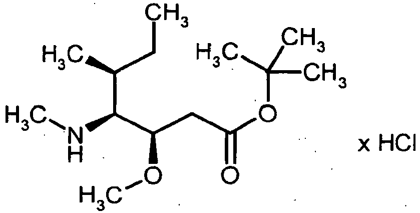

Die Titelverbindung kann auf verschiedenen Wegen nach Literaturvorschriften hergestellt werden, siehe z.B. Pettit et al., J. Org. Chem. 1994, 59, 1796; Koga et al., Tetrahedron Lett. 1991, 32, 2395; Shioiri et al., Tetrahedron Lett. 1991, 32, 931; Shioiri et al., Tetrahedron 1993, 49, 1913. Sie wurde hier entsprechend der Vorschrift von Koga et al. (Tetrahedron Lett. 1991, 32, 2395) synthetisiert.

### Intermediat 1

### tert.-Butyl-(3R,4S,5S)-4-[{N-[(benzyloxy)carbonyl]-L-valyl}(methyl)amino]-3-methoxy-5-methyl-heptanoat

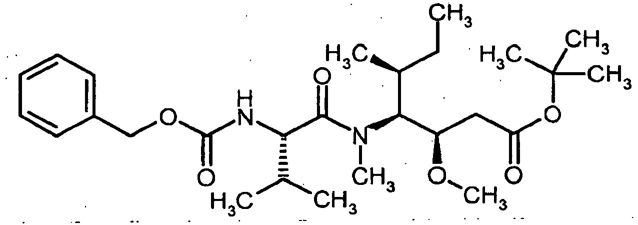

425 mg (1.7 mmol) *N*-[(Benzyloxy)carbonyl]-L-valin wurden in 50 ml DMF gelöst und nacheinander mit 500 mg (1.7 mmol) *tert*.-Butyl-(3*R*,4*S*,5*S*)-3-methoxy-5-methyl-4-(methylamino)-heptanoat-Hydrochlorid (Ausgangsverbindung 2), 356 mg (1.9 mmol) 1-(3-Dimethylaminopropyl)-3-ethylcarbodiimid-Hydrochlorid, 285 mg (1.9 mmol) 1-Hydroxy-1*H*-benzotriazol-Hydrat sowie 655 mg (5.1 mmol) *N,N-*Diisopropylethylamin versetzt. Die Mischung wurde 20 h bei RT gerührt. Anschließend wurden nochmals 142 mg (0.5 mmol) *N*-[(Benzyloxy)carbonyl]-L-valin, 119 mg (0.6 mmol) 1-(3-Dimethylaminopropyl)-3-ethylcarbodiimid-Hydrochlorid, 95 mg (0.6 mmol) 1-Hydroxy-1*H-*benzotriazol-Hydrat sowie 218 mg (1.7 mmol) *N,N-*Diisopropylethylamin zugesetzt und die Mischung 90 min lang mit Ultraschall behandelt. Der Ansatz wurde dann in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt.

Der Rückstand wurde durch präparative HPLC gereinigt. Es wurden 329 mg (40% d. Th.) der Titelverbindung als farbloses Öl erhalten.

HPLC (Methode 5): Rₜ = 2.5 min;

LC-MS (Methode 1): Rₜ = 1.45 min; MS (ESIpos): m/z = 493 (M+H)⁺.

### Intermediat 2

### tert.-Butyl-(3R,4S,5S)-3-methoxy-5-methyl-4-[methyl(L-valyl)amino]heptanoat

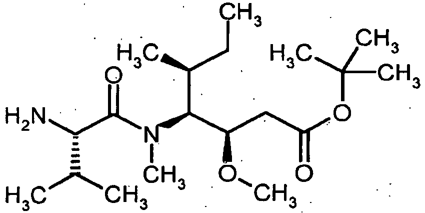

500 mg (1 mmol) *tert*.-Butyl-(3*R*,4*S*,5*S*)-4-[{*N-*[(benzyloxy)carbonyl]-L-valyl)(methyl)amino]-3-methoxy-5-methylheptanoat (Intermediat 1) wurden in 50 ml Methanol gelöst und nach Zugabe von 100 mg 10%-igem Palladium auf Aktivkohle 1 h lang bei RT unter Normaldruck hydriert. Der Katalysator wurde danach abfiltriert und das Lösungsmittel im Vakuum entfernt. Man erhielt 370 mg (quant.) der Titelverbindung als nahezu farbloses Öl.

HPLC (Methode 5): Rₜ= 1.59 min;.

LC-MS (Methode 1): Rₜ = 0.74 min; MS (ESIpos):m/z = 359 (M+H)⁺.

### Intermediat 3

### N-[(9H-Fluoren-9-ylmethoxy)caibonyl]-N-methyl-L-valyl-N-[(3R,4S,5S)-1-tert.-butoxy-3-methoxy-5-methyl-1-oxoheptan-4-yl]-N-methyl-L-valinamid

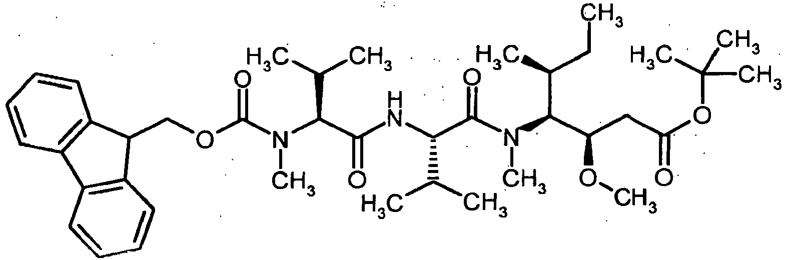

396 mg (1.1 mmol) *N-*[(9*H*-Fluoren-9-ylmethoxy)carbonyl]-*N-*methyl-L-valin wurden in 20 ml DMF gelöst und nacheinander mit 365 mg (1 mmol) *tert*.-Butyl-(3*R*,4*S*,5*S*)-3-methoxy-5-methyl-4-[methyl(L-valyl)amino]heptanoat (Intermediat 2), 234 mg (1.2 mmol) 1-(3-Dimethylaminopropyl)-3-ethylcarbodiimid-Hydrochlorid sowie 187 mg (1.2 mmol) 1-Hydroxy-1*H-*benzotriazol-Hydrat versetzt. Die Mischung wurde über Nacht bei RT gerührt. Der Ansatz wurde danach in eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und gesättigter Natriumchlorid-Lösung gewaschen, über Magnesiumsulfat getrocknet, filtriert und eingeengt. Der Rückstand wurde direkt, ohne weitere Reinigung, in der nächsten Stufe eingesetzt.

Ausbeute: 660 mg (68% d. Th.)

HPLC (Methode 5): Rₜ= 3.0 min;

LC-MS (Methode 1): Rₜ=1.61 min; MS (ESIpos): m/z = 694 (M+H)⁺.

### Intermediat 4

### N-[(9H-Fluoren-9-ylmethoxy)carbonyl]-N-methyl-L-valyl-N-[(2R,3S,4S)-1-carboxy-2-methoxy-4-methylhexan-3-yl]-N-methyl-L-valinamid

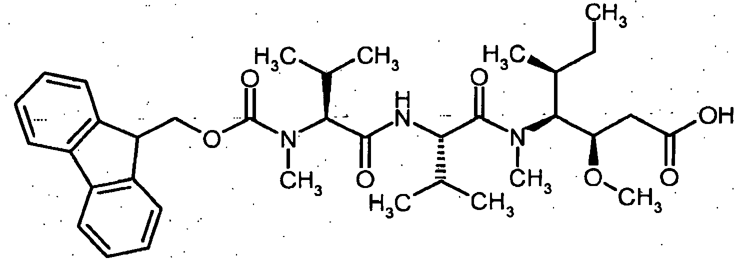

650 mg (0.94 mmol) *N*-[(9*H*-Fluoren-9-ylmethoxy)carbonyl]-*N*-methyl-L-valyl-*N*-[(3*R*,4*S*,5*S*)-1-*tert.*-butoxy-3-methoxy-5-methyl-1-oxoheptan-4-yl]-*N*-methyl-L-valinamid (Intermediat 3) wurden in 5 ml Dichlormethan aufgenommen, mit 5 ml Trifluoressigsäure versetzt und über Nacht bei RT gerührt. Anschließend wurde im Vakuum eingeengt und der verbliebene Rückstand durch präparative HPLC gereinigt. Es wurden 430 mg (72% d. Th) der Titelverbindung als farbloser Schaum erhalten.

HPLC (Methode 5): Rₜ = 2.4 min;

LC-MS (Methode 2): Rₜ= 1.51 min; MS (ESIpos): m/z = 638 (M+H)⁺.

### Intermediat 5

### N-(tert.-Butoxycarbonyl)-N-methyl-L-valyl-N-[(2R,3S,4S)-1-carboxy-2-methoxy-4-methylhexan-3-yl]-N-methyl-L-valinamid

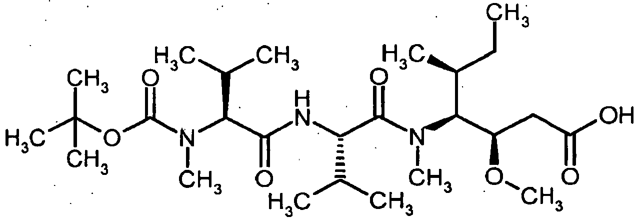

51 mg (0.08 mmol) *N-*[(9*H-*Fluoren-9-ylmethoxy)carbonyl]-*N-*methyl-L-valyl-*N-*[(2*R*,3*S*,4*S*)-1-carboxy-2-methoxy-4-methylhexan-3-yl]-*N*-methyl-L-valinamid (Intermediat 4) wurden in 10 ml DMF gelöst und mit 0.5 ml Piperidin versetzt. Nach 10 min Rühren bei RT wurde der Ansatz im Vakuum eingeengt und der Rückstand mit Diethylether verrührt. Die unlöslichen Bestandteile wurden abfiltriert und mehrfach mit Diethylether gewaschen. Dann wurde der Filter-Rückstand in 5 ml Dioxan/Wasser (1:1) aufgenommen und die Lösung mit 1 N Natronlauge auf pH 11 eingestellt. Unter Ultraschallbehandlung wurden in mehreren Portionen insgesamt 349 mg (1.6 mmol) *Di-tert.-*butyldicarbonat zugegeben, wobei der pH-Wert der Lösung bei 11 gehalten wurde. Nach Beendigung der Reaktion wurde das Dioxan abgedampft und die wässrige Lösung mit Zitronensäure auf einen pH-Wert von 2-3 eingestellt. Man extrahierte zweimal mit je 50 ml Ethylacetat. Die organischen Phasen wurden vereinigt, über Magnesiumsulfat getrocknet und im Vakuum eingeengt. Der Rückstand wurde in Diethylether aufgenommen und das Produkt mit Pentan ausgefällt. Durch Dekantieren wurde das Lösungsmittel abgetrennt. Der Rückstand wurde noch mehrmals mit Pentan digeriert und schließlich im Hochvakuum getrocknet. Es wurden so 31 mg (93% d. Th.) der Titelverbindung erhalten.

HPLC (Methode 6): Rₜ = 2.2 min;

LC-MS (Methode 2): Rₜ = 1.32 min; MS (ESIpos): m/z = 516 (M+H)⁺.

### Intermediat 6

### N-[(9H-Fluoren-9-ylmethoxy)carbonyl]-N-methyl-L-valyl-N-[(3R,4S,5S)-1-{(25)-2-[(1R,2R)-2-carboxy-1-methoxypropyl]pyrrolidin-1-yl}-3-methoxy-5-methyl-1-oxoheptan-4-yl]-N-methyl-L-valinamid

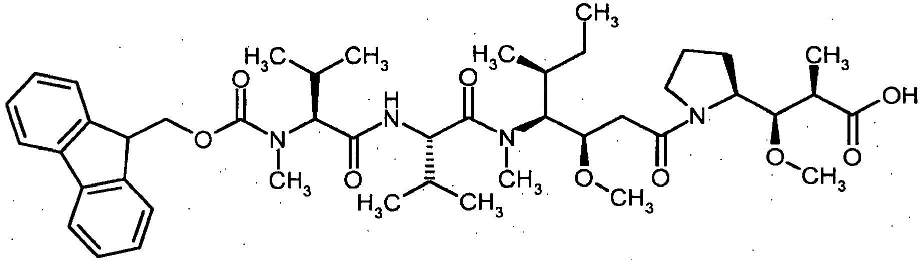

315 mg (0.494 mmol) *N*-[(9*H*-Fluoren-9-ylmethoxy)carbonyl]-*N*-methyl-L-valyl-*N*-[(2*R*,3*S*,4*S*)-1-carboxy-2-methoxy-4-methylhexan-3-yl]-*N-*methyl-L-valinamid (Intermediat 4) wurden in 12 ml DMF gelöst, mit 104 mg (0.543 mmol) 1-(3-Dimethylaminopropyl)-3-ethylcarbodiimid-Hydrochlorid sowie 83 mg (0.543 mmol) 1-Hydroxy-1*H*-benzotriazol-Hydrat versetzt und 90 min bei RT gerührt. Anschließend wurden 112 µl *N*,*N*-Diisopropylethylamin sowie 149 mg (0.494 mmol) (2*R*,3*R*)-3-Methoxy-2-methyl-3-[(2*S*)-pyrrolidin-2-yl]propansäure Trifluoressigsäure-Salz, welches zuvor aus der Ausgangsverbindung 1 durch Abspaltung der Boc-Schutzgruppe mittels Trifluoressigsäure hergestellt worden war, hinzugegeben. Die Mischung wurde 2 h bei RT gerührt und anschließend im Hochvakuum eingeengt. Der verbliebene Rückstand wurde durch zweimalige präparative HPLC aufgereinigt. Es wurden 140 mg (35% d. Th.) der Titelverbindung in Form eines farblosen Schaumes erhalten.

HPLC (Methode 5): Rₜ = 2.4 min;

LC-MS (Methode 1): Rₜ=1.38 min; MS (ESIpos): m/z = 807 (M+H)⁺.

### Intermediat 7

### Benzyl-(βS)-N-{(2R,3R)-3-methoxy-2-methyl-3-[(2S)-pyrrolidin-2-yl]propanoyl)-β-methyl-L-phenylalaninat Trifluoressigsäure-Salz

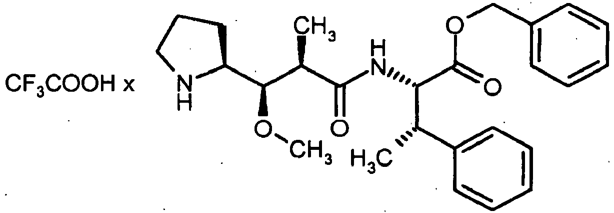

Zunächst wurde (2*R*,3*R*)-3-[(2*S*)-1-(*tert*.-Butoxycarbonyl)pyrrolidin-2-yl]-3-methoxy-2-methylpropansäure aus 351 mg (0.75 mmol) des Dicyclohexylamin-Salzes (Ausgangsverbindung 1) durch Aufnehmen in Ethylacetat und Ausschütteln mit wässriger Kaliumhydrogensulfat-Lösung freigesetzt. Die organische Phase wurde über Magnesiumsulfat getrocknet, filtriert und eingeengt. Der Rückstand wurde in 10 ml DMF aufgenommen und nacheinander mit 373 mg (0.75 mmol) Benzyl(βS)-β-methyl-L-phenylalaninat Trifluoressigsäure-Salz [hergestellt aus kommerziell erhältlichem (β*S*)-*N-*(*tert*.-Butoxycarbonyl)-β-methyl-L-phenylalanin durch EDC/DMAP-vermittelte Veresterung mit Benzylalkohol und nachfolgende Abspaltung der Boc-Schutzgruppe mit Trifluoressigsäure], 428 mg (1.125 mmol) *O*-(7-Azabenzotriazol-1-yl)-*N*,*N,N',N*'-tetramethyluronium-hexafluorophosphat (HATU) sowie 392 ul *N,N*-Diisopropylethylamin versetzt. Die Mischung wurde 20 h bei RT gerührt. Der Ansatz wurde dann auf eine Mischung aus halbgesättigter wässriger Ammoniumchlorid-Lösung und Ethylacetat gegossen. Die organische Phase wurde abgetrennt, nacheinander mit gesättigter Natriumhydrogencarbonat-Lösung und Natriumchlorid-Lösung gewaschen und anschließend eingeengt. Der Rückstand wurde mittels präparativer HPLC gereinigt. Man erhielt so 230 mg (57% d. Th.) des Boc-geschützten Intermediats Benzyl-(βS)-*N-*{(2*R*,3*R*)-3-[(2*S*)-1-(*tert*.-butoxycarbonyl)pyrrolidin-2-yl]-3-methoxy-2-methylpropanoyl}-β-methyl-L-phenylalaninat.

HPLC (Methode 6): Rₜ = 2.3 min;

LC-MS (Methode 1): Rₜ = 1:36 min; MS (ESIpos): m/z = 539 (M+H)⁺.

230 mg (0.42 mmol) dieses Intermediats wurden in 5 ml Dichlormethan aufgenommen, mit 5 ml Trifluoressigsäure versetzt und 30 min bei RT gerührt. Anschließend wurde im Vakuum eingeengt. Der verbliebene Rückstand wurde im Vakuum weiter getrocknet und dann aus Acetonitril/Wasser lyophilisiert. Auf diese Weise wurden 230 mg (quant.) der Titelverbindung erhalten.

HPLC (Methode 6): Rₜ = 1.6 min.

### Intermediat 8

### Benzyl-N-{(2R,3R)-3-methoxy-2-methyl-3-[(2S)-pyrrolidin-2-yl]propanoyl}-L-phenylalaninat Trifluoressigsäure-Salz

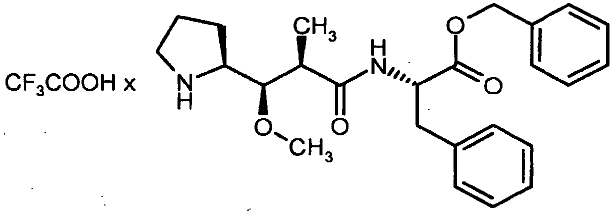

Die Titelverbindung wurde in Analogie zur Synthese von Intermediat 7 aus der Ausgangsverbindung 1 und Benzyl-L-phenylalaninat hergestellt.

HPLC (Methode 5): Rₜ = 1.6 min;

LC-MS (Methode 1): Rₜ = 0.85 min; MS (ESIpos): m/z = 425 (M+H)⁺.

### Intermediat 9

### N-Benzyl-N-methyl-L-phenylalaninamid Trifluoressigsäure-Salz

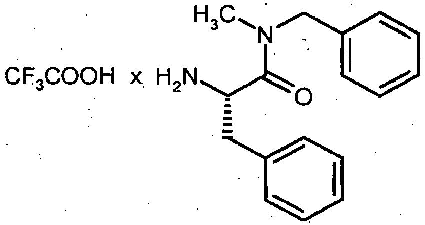

1000 mg (3.77 mmol) *N-*(*tert*.-Butoxycarbonyl)-L-phenylalanin wurden in 10 ml DMF gelöst und mit 457 mg (3.77 mmol) *N-*Methylbenzylamin, 2150 mg (5.65 mmol) *O*-(7-Azabenzotriazol-1-yl)-*N*,*N*,*N'*,*N*'-tetramethyluronium-hexafluorophosphat und 657 µl *N,N-*Diisopropylethylamin versetzt. Der Ansatz wurde 30 min bei RT gerührt und anschließend im Vakuum eingeengt. Der Rückstand wurde in Dichlormethan aufgenommen und dreimal mit Wasser ausgeschüttelt. Die organische Phase wurde über Magnesiumsulfat getrocknet und eingeengt. Der Rückstand wurde durch FlashChromatographie an Kieselgel mit Petrolether/Ethylacetat 3:1 als Laufmittel gereinigt. Die Produktfraktionen wurden eingeengt und der Rückstand im Hochvakuum getrocknet. Man erhielt so 1110 mg (75% d. Th.) des Boc-geschützten Intermediats *N-*Benzyl-*N*^{α}-(*tert*.-butoxycarbonyl)-*N-*methyl-L-phenylalaninamid.

HPLC (Methode 5): Rₜ = 2.1 min;

LC-MS (Methode 1): Rₜ = 1.14 min; MS (ESIpos): m/z = 369 (M+H)⁺.

1108 mg (3.007 mmol) dieses Intermediats wurden in 30 ml Dichlormethan aufgenommen, mit 10 ml Trifluoressigsäure versetzt und 30 min bei RT gerührt. Anschließend wurde im Vakuum eingeengt, der verbliebene Rückstand mit Dichlormethan verrührt und das Lösungsmittel abdestilliert. Der Rückstand wurde noch zweimal mit Pentan verrührt, das Lösungsmittel jeweils wieder abdekantiert und das Produkt schließlich im Hochvakuum getrocknet. Es wurden so 1075 mg (93% d. Th.) der Titelverbindung als ein Harz erhalten.

HPLC (Methode 5): Rₜ = 1.6 min;

LC-MS (Methode 1): Rₜ = 0.6 min; MS (ESIpos): m/z = 269 (M+H)⁺.

### Intermediat 10

### N-Benzyl-N^{α}-{(2R,3R)-3-methoxy-2-methyl-3-[(2S)-pyrrolidin-2-yl]propanoyl}-N-methyl-L-phenylalaninamid Trifluoressigsäure-Salz

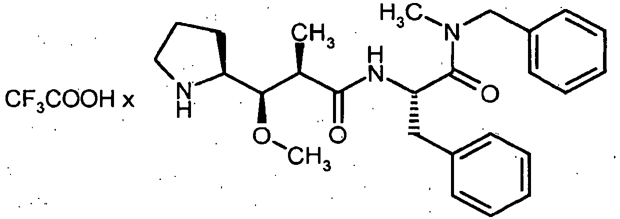

Zunächst wurde (2*R*,3*R*)-3-[(2*S)*-1-(*tert*.-Butoxycarbonyl)pyrrolidin-2-yl]-3-methoxy-2-methylpro-pansäure aus 141 mg (0.491 mmol) des Dicyclohexylamin-Salzes (Ausgarigsverbindung 1) durch Aufnehmen in Ethylacetat und Ausschütteln mit 5%-iger wässriger Schwefelsäure freigesetzt. Die organische Phase wurde über Magnesiumsulfat getrocknet, filtriert und eingeengt. Der Rückstand wurde in 10 ml DMF aufgenommen und mit 187.6 mg (0:49 mmol) *N-*Benzyl-*N-*methyl-L-phenylalaninamid Trifluoressigsäure-Salz (Intermediat 9), 190.3 mg (1.47 mmol) *O*-(7-Azabenzotriazol-1-yl)-*N*,*N*,*N',N*'-tetramethyluronium-hexafluorophosphat (HATU) sowie 256 µl *N*,*N-*Diisopropylethylamin versetzt. Die Mischung wurde 1 h bei RT gerührt. Der Ansatz wurde danach eingeengt, der Rückstand in Ethylacetat aufgenommen und die Lösung nacheinander mit gesättigter Ammoniumchlorid-Lösung, Natriumhydrogencarbonat-Lösung und Wasser ausgeschüttelt. Die organischen Phase wurde über Magnesiumsulfat getrocknet und eingeengt. Der Rückstand wurde durch FlashChromatographie an Kieselgel mit Acetonitril/Wasser 30:1 als Eluent gereinigt. Die Produktfraktionen wurden eingeengt und der Rückstand im Hochvakuum getrocknet. Man erhielt so 168 mg (64% d. Th.) des Boc-geschützten Intermediats *tert*.-Butyl-(2*S*)-2-[(1*R*,2*R*)-3-({(2*S*)-1-[benzyl-(methyl)amino]-1-oxo-3-phenylpropan-2-yl}amino)-1-methoxy-2-methyl-3-oxopropyl]pyrrolidin-1-carboxylat.

HPLC (Methode 5): Rₜ = 2.2 min;

LC-MS (Methode 2): Rₜ = 1.22 min; MS (ESIpos): m/z = 538 (M+H)⁺.

168 mg (0.312 mmol) dieses Intermediats wurden in 15 ml Dichlormethan aufgenommen, mit 3 ml Trifluoressigsäure versetzt und 30 min bei RT gerührt. Anschließend wurde im Vakuum eingeengt.

Der verbliebene Rückstand wurde zunächst mit Dichlormethan, dann mit Diethylether verrührt und das Lösungsmittel jeweils wieder abdestilliert. Nach Trocknen im Hochvakuum wurden 170 mg (99% d. Th.) der Titelverbindung als ein Harz erhalten.

HPLC (Methode 5): Rₜ = 1.7 min;

LC-MS (Methode 1): Rₜ = 0.73 min; MS (ESIpos): m/z = 438 (M+H)⁺.

### Intermediat 11

### Methyl-N-{(2R,3R)-3-inethoxy-2-methyl-3-[(2S)-pyrrolidin-2-yl]propanoyl}-L-phenylalaninat Trifluoressigsäure-Salz

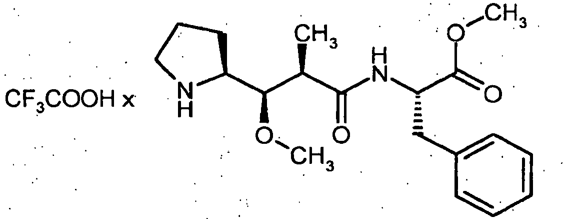

Die Titelverbindung wurde in Analogie zur Synthese von Intermediat 10 ausgehend von (2R,3R)-3-[(2*S*)-1-(*tert*.-Butoxycarbonyl)pyrrolidin-2-yl]-3-methoxy-2-methylpropansäure DicyclohexylaminSalz (Ausgangsverbindung 1) und Methyl-L-phenylalaninat-Hydrochlorid hergestellt.

HPLC (Methode 6): Rₜ = 0.6 min,

LC-MS (Methode 3): Rₜ = 1.17 min; MS (ESIpos): m/z = 349 (M+H)⁺.

### Intermediat 12.

### Benzyl-N-{(2R,3R)-3-methoxy-2-methyl-3-[(2S)-pyrrolidin-2-yl]propanoy]}-L-tryptophanat Trifluoressigsäure-Salz

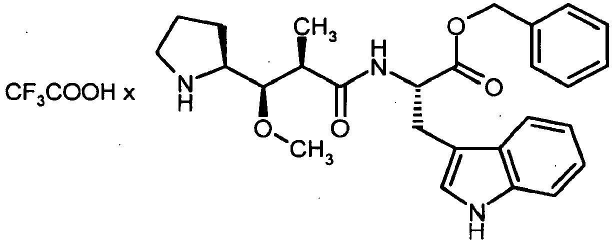

Die Titelverbindung wurde in Analogie zur Synthese von Intermediat 10 ausgehend von (2R,3R)-3-[(2*S*)-1-(*tert*.-Butoxycarbonyl)pyrrolidin-2-yl]-3-methoxy-2-methylpropansäure Dicyclohexylamin-Salz (Ausgangsverbindung 1) und Benzyl-L-tryptophanat hergestellt.

HPLC (Methode 5): Rₜ = 2.0 min;

LC-MS (Methode 1): Rₜ = 0.8 min; MS (ESIpos): m/z = 464 (M+H)⁺.

### Intermediat 13

### Benzyl-(1S,2R)-1-({(2R,3R)-3-methoxy-2-methyl-3-[(2S)-pyrrolidin-2-yl]propanoyl}amino)-2-phenylcyclopropancarboxylat Trifluoressigsäure-Salz

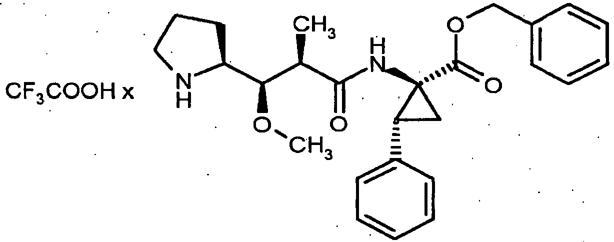

Die Titelverbindung wurde in Analogie zur Synthese von Intermediat 10 ausgehend von (2R,3R)-3-[(2*S*)-1-(*tert*.-Butoxycarbonyl)pyrrolidin-2-yl]-3-methoxy-2-methylpropansäure Dicyclohexylamin-Salz (Ausgangsverbindung 1) und Benzyl-(1*S*,2*R*)-1-amino-2-phenylcyclopropancarboxylat hergestellt. Letztere Verbindung wurde zuvor nach Standardmethoden durch Veresterung von kommerziell erhältlicher (1*S*,2*R*)-1-[(*tert.*-Butoxycarbonyl)amino]-2-phenylcyclopropancarbonsäure mit Benzylalkohol und anschließende Boc-Abspaltung mit Trifluoressigsäure hergestellt.

HPLC (Methode 6): Rₜ = 1.5 min;

LC-MS (Methode 2): Rₜ = 0.93 min; MS (ESIpos): m/z = 437 (M+H)⁺.

### Intermediat 14

### Benzyl-(2R,3R)-3-methoxy-2-methyl-3-[(2S)-pyrrolidin-2-yl]propanoat Trifluoressigsäure-Salz

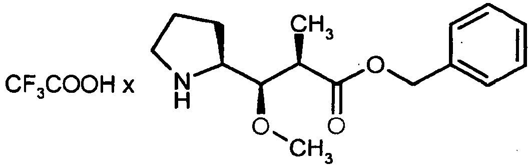

Zunächst wurde (2*R*,3*R*)-3-[(2*S*)-1-(*tert*.-Butoxycarbonyl)pyrrolidin-2-yl]-3-methoxy-2-methylpropansäure aus 1.82 g (3.88 mmol) des Dicyclohexylamin-Salzes (Ausgangsverbindung 1) durch Aufnehmen in 150 ml Ethylacetat und Ausschütteln mit 100 ml 0.5%-iger wässriger Schwefelsäure freigesetzt. Die organische Phase wurde über Magnesiumsulfat getrocknet, filtriert und eingeengt. Der Rückstand wurde in 10 ml Dioxan und 10 ml Wasser aufgenommen, mit 1517 mg (4.66 mmol) Cäsiumcarbonat versetzt und 5 min im Ultraschallbad behandelt. Anschließend wurde im Vakuum eingeengt und der Rückstand einmal mit DMF co-destilliert. Der Rückstand wurde dann in 15 ml DMF aufgenommen und mit 1990 mg (11.64 mmol) Benzylbromid versetzt. Die Mischung wurde 15 min im Ultraschallbad behandelt und anschließend im Vakuum eingeengt. Der Rückstand wurde zwischen Ethylacetat und Wasser verteilt. Die organische Phase wurde abgetrennt, mit gesättigter Natriumchlorid-Lösung gewaschen und danach eingeengt. Der Rückstand wurde schließlich durch präparative HPLC gereinigt. Man erhielt auf diese Weise 1170 mg (80% d. Th.) des Boc-geschützten Intermediats *tert.*-Butyl-(2*S*)-2-[(1*R*,2*R*)-3-(benzyloxy)-1-methoxy-2-methyl-3-oxopropyl]pyrrolidin-1-carboxylat.

Sofort anschließend wurden diese 1170 mg des Intermediats in 15 ml Dichlormethan aufgenommen und mit 5 ml Trifluoressigsäure versetzt. Nach 15 min Rühren bei RT wurde der Ansatz im Vakuum eingeengt und der Rückstand aus Dioxan lyophilisiert. Nach Trocknen im Hochvakuum verblieben 1333 mg (84% d. Th.) der Titelverbindung als gelbes Öl.

HPLC (Methode 5): Rₜ = 1.5 min;

LC-MS (Methode 1): Rₜ = 0.59 min; MS (ESIpos): m/z =278 (M+H)⁺.

### Intermediat 15

### N-(tert.-Butoxycarbonyl)-N-methyl-L-valyl-N-[(3R,4S,5S)-1-{(2S)-2-[(1R,2R)-2-carboxy-1-methoxypropyl]pyrrolidin-1-yl}-3-methoxy-5-methyl-1-oxoheptan-4-yl]-N-methyl-L-valinamid

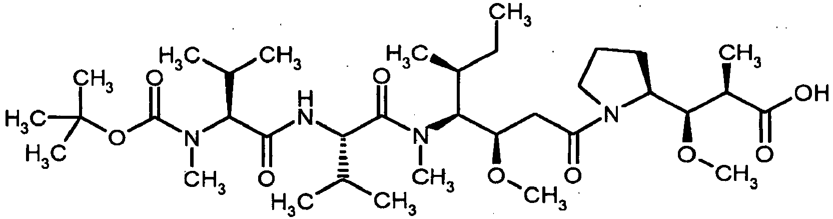

1200 mg (2.33 mmol) *N*-(*tert*.-Butoxycarbonyl)-*N*-methyl-L-valyl-*N*-[(2*R*,3*S*,4*S*)-1-carboxy-2-methoxy-4-methylhexan-3-yl]-*N*-methyl-L-valinamid (Intermediat 5) wurden mit 910.8 mg (2.33 mmol) Benzyl-(2*R*,3*R*)-3-methoxy-2-methyl-3-[(2*S*)-pyrrolidin-2-yl]propanoat Trifluoressigsäure-Salz (Intermediat 14), 1327 mg (3.49 mmol) *O*-(7-Azabenzotriazol-1-yl)-*N*,*N*,*N',*N'-tetramethyluroniumhexafluorophosphat und 2027 µl *N*,*N-*Diisopropylethylamin in 50 ml DMF zusammengegeben und 5 min bei RT gerührt. Danach wurde das Lösungsmittel im Vakuum entfernt. Der verbliebene Rückstand wurde in Ethylacetat aufgenommen und nacheinander mit 5%-iger wässriger Zitronensäure-Lösung und gesättigter Natriumhydrogencarbonat-Lösung ausgeschüttelt. Die organische Phase wurde abgetrennt und eingeengt. Der Rückstand wurde mittels präparativer HPLC gereinigt. Die Produktfraktionen wurden vereinigt, eingeengt und der Rückstand im Hochvakuum getrocknet. Es wurden so 1000 mg (55% d. Th.) des Benzylester-Intermediats *N-*(*tert*.-Butoxycarbonyl)-*N*-methyl-L-valyl-*N*-[(3*R*,4*S*,5*S*)-1-{(2*S*)-2-[(1*R*,2*R*)-3-(benzyloxy)-1-methoxy-2-methyl-3-oxopropyl]pyrrolidin-1-yl}-3-methoxy-5-methyl-1-oxoheptan-4-yl]-*N*-methyl-L-valinamid als ein Harz erhalten.

LC-MS (Methode 1): Rₜ = 1.56 min; MS (ESIpos): m/z = 775 (M+H)⁺.

Die gesamte erhaltene Menge dieses Intermediats wurde in 25 ml einer Mischung aus Methanol und Dichlormethan (20:1) aufgenommen und die Benzylester-Gruppe durch Hydrierung unter Formaldruck mit 10% Palladium auf Aktivkohle als Katalysator entfernt. Nach 30 min Rühren bei RT wurde der Katalysator abfiltriert und das Filtrat im Vakuum eingeengt. Man erhielt 803 mg (91% d. Th.) der Titelverbindung als weißen Feststoff.

HPLC (Methode 5): Rₜ = 2.1 min;

LC-MS (Methode 1): Rₜ = 1.24 min; MS (ESIpos): m/z = 685 (M+H)⁺.

### Intermediat 16

### (1S,2R)-1-Amino-2-phenyl-N-propylcyclopropancarboxamid Trifluoressigsäure-Sälz

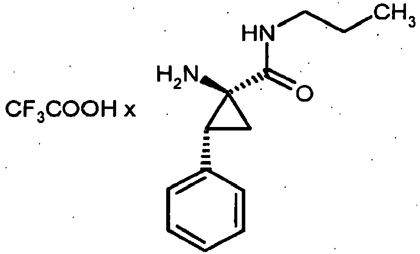

Die Titelverbindung wurde durch Kupplung von kommerziell erhältlicher (1*S*,2*R*)-1-[(*tert*.-Butoxy-carbonyl)amino]-2-phenylcyclopropancarbonsäure mit *n*-Propylamin in Gegenwart von *O*-(7-Azabenzotriazol-1-yl)-*N,N,N,N'*-tetramethyluronium-hexafluorophosphat (HATU) und nachfolgende Boc-Abspaltung mit Trifluoressigsäure hergestellt (Ausbeute 85% d. Th. über beide Stufen).

HPLC (Methode 5): Rₜ = 1.2 min;

LC-MS (Methode 1): Rₜ = 0.52 min; MS (ESIpos): m/z = 219 (M+H)⁺.

### Intermediat 17

### Ethyl-(1S,2R)-1-amino-2-phenylcyclopropancarboxylat Trifluoressigsäure-Salz

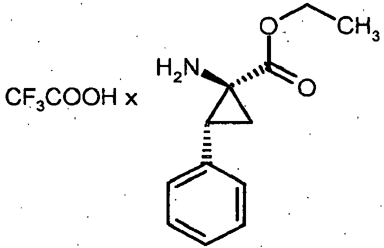

Die Titelverbindung wurde nach Standardmethoden durch Veresterung von kommerziell erhältlicher (1*S*,2*R*)-1-[(*tert*.-Butoxycarbonyl)amino]-2-phenylcyclopropancarbonsäure mit Ethanol und anschließende Boc-Abspaltung mit Trifluoressigsäure hergestellt.

LC-MS (Methode 1): Rₜ = 0.50 min; MS (ESIpos): m/z = 206 (M+H)⁺.

### Intermediat 18

### 1-Naphthylmetliyl-N-(tert.-butoxycarbonyl)-L-phenylalaninat

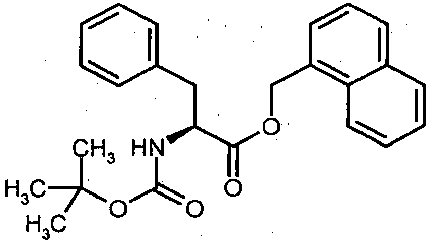

Zu einer Lösung von 500 mg (1.89 mmol) *N-*Boc-L-Phenylalanin in 25 ml Dichlormethan wurden bei RT 1192 mg (6.2 mmol) EDC, 578 µl (4.1 mmol) Triethylamin, 345 mg (2.8 mmol) DMAP und 328 mg (2.1 mmol) 1-Naphthylmethanol gegeben. Das Reaktionsgemisch wurde über Nacht gerührt, dann mit 50 ml Dichlormethan verdünnt und nacheinander mit 10%-iger wässriger Zitronensäure-Lösung, Wasser und gesättigter Kochsalz-Lösung gewaschen. Die organische Phase wurde über Magnesiumsulfat getrocknet, anschließend eingeengt und der Rückstand durch präparative HPLC gereinigt. Es wurden 501 mg (66% d. Th.) der Titelverbindung erhalten.

LC-MS (Methode 1): Rₜ = 1.33 min; m/z = 406 (M+H)⁺.

### Intermediat 19

### 1-Naphthylmethyl-L-phenylalaninat-Hydrochlorid

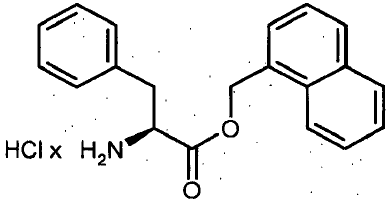

500 mg (1.2 mmol) 1-Naphthylmethyl-*N*-(*tert*.-butoxycarbonyl)-L-phenylalaninat (Intermediat 18) wurden in 20 ml einer 4 N Lösung von Chlorwasserstoff in Dioxan gelöst und 1 h bei RT gerührt. Anschließend wurde das Reaktionsgemisch eingeengt und der Rückstand im Vakuum getrocknet. Es wurden 421.5 mg (quant.) der Titelverbindung erhalten.

LC-MS (Methode 1): Rₜ = 0.80 min; m/z = 306 (M+H)⁺.

### Intermediat 20

### N-(tert.-Butoxycarbonyl)-N-methyl-L-valyl-N-[(3R,4S,5S)-3-methoxy-1-{(2S)-2-[(1R,2R)=1-methoxy-2-methyl-3-{[(2S)-1-(1-naphthylmethoxy)-1-oxo-3-phenylpropan-2-yl]amino}-3-oxo-propyl]pyrrolidin-1-yl}-5-methyl-1-oxoheptan-4-yl]-N-methyl-L-valinamid

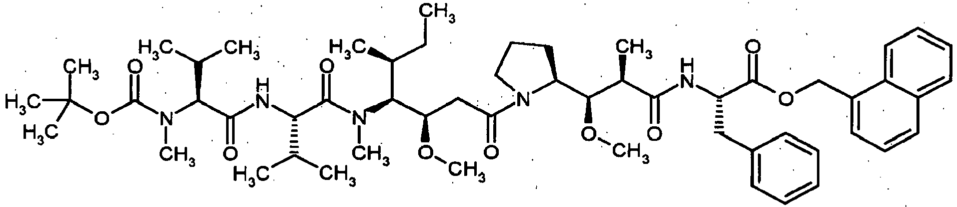

Zu einer Lösung von 20 mg (29 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N-*methyl-L-valinamid (Intermediat 15) in 1 ml DMF wurden bei RT 15.3 µl (88 µmol) *N***,***N-*Diisopropylethylamin, 6.7 mg (44 µmol) HOBt und 6.7 mg (35 µmol) EDC gegeben und die Mischung 30 min lang gerührt. Anschließend wurden 11 mg (32 µmol) 1-Naphthylmethyl-L-phenylalaninat-Hydrochlorid (Intermediat 19) hinzugefügt. Nach Rühren über Nacht wurde das Reaktionsgemisch direkt über präparative HPLC in seine Komponenten aufgetrennt. Es wurden 26.1 mg (92% d. Th.) der Titelverbindung erhalten.

LC-MS (Methode 1): Rₜ = 1.61 min; m/z = 973 (M+H)⁺.

### Intermediat 21

### N-Methyl-L-valyl-N-[(3R,4S,5S)-3-methoxy-1-{(25)-2-[(1R,2R)-1-methoxy-2-methyl-3-{[(2S)-1-(1-naphthylmethoxy)-1-oxo-3-phenylpropan-2-yl]amino}-3-oxopropyl]pyrrolidin-1-yl}-5-methyl-1-oxoheptan-4-yl]-N-methyl-L-valinamid Trifluoressigsäure-Salz

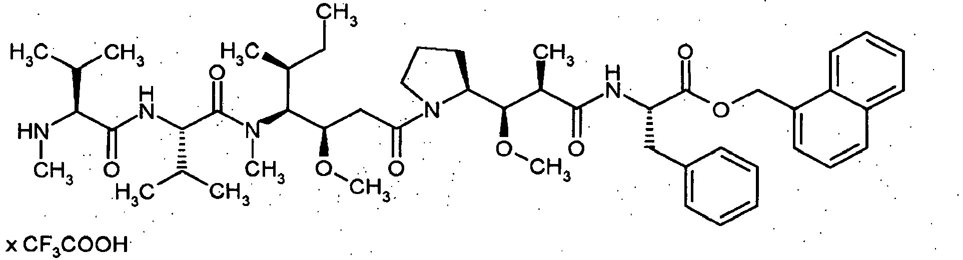

26 mg (27 µmol) *N-*(*tert*.-Butoxycarbonyl)-*N-*methyl-L-valyl-*N-*[(3*R*,4*S*,5*S)*-3-methoxy-1-{(2*S*)-2-[(1*R*,2*R*)-1-methoxy-2-methyl-3-{[(2*S*)-1-(1-naphthylmethoxy)-1-oxo-3-phenylpropan-2-yl]amino}-3-oxopropyl]pyrrolidin-1-yl}-5-methyl-1-oxoheptan-4-yl]-*N-*methyl-L-valinamid (Intermediat 20) wurden in 1 ml Dichlormethan gelöst und mit 0.2 ml TFA versetzt. Das Reaktionsgemisch wurde 30 min bei RT gerührt und anschließend eingeengt. Es wurden 26.3 mg (99.7% d. Th.) der Titelverbindung erhalten.

LC-MS (Methode 1): Rₜ = 1.02 min; m/z = 873 (M+H)⁺.

### Intermediat 22

### Adarnantan-1-yl-N-(tert.-butoxycarbonyl)-L-phenylalaninat

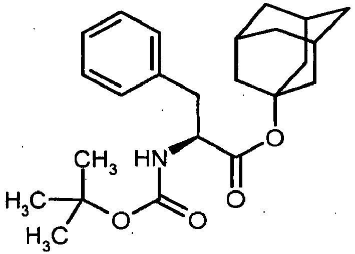

Zu einer Lösung von 500 mg (1.89 mmol) *N-*Boc-L-Phenylalanin in 25 ml Dichlormethan wurden bei RT 1192 mg (6.2 mmol) EDC, 578 µl (4.1 mmol) Triethylamin, 345 mg (2.8 mmol) DMAP und 316 mg (2.1 mmol) 1-Adamantanol gegeben. Das Reaktionsgemisch wurde über Nacht gerührt, dann mit 50 ml Dichlormethan verdünnt und nacheinander mit 10%-iger wässriger Zitronensäure-Lösung, Wasser und gesättigter Kochsalz-Lösung gewaschen. Die organische Phase wurde über Magnesiumsulfat getrocknet, anschließend eingeengt und der Rückstand durch präparative HPLC gereinigt. Es wurden 336 mg (43% d. Th.) der Titelverbindung erhalten.

LC-MS (Methode 1): Rₜ = 1.49 min; m/z = 400 (M+H)⁺.

### Intermediat 23

### Adamantan-1-yl-L-phenylalaninat-Hydrochlorid

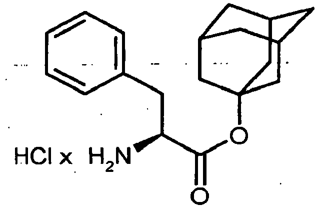

336 mg (840 µmol) Adamantan-1-yl-*N-*(*tert*.-butoxycarbonyl)-L-phenylalaninat (Intermediat 22) wurden in 12 ml einer 4 N Lösung von Chlorwasserstoff in Dioxan gelöst und 1 h bei RT gerührt. Anschließend wurde das Reaktionsgemisch eingeengt und der Rückstand über präparative HPLC gereinigt (Eluenten-Gradient Methanol/Wasser + 0.01% TFA). Es wurden 228 mg (81% d. Th.) der Titelverbindung erhalten.

LC-MS (Methode 2): Rₜ = 1.03 min; m/z = 300 (M+H)⁺.

### Intermediat 24

### N-(tert.-Butoxycarbonyl)-N-methyl-L-valyl-N-[(3R,4S,5S)-1-{(2S)-2-[(1R,2R)-3-{[(2S)-1-(adamantan-1-yloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

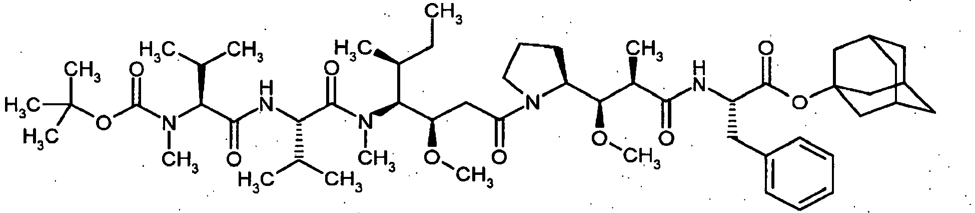

Zu einer Lösung von 20 mg (29 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) in 1 ml DMF wurden bei RT 15.3 µl (88 µmol) *N*,*N-*Diisopropylethylamin, 6.7 mg (44 µmol) HOBt und 6.7 mg (35 µmol) EDC gegeben und die Mischung 30. min lang gerührt. Anschließend wurden 9.6 mg (32 µmol) Adamantan-1-yl-L-phenylalaninat-Hydrochlorid (Intermediat 23) hinzugefügt. Nach Rühren über Nacht wurde das Reaktionsgemisch direkt über präparative HPLC in seine Komponenten aufgetrennt. Es wurden 15 mg (90% Reinheit, 48% d. Th.) der Titelverbindung erhalten.

LC-MS (Methode 1): Rₜ = 1.67 min; m/z = 967 (M+H)⁺.

### Intermediat 25

### N-Methyl-L-valyl-N-[(3R,4S,5S)-1-{(2S)-2-[(1R,2R)-3-{[(2S)-1-(adamantan-1-yloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

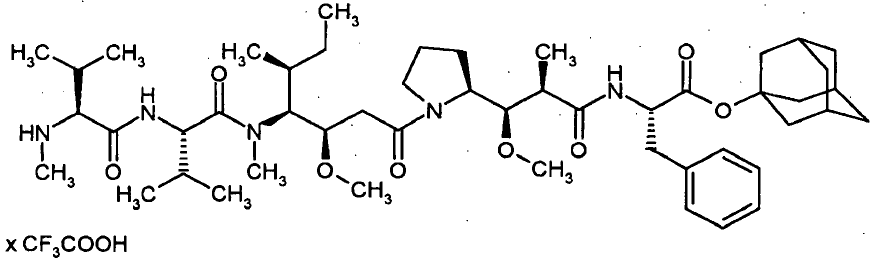

15 mg (16 µmol) *N-*(*tert*.-Butoxycarbonyl)-*N-*methyl-L-valyl-*N-*[(3*R*,4*S*,5*S)*-1-{(2*S*)-2-[(1*R*,2*R*)-3-{[(2*S*)-1-(adamantan-1-yloxy)-1-oxo-3-phenylpropan-2-yl]amino}-1-methoxy-2-methyl-3-oxo-propyl]pyrrolidin-1-yl}-3-methoxy-5-methyl-1-oxoheptan-4-yl]-*N*-methyl-L-valinamid (Intermediat 24) wurden in 1 ml Dichlormethan gelöst und mit 0.2 ml TFA versetzt. Das Reaktionsgemisch wurde 30 min bei RT gerührt und anschließend eingeengt. Das Rohprodukt wurde über präparative HPLC gereinigt. Es wurden 4.8 mg (32% d. Th.) der Titelverbindung erhalten.

LC-MS (Methode 1): Rₜ = 1.10 min; m/z = 867 (M+H)⁺.

### Intermediat 26

### Adamantan-1-ylmethyl-N-(tert.-butoxycarbonyl)-L-phenylalaninat

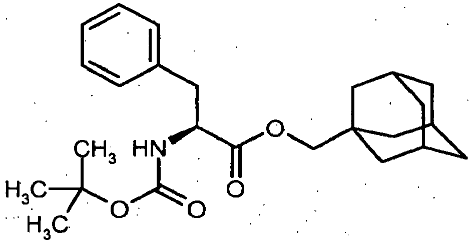

Zu einer Lösung von 500 mg (1.89 mmol) *N-*Boc-L-Phenylalanin in 25 ml Dichlormethan wurden bei RT 1192 mg (6.2 mmol) EDC, 578 µl (4.1 mmol) Triethylamin, 345 mg (2.8 mmol) DMAP und 345 mg (2.1 mmol) 1-Adamantylmethanol gegeben. Das Reaktionsgemisch wurde über Nacht gerührt, dann mit 50 ml Dichlormethan verdünnt und nacheinander mit 10%-iger wässriger Zitronensäure-Lösung, Wasser und gesättigter Kochsalz-Lösung gewaschen. Die organische Phase wurde über Magnesiumsulfat getrocknet, anschließend eingeengt und der Rückstand durch präparative HPLC gereinigt. Es wurden 769 mg (90% d. Th.) der Titelverbindung erhalten.

LC-MS (Methode 2): Rₜ = 1.84 min; m/z = 414 (M+H)⁺.

### Intermediat 27

### Adamantan-1-ylmethyl-L-phenylalaninat-Hydrochlorid

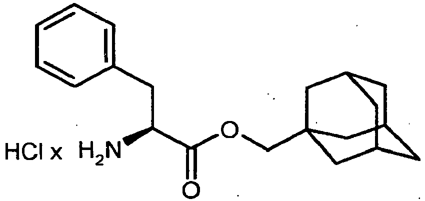

769 mg (1.86 mmol) Adamantan-1-ylmethyl-*N*-(*tert*.-butoxycarbonyl)-L-phenylalaninat (Intermediat 26) wurden in 25 ml einer 4 N Lösung von Chlorwasserstoff in Dioxan gelöst und 1 h bei RT gerührt. Anschließend wurde das Reaktionsgemisch eingeengt und der Rückstand im Vakuum getrocknet. Es wurden 619 mg (95% d. Th.) der Titelverbindung erhalten.

LC-MS (Methode 1): Rₜ = 0.82 min; m/z = 314 (M+H)⁺.

### Intermediat 28

### N-(tert.-Butoxycarbonyl)-N-methyl-L-valyl-N-[(3R,4S,5S)-1-{(2S)-2-[(1R,2R)-3-{[(2S)-1-(adamantan-1-ylmethoxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

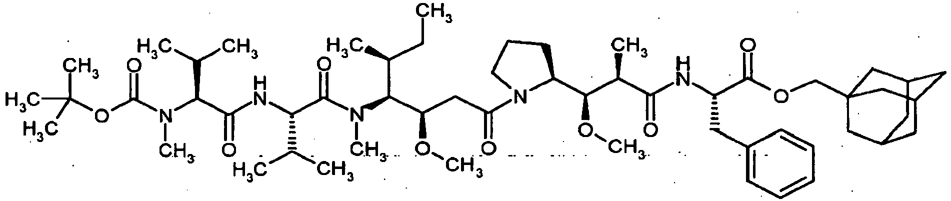

Zu einer Lösung von 20 mg (29 µmol) *N-(tert.-*Butoxycarbonyl)*-N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N-*methyl-L-valinamid (Intermediat 15) in 1 ml DMF wurden bei RT 15.3 µl (88 µmol) *N,N-*Diisopropylethylamin, 6.7 mg (44 µmol) HOBt und 6.7 mg (35 µmol) EDC gegeben und die Mischung 30 min lang gerührt. Anschließend wurden 10.1 mg (32 µmol) Adamantan-1-yl-L-phenylalaninat-Hydrochlorid (Intermediat 27) hinzugefügt. Nach Rühren über Nacht wurde das Reaktionsgemisch direkt über präparative HPLC in seine Komponenten aufgetrennt. Es wurden 27.5 mg (93% d. Th.) der Titelverbindung erhalten.

LC-MS (Methode 1): Rₜ = 1.70 min; m/z = 980 (M+H)⁺.

### Intermediat 29

### N-Methyl-L-valyl-N-[(3R,4S,5S)-1-{(2S)-2-[(1R,2R)-3-{[(2S)-1-(adamantan-1-ylmethoxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

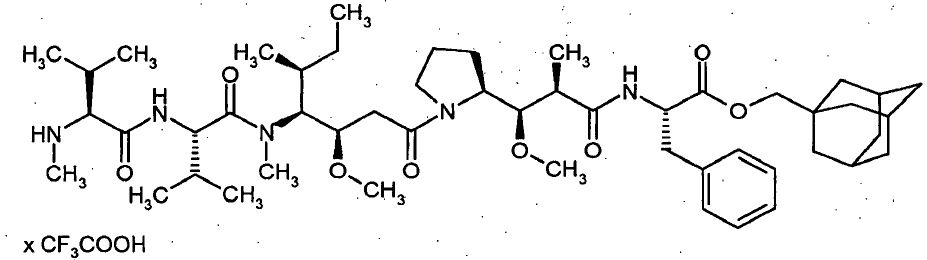

27.5 mg (28 µmol) *N-*(*tert*.-Butoxycarbonyl)-*N-*methyl-L-valyl-*N-*[(3*R*,4*S*,5*S)*-1-{(2*S)-*2-[(1*R*,2*R*)-3-{[(2*S*)-1-(adamantan-1-ylmethoxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N-*methyl-L-valinamid (Intermediat 28) wurden in 1.8 ml Dichlormethan gelöst und mit 361 µl TFA versetzt. Das Reaktionsgemisch wurde 30 min gerührt und dann eingeengt. Der Rückstand wurde in Wasser aufgenommen und lyophilisiert. Es wurden 22.7 mg (81% d. Th.) der Titelverbindung erhalten.

LC-MS (Methode 1): Rₜ = 1.14 min; m/z = 880 (M+H)⁺.

### Intermediat 30

### tert.-Butyl-[(2S)-1-(benzyloxy)-3-phenylpropan-2-yl]carbamat

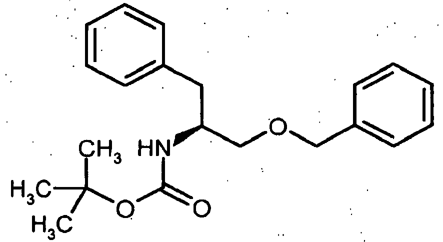

Unter Argonatmosphäre wurden 500 mg (1.99 mmol) *N-*Boc-L-Phenylalaninol in 5 ml DMF gelöst und auf 0°C gekühlt. Anschließend wurden 159 mg (3.98 mmol) einer 60%-igen Suspension von Natriumhydrid in Paraffinöl zugegeben. Das Reaktionsgemisch wurde bis zur Beendigung der Gasentwicklung gerührt und anschließend mit 260 µl (2.19 mmol) Benzylbromid versetzt. Das Kühlbad wurde entfernt und die Reaktionsmischung 2 h bei RT gerührt. Danach wurde der Ansatz eingeengt, der Rückstand in Eiswasser aufgenommen und das Gemisch mit Dichlormethan extrahiert. Die organische Phase wurde mit gesättigter Kochsalz-Lösung gewaschen, über Magnesiumsulfat getrocknet und eingeengt. Der Rückstand wurden mittels präparativer HPLC gereinigt. Es wurden 226 mg (33% d. Th.) der Titelverbindung erhalten.

LC-MS (Methode 1): Rₜ = 1.28 min; m/z = 342 (M+H)⁺.

### Intermediat 31

### (2S)-1-(Benzyloxy)-3-phenylpropan-2-amin-Hydrochlorid

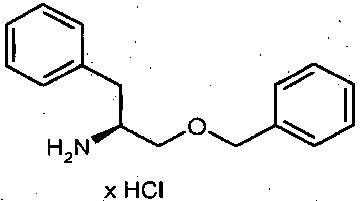

220 mg (644 µmol) *tert*.-Butyl-[(2*S)*-1-(benzyloxy)-3-phenylpropan-2-yl]carbamat (Intermediat 30) wurden in 11 ml einer 4 N Lösung von Chlorwasserstoff in Dioxan gelöst und 1 h bei RT gerührt. Dann wurde der Ansatz eingeengt und der Rückstand im Vakuum getrocknet. Es wurden 138 mg (77% d. Th.) der Titelverbindung erhalten.

LC-MS (Methode 1): Rₜ = 0.65 min; m/z = 242 (M+H)⁺.

### Intermediat 32

### N-(tert.-Butoxycarbonyl)-N-methyl-L-valyl-N-[(3R,4S,5S)-1-{(2S)-2-[(1R,2R)-3-{[(2S)-1-(benzyl-oxy)-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

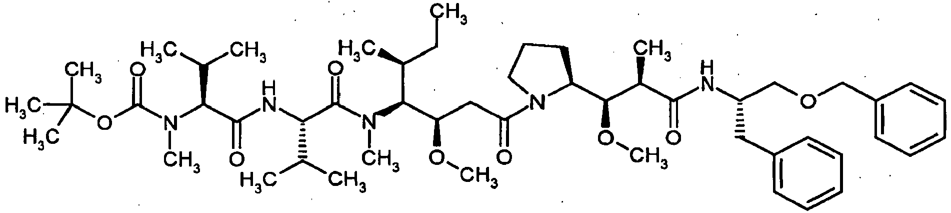

Zu einer Lösung von 20 mg (29 µmol) *N-*(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N-*methyl-L-valinamid (Intermediat 15) in 1 ml DMF wurden bei RT 15.3 µl (88 µmol) *N,N-*Diisopropylethylamin, 6.7 mg (44 µmol) HOBt und 6.7 mg (35 µmol) EDC gegeben und die Mischung 30 min lang gerührt. Anschließend wurden 7.8 mg (32 µmol) (2*S*)-1-(Benzyloxy)-3-phenylpropan-2-amin-Hydrochlorid (Intermediat 31) hinzugefügt. Nach Rühren über Nacht wurde das Reaktionsgemisch direkt über präparative HPLC in seine Komponenten aufgetrennt. Es wurden 26 mg (98% d. Th.) der Titelverbindung erhalten.

LC-MS (Methode 1): Rₜ = 1.51 min; m/z = 909 (M+H)⁺.

### Intermediat 33

### N-Methyl-L-valyl-N-[(3R,4S,5S)-1-{(2S)-2-[(1R,2R)-3-{[(2S)-1-(benzyloxy)-3-phenylpropan-2-yl]-amino}-1-methoxy-2-methyl-3-oxopropyl]pyrrolidin-1-yl}-3-methoxy-5-methyl-1-oxoheptan-4-yl]-N-methyl-L-valinamid Trifluoressigsäure-Salz

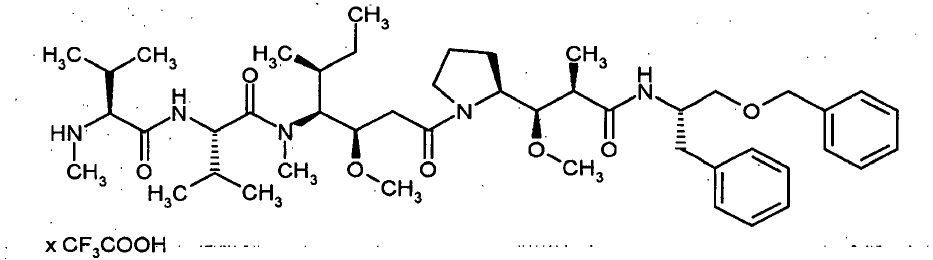

26 mg (29 µmol) *N-*(*tert*.-Butoxycarbonyl)-*N-*methyl-L-valyl-*N-*[(3*R*,4*S*,5*S*)-1-{(2*S)*-2-[(1*R*,2*R*)-3-{[(2*S*)-1-(benzyloxy)-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid (Intermediat 32) wurden in 1.8 ml Dichlormethan gelöst und mit 370 µl TFA versetzt. Das Reaktionsgemisch wurde 30 min bei RT gerührt und anschließend eingeengt. Der Rückstand wurde in Wasser aufgenommen und lyophilisiert. Es wurden 26.4 mg (quant.) der Titelverbindung erhalten.

LC-MS (Methode 1): Rₜ = 0.97 min; m/z = 809 (M+H)⁺.

### Intermediat 34

### tert.-Butyl-[(2S)-1-(2-benzoylhydrazino)-1-oxo-3-phenylpropan-2-yl]carbamat

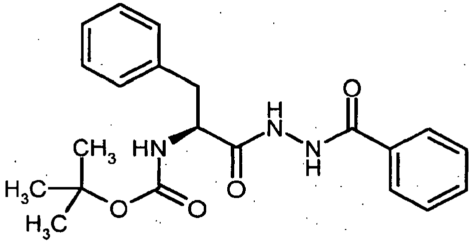

200 mg (754 µmol) *N-*Boc-L-Phenylalanin wurden in 8 ml DMF gelöst und mit 131 µl (754 µmol) *N,N-*Diisopropylethylamin, 346 mg (2261 µmol) HOBt, 434 mg (2261 µmol) EDC sowie 411 mg (3015 µmol) Benzoylhydrazin versetzt. Das Reaktionsgemisch wurde über Nacht bei RT gerührt, dann eingeengt und der Rückstand über präparative HPLC gereinigt. Es wurden 313 mg (95% Reinheit, 100% d. Th.) der Titelverbindung erhalten.

LC-MS (Methode 1): Rₜ = 0.94 min; m/z = 384 (M+H)⁺.

### Intermediat 35

### N'-[(2S)-2-Amino-3-phenylpropanoyl]benzohydrazid-Hydrochlorid

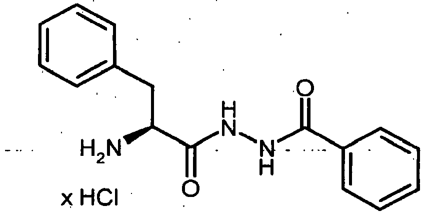

313 mg (818 µmol) *tert*.-Butyl-[(2*S*)-1-(2-benzoylhydrazino)-1-oxo-3-phenylpropan-2-yl]carbamat (Intermediat 34) wurden in 13 ml einer 4 N Lösung von Chlorwasserstoff in Dioxan gelöst und 1 h bei RT gerührt. Anschließend wurde das Reaktionsgemisch eingeengt und der Rückstand im Vakuum getrocknet. Es wurden 255 mg (92% d. Th.) der Titelverbindung erhalten.

LC-MS (Methode 1): Rₜ = 0.44 min; m/z = 284 (M+H)⁺.

### Intermediat 36

### tert.-Butyl-[(2S)-1-{[(2S)-1-{[(3R,4S,5S)-1-{(2S)-2-[(1R,2R)-3-{[(2S)-1-(2-benzoylhydrazino)-1-oxo-3-phenylpropan-2-yl]amino}-1-methoxy-2-methyl-3-oxopropyl]pyrrolidin-1-yl}-3-methoxy-5-methyl-1-oxoheptan-4-yl](methyl)amino}-3-methyl-1-oxobutan-2-yl]amino}-3-methyl-1-oxobutan-2-yl]methylcarbamat

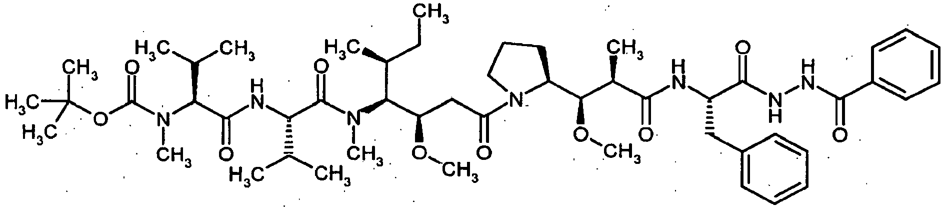

Zu einer Lösung von 20 mg (29 µmol) *N*-(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N-*methyl-L-valinamid (Intermediat 15) in 1 ml DMF wurden bei RT 15.3 µl (88 µmol) *N*,*N-*Diisopropylethylamin, 6.7 mg (44 µmol) HOBt und 6.7 mg (35 µmol) EDC gegeben und die Mischung 30 min lang gerührt. Anschließend wurden 9.1 mg (32 µmol) *N'*[(2S)-2-Amino-3-phenylpropanoyl]benzohydrazid-Hydrochlorid (Intermediat 35) hinzugefügt. Nach Rühren über Nacht wurde das Reaktionsgemisch direkt über präparative HPLC in seine Komponenten aufgetrennt. Es wurden 6.7 mg (24% d. Th.) der Titelverbindung erhalten.

LC-MS (Methode 1): Rₜ = 1.32 min; m/z = 951 (M+H)⁺.

### Intermediat 37

### (2S)-N-[(3R,4S,5S)-1-{(2S)-2-[(1R,2R)-3-{[(2S)-1-(2-Benzoylhydrazino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N,3-dimethyl-2-{[(2S)-3-methyl-2-(methylamino)butanoyl]amino}butanamid Trifluoressigsäure-Salz

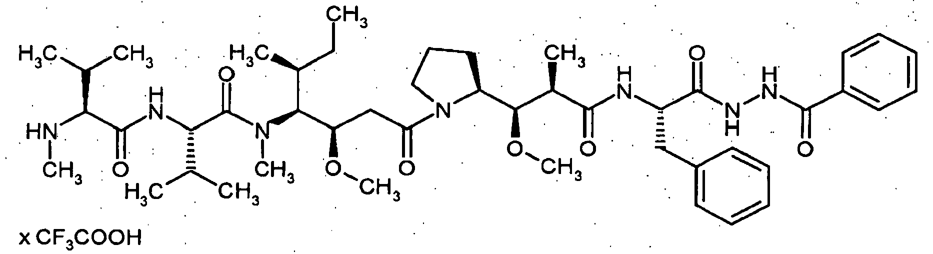

6.7 mg (7 µmol) *tert*.-Butyl-[(2*S*)-1-{[(2*S*)-1-{[(3*R*,4*S*,5*S*)-1-{(2*S*)-2-[(1*R*,2*R*)-3-{[(2*S*)-1-(2-benzoylhydrazino)-1-oxo-3-phenylpropan-2-yl]amino}-1-methoxy-2-methyl-3-oxopropyl]pyrrolidin-1-yl}-3-methoxy-5-methyl-1-oxoheptan-4-yl](methyl)amino}-3-methyl-1-oxobutan-2-yl]amino}-3-methyl-1-oxobutan-2-yl]methylcarbamat (Intermediat 36) wurden in 454 µl Dichlormethan gelöst und mit 91 µl TFA versetzt. Das Reaktionsgemisch wurde 30 min bei RT gerührt und anschließend eingeengt. Der Rückstand wurde in Wasser aufgenommen und lyophilisiert. Es wurden 6.8 mg (quant.) der Titelverbindung erhalten.

LC-MS (Methode 1): Rₜ = 0.80 min; m/z = 851 (M+H)⁺.

### Intermediat 38

### Benzyl-(1S,2R)-1-amino-2-phenylcyclopropancarboxylat Trifluoressigsäure-Salz

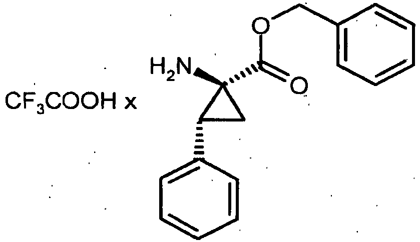

Die Titelverbindung wurde nach Standardmethoden durch Veresterung von kommerziell erhältlicher (1*S*,2*R*)-1-[(*tert*.-Butoxycarbonyl)amino]-2-phenylcyclopropancarbonsäure mit Benzylalkohol und anschließende Boc-Abspaltung mit Trifluoressigsäure hergestellt.

LC-MS (Methode 1): Rₜ = 0.72 min; MS (ESIpos): m/z = 268 (M+H)⁺.

### Intermediat 39

### N-(tert.-Butoxycarbonyl)-N-methyl-L-valyl-N-[(3R,4S,5S)-1-{(2S)-2-[(1R,2R)-3-{[(1S)-1-carboxy-2-phenylethyl]amino}-1-methoxy-2-methyl-3-oxopropyl]pyrrolidin-1-yl}-3-methoxy-5-methyl-1-oxoheptan-4-yl]-N-methyl-L-valinamid

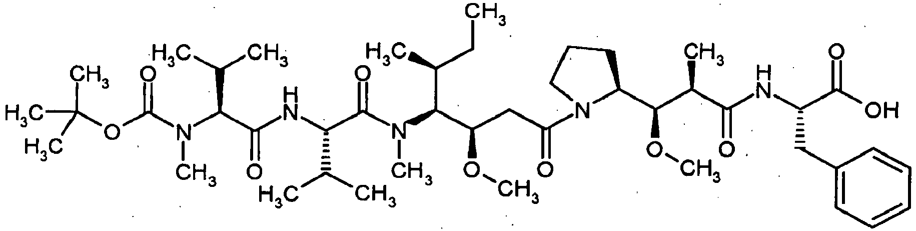

383 mg (0.743 mmol) *N-*(*tert*.-Butoxycarbonyl)-*N-*methyl-L-valyl-*N-*[(2*R*,3*S*,4*S*)-1-carboxy-2-methoxy-4-methylhexan-3-yl]-*N*-methyl-L-valinamid (Intermediat 5) wurden mit 485 mg (0.743 mmol) Benzyl-*N-*{(2*R*,3*R*)-3-methoxy-2-methyl-3-[(2*S*)-pyrrolidin-2-yl]propanoyl}-L-phenylalaninat Trifluoressigsäure-Salz (Intermediat 8), 424 mg (1.114 mmol) *O*-(7-Azabenzotriazol-1-yl)-*N,N,N',N'*-tetramethyluronium-hexafluorophosphat und 388 µl *N,N*-Diisopropylethylamin in 15 ml DMF zusammengegeben und 10 min bei RT gerührt. Anschließend wurde das Lösungsmittel im Vakuum entfernt. Der verbliebene Rückstand wurde in Ethylacetat aufgenommen und nacheinander mit 5%-iger wässriger Zitronensäure-Lösung und gesättigter Natriumhydrogencarbonat-Lösung ausgeschüttelt. Die organische Phase wurde abgetrennt, eingeengt und der Rückstand mittels präparativer HPLC gereinigt. Die Produktfraktionen wurden vereinigt, eingeengt und der Rückstand im Hochvakuum getrocknet. Es wurden 335 mg (48% d. Th.) des Benzylester-Intermediats als ein Schaum erhalten.

LC-MS (Methode 1): Rₜ = 1.49 min; MS (ESIpos): m/z = 922 (M+H)⁺.

100 mg (0.11 mmol) dieses Intermediats wurden in 15 ml Methanol aufgenommen und die Benzylester-Gruppe durch Hydrierung unter Normaldruck mit 10% Palladium auf Aktivkohle als Katalysator entfernt. Nach 1 h Rühren bei RT wurde der Katalysator abfiltriert und das Filtrat im Vakuum eingeengt. Nach Lyophilisation aus Dioxan wurden 85 mg (94% d. Th.) der Titelverbindung als weißer Feststoff erhalten.

HPLC (Methode 5): Rₜ = 2.4 min;

LC-MS (Methode 1): Rₜ = 1.24 min; MS (ESIpos): m/z = 832 (M+H)⁺.

### Intermediat 40

### N-Benzyl-L-tryptophanamid Trifluoressigsäure-Salz

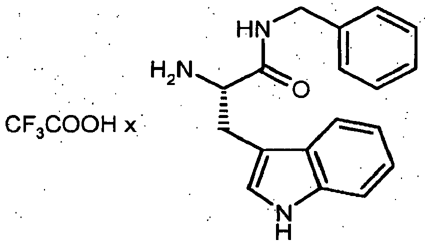

202 mg (0.5 mmol) 2,5-Dioxopyrrolidin-l-yl-*N*-(tert.-butoxycarbonyl)-L-tryptophanat und 45 mg (0.42 mmol) Benzylamin wurden in 10 ml DMF gelöst und mit 110 µl (630 µmol) *N,N-*Diisopropylethylamin versetzt. Der Ansatz wurde 3 h bei RT gerührt. Anschließend wurde im Vakuum eingeengt und der Rückstand durch Flash-Chromatographie an Kieselgel gereinigt (Laufmittel: Dichlormethan/Methanol/17% aq. Ammoniak 20:0.5:0.05). Die entsprechenden Fraktionen wurden vereinigt und eingeengt. Der resultierende Rückstand wurde mit Diethylether digeriert und dann im Hochvakuum getrocknet. Anschließend wurde dieser Rückstand in 10 ml Dichlormethan aufgenommen und mit 3 ml wasserfreier Trifluoressigsäure versetzt. Nach 45 min Rühren bei RT wurde eingeengt und der Rückstand durch präparative HPLC gereinigt. Nach Trocknen im Hochvakuum wurden 117 mg (57% d. Th. über beide Stufen) der Titelverbindung erhalten.

HPLC (Methode 5): Rₜ =1.6 min;

LC-MS (Methode 1): Rₜ = 0.66 min; MS (ESIpos): m/z = 294 (M+H)⁺.

### Intermediat 41

### (1S,2R)-1-Amino-2-phenylcyclopropancarboxamid Trifluoressigsäure-Salz

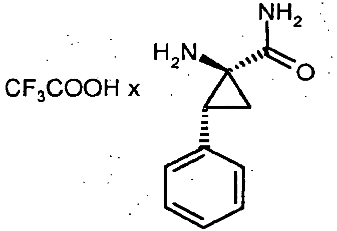

50 mg (180 µmol) kommerziell erhältlicher (1*S*,2*R*)-1-[(*tert*.-Butoxycarbonyl)amino]-2-phenylcyclo-propancarbonsäure wurden in 5 ml DMF gelöst, mit 94 µl (541 µmol) *N*,*N*-Diisopropylethylamin, 31 mg (270 µmol) *N*-Hydroxysuccinimid sowie 41.5 mg (216 µmol) EDC versetzt und anschließend über Nacht bei RT gerührt. Das Reaktionsgemisch wurde danach eingeengt, der Rückstand in Dioxan aufgenommen, mit 71 mg (901 µmol) Ammoniumhydrogencarbonat versetzt und der Ansatz dann 3 Tage bei RT stehen gelassen. Das Reaktionsgemisch wurde danach mit einer 1:1-Mischung aus Ethylacetat und Wasser verdünnt. Die organische Phase wurde abgetrennt, über Magnesiumsulfat getrocknet und eingeengt. Der resultierende Rückstand wurde anschließend in 3 ml Dichlormethan aufgenommen und mit 3 ml wasserfreier Trifluoressigsäure versetzt. Nach 1 h Rühren bei RT wurde eingeengt. Der Rückstand wurde mit Pentan verrührt, abgesaugt und aus Dioxan lyophilisiert. Auf diese Weise wurden 32 mg (62% d. Th. über beide Stufen) der Titelverbindung erhalten.

HPLC (Methode 5): Rₜ = 0.38 min;

LC-MS (Methode 1): Rₜ = 0.20 min; MS (ESIpos): m/z = 177 (M+H)⁺.

### Intermediat 42

### N^{α}-{(2R,3R)-3-Methoxy-2-methyl-3-[(2S)-pyrrolidin-2-yl]propanoyl}-L-tryptophanamid Trifluoressigsäure-Salz,

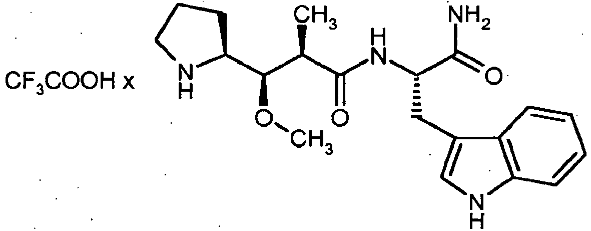

Die Titelverbindung wurde in Analogie zur Synthese von Intermediat 7 aus der Ausgangsverbindung 1 und L-Tryptophanamid-Hydrochlorid hergestellt.

HPLC (Methode 5): Rₜ = 1.4 min;

LC-MS (Methode 1): Rₜ = 0.92 min; MS (ESIpos): m/z = 473 (M+H)⁺.

### Intermediat 43

### tert. -Butyl-4-(brommethyl)benzoat

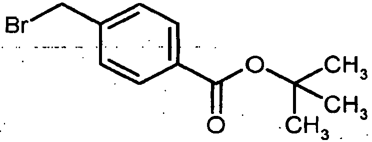

1000 mg (4.65 mmol) 4-(Brommethyl)benzoesäure wurden in 6 ml Dichlormethan vorgelegt, dann wurden zuerst 18 µl (0.23 mmol) DMF zugegeben und anschließend 811 µl (9.3 mmol) Oxalsäuredichlorid zugetropft. Der Ansatz wurde 15 min bis zur Beendigung der Gasentwicklung gerührt und danach eingeengt. Der Rückstand wurde in 10 ml Toluol aufgenommen und abermals eingeengt. Anschließend wurde das so erhaltene Benzoesäurechlorid in 30 ml Diethylether suspendiert und portionsweise mit einer Suspension von 522 mg (4.65 mmol) Kalium-*tert*.-butylat in 40 ml Diethylether versetzt. Das Reaktionsgemisch wurde 1 h bei RT gerührt und dann filtriert. Das Filtrat wurde je dreimal mit Wasser, gesättigter Ammoniumchlorid-Lösung und gesättigter Natriumchlorid-Lösung gewaschen, über Magnesiumsulfat getrocknet und nach Filtration im Vakuum eingeengt. Das erhaltene Rohprodukt wurde per Flashchromatographie über Kieselgel gereinigt (Laufmittel. Cyclohexan/Ethylacetat 10:1). Es wurden so 770 mg (51% d. Th.) der Titelverbindung erhalten.

MS (DCI): m/z = 217 (M-54)⁺.

### Intermediat 44

### 4-({[(2S)-2-Amino-3-phenylpropyl]oxy}methyl)benzoesäure Trifluoressigsäure-Salz

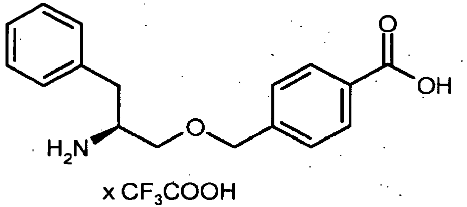

168.5 mg (0.67 mmol) *tert*.-Butyl-[(2*S*)-1-hydroxy-3-phenylpropan-2-yl]carbamat wurden unter Argon in 4.9 ml DMF vorgelegt und bei 0°C mit 53.6 mg (1.3 mmol) Natriumhydrid (als 60%-ige Dispersion in Paraffinöl) versetzt. Nach 30 min bei 0°C wurden 200 mg (0.74 mmol) *tert.-*Butyl-4-(brommethyl)benzoat zugegeben. Nach beendeter Zugabe wurde der Ansatz 2 h bei RT gerührt. Danach wurde der Ansatz am Rotationsverdampfer eingeengt, der Rückstand in Eiswasser aufgenommen und das Gemisch dreimal mit je 50 ml Dichlormethan extrahiert. Die vereinigten organischen Phasen wurden mit gesättigter Natriumchlorid-Lösung gewaschen, über Magnesiumsulfat getrocknet und im Vakuum eingeengt. Das so erhaltene Rohprodukt wurde mittels präparativer HPLC gereinigt. Es wurden 50.2 mg (17% d. Th.) des Intermediats *tert:-*Butyl-4-[({(2*S*-2-[(*tert.-*butoxycarbonyl)amino]-3-phenylpropyl}oxy)methyl]benzoat erhalten.

HPLC (Methode 10): Rₜ = 4.06 min;

LC-MS (Methode 1): Rₜ = 1.45 min; MS (ESIpos): m/z = 442 (M+H)⁺.

50 mg (0.11 mmol) dieses Intermediats wurden bei RT in 1.3 ml Trifluoressigsäure und 6.4 ml Dichlormethan gelöst und 15 min gerührt. Danach wurde der Ansatz am Rotationsverdampfer eingeengt und der Rückstand im Vakuum getrocknet. Es wurden so 51.2 mg (99% d. Th.) der Titelverbindung erhalten.

HPLC (Methode 10): Rₜ = 2.40 min;

LC-MS (Methode 1): Rₜ = 0.53 min; MS (ESIpos): m/z = 286 (M+H)⁺.

### Intermediat 45

### Methyl-4-({[(2S)-2-amino-3-phenylpropyl]oxy}methyl)benzoat-Hydrochlorid

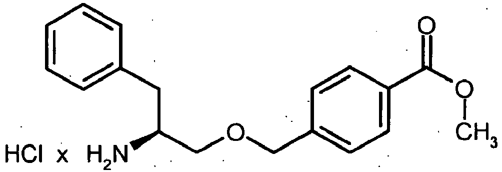

51.2 mg (128 µmol) 4-({[(2*S*)-2-Amino-3-phenylpropyl]oxy}methyl)benzoesäure Trifluoressigsäure-Salz wurden in 5 ml Methanol vorgelegt und mit 2.4 mg (13 µmol) 4-ToluolsulfonsäureMonohydrat versetzt. Der Ansatz wurde über Nacht unter Rückfluss gerührt. Anschließend wurden 18.7 µl (256 µmol) Thionylchlorid zugegeben und das Gemisch nochmals 6 h unter Rückfluss erhitzt. Danach wurde die Reaktionsmischung am Rotationsverdampfer eingeengt und der Rückstand im Vakuum getrocknet. Es wurden so 42.3 mg (80% Reinheit, 88% d. Th.) der Titelverbindung erhalten, die ohne weitere Aufreinigung in der Folgereaktion eingesetzt wurde.

HPLC (Methode 10): Rₜ = 2.48 min;

LC-MS (Methode 1): Rₜ = 0.68 min; MS (ESIpos): m/z = 300 (M+H)⁺.

### Intermediat 46

### N-Methyl-L-valyl-N-[(3R,4S,5S)-3-methoxy-1-{(2S)-2-[(1R,2R)-1-methoxy-3-{[(2S-1-{[4-(methoxycarbonyl)benzyl]oxy}-3-phenylpropan-2-yl]amino}-2-methyl-3-oxopropyl]pyrrolidin-1-yl}-5-methyl-1-oxoheptan-4-yl]-N-methyl-L-valinamid Trifluoressigsäure-Salz

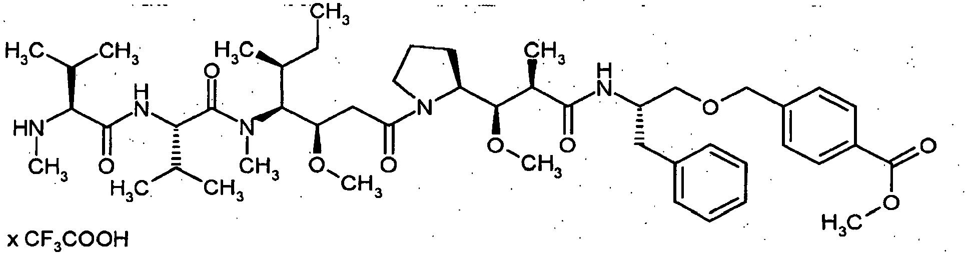

20 mg (29 µmol) *N*-(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) wurden in 0.59 ml DMF vorgelegt und mit 13.3 mg (35 µmol) HATU sowie 20 µl (117 µmol) *N*,*N-*Diisopropylethylamin versetzt. Nach 30 min wurden 9.6 mg (32 µmol) Methyl-4-({[(2*S*)-2-amino-3-phenylpropyl]oxy}methyl)benzoat-Hydrochlorid (Intermediat 45) zugegeben. Das Reaktionsgemisch wurde über Nacht bei RT gerührt und anschließend direkt, ohne weitere Aufarbeitung, durch präparative HPLC in seine Komponenten aufgetrennt. Es wurden 15.2 mg (54% d. Th.) des Boc-geschützten Intermediats *N*-(*tert*.-Butoxycarbonyl)-*N-*methyl-L-valyl-*N-*[(3*R*,4*S*,5*S*)-3-methoxy-1-{(2*S*)-2-[(1*R*,2*R*)-1-methoxy-3-{[(2*S*)-1-{[4-(methoxycarbonyl)benzyl]-oxy}-3-phenylpropan-2-yl]amino}-2-methyl-3-oxopropyl]pyrrolidin-1-yl}-5-methyl-1-oxoheptan-4-yl]-*N-*methyl-L-valinamid erhalten.

LC-MS (Methode 1): Rₜ = 1.49 min; MS (ESIpos): m/z = 967 (M+H)⁺.

Durch nachfolgende Abspaltung der Boc-Schutzgruppe mit Trifluoressigsäure erhielt man dann 15.8 mg (99% d. Th.) der Titelverbindung.

LC-MS (Methode 11): Rₜ = 0.90 min; MS (ESIpos): m/z = 867 (M+H)⁺.

### Ausführungsbeispiele:

### Beispiel 1

### N-(3-Carboxypropyl)-N-methyl-L-valyl-N-[(3R,4S,5S)-3-methoxy-1-{(2S)-2-[(1R,2R)-1-methoxy-2-Methyl-3-{[(2S)-1-(1-naphthylmethoxy)-1-oxo-3-phenylpropan-2-yl]amino}-3-oxopropyl]pyrrolidin-1-yl}-5-methyl-1-oxoheptan-4-yl]-N-methyl-L-valinamid

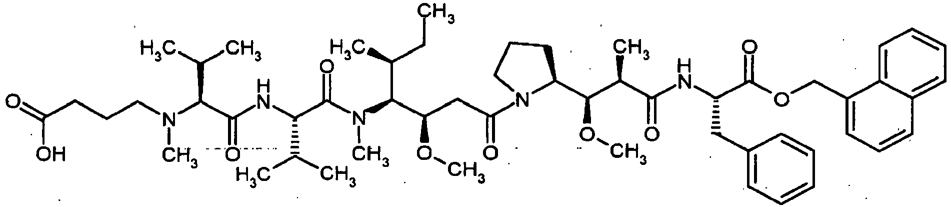

24 mg (24 µmol) *N*-Methyl-L-valyl-*N-*[(3*R*,4*S*,5*S*)-3-methoxy-1-{(2*S*)-2-[(1*R*,2*R*)-1-methoxy-2-methyl-3-{[(2*S*)-1-(1-naphthylmethoxy)-1-oxo-3-phenylpropan-2-yl]amino}-3-oxopropyl]pyrrolidin-1-yl}-5-methyl-1-oxoheptan-4-yl]-*N-*methyl-L-valinamid Trifluoressigsäure-Salz (Intermediat 21) und 31.5 µl einer 15%-igen wässrigen Bernsteinaldehydsäure-Lösung (49 µmol) wurden in 900 µl eines 1:1-Dioxan/Wasser-Gemisches gelöst und für 1 h auf 100°C erhitzt. Nach kurzem Abkühlen wurden 1.7 mg (27 µmol) Natriumcyanoborhydrid zugegeben. Das Reaktionsgemisch wurde durch Zugabe von 0.1 N Salzsäure auf pH 3 eingestellt und für weitere 2 h auf 100°C erhitzt. Nach erneuter Zugabe gleicher Mengen an Bernsteinaldehydsäure-Lösung, Natriumcyanoborhydrid und Salzsäure wurde nochmals für 2 h auf 100°C erhitzt. Das Reaktionsgemisch wurde danach direkt mittels präparativer HPLC in seine Komponenten aufgetrennt. Es wurden so 20.1 mg (86% d. Th.) der Titelverbindung erhalten.

LC-MS (Methode 1): Rₜ = 1.06 min; m/z = 959 (M+H)⁺

¹H-NMR(500 MHz, DMSO-d₆): δ [ppm] = 0.73-0.81 (m, 3H), 0.82-1.05 (m, 15H), 1.19-1.48 (m, 3H), 1.53-2.10 (m, 4H), 2.12-2.41 (m, 5H), 2.79 (d, 2H), 2.85-3.06 (m, 3H), 3.06-3.13 (m, 3H), 3.13-3.28 (m, 6H), 3.35-3.43 (m, 1H), 3.89-4.14 (m, 1H), 4.45-4.81 (m, 3H), 5.48-5.73 (m, 2H), 7.13-7.33 (m, 5H), 7.45-7.53 (m, 1H), 7.53-7.64 (m, 3H), 7.91-8.05 (m, 3H), 8.21-8.60 (m, 1H), 8.69-9.01 (m, 1H), 9.27-9.60 (m, 1H) [weitere Signale unter Lösungsmittel-Peaks verborgen].

### Beispiel 2

### N-(3-Carboxypropyl)-N-methyl-L-valyl-N-[(3R,4S,5S)-1-{(2S)-2-[(1R,2R)-3-{[(2S)-1-(adamantan-1-yloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

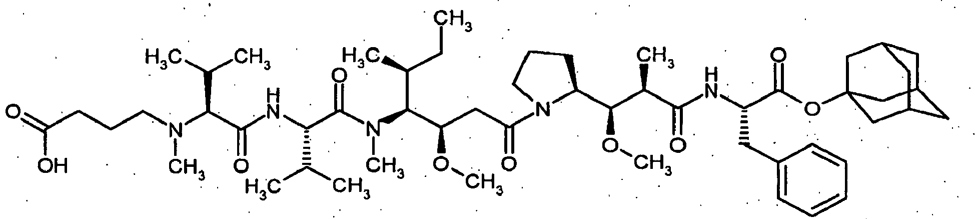

4.8 mg (5 µmol) *N-*Methyl-L-valyl-*N-*[(3*R*,4*S*,5*S*)-1-{(2*S*)-2-[(1*R*,2*R*)-3-{[(2*S*)-1-(adamantan-1-yl-oxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N-*methyl-L-valinamid Trifluoressigsäure-Salz (Intermediat 25) und 6.3 µl einer 15%-igen wässrigen Bernsteinaldehydsäure-Lösung (10 µmol) wurden in 180 µl eines 1:1-Dioxan/Wasser-Gemisches gelöst und für 1 h auf 100°C erhitzt. Nach kurzem Abkühlen wurden 0.34 mg (5 µmol) Natriumcyanoborhydrid zugegeben. Das Reaktionsgemisch wurde durch Zugabe von 0.1 N Salzsäure auf pH 3 eingestellt und für weitere 2 h auf 100°C erhitzt. Nach erneuter Zugabe gleicher Mengen an Bernsteinaldehydsäure-Lösung, Natriumcyanoborhydrid und Salzsäure wurde nochmals für 2 h auf 100°C erhitzt. Das Reaktionsgemisch wurde danach direkt mittels präparativer HPLC in seine Komponenten aufgetrennt. Es wurden so 3.2 mg (69% d. Th.) der Titelverbindung erhalten.

LC-MS (Methode 1): Rₜ = 1.14 min; m/z = 952 (M+H)⁺.

### Beispiel 3

### N-(3-Carboxypropyl)-N-methyl-L-valyl-N-[(3R,4S,5S)-1-{(2S)-2-[(1R,2R)-3-{[(2S)-1-(adamantan-1-ylmethoxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

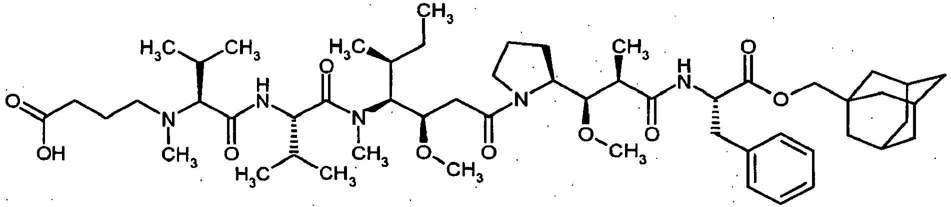

26 mg (26 µmol) *N*-Methyl-L-valyl-*N-*[(3*R*,4*S*,5*S*)-1-{(2*S*)-2-[(1*R*,2*R*)-3-{[(2*S*)-1-(adamantan-1-yl-methoxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N-*methyl-L-valinamid Trifluoressigsäure-Salz (Intermediat 29) und 33.9 µl einer 15%-igen wässrigen Bernsteinaldehydsäure-Lösung (53 µmol) wurden in 957 µl eines 1:1-Dioxan/Wasser-Gemisches gelöst und für 1 h auf 100°C erhitzt. Nach kurzem Abkühlen wurden 1.81 mg (29 µmol) Natriumcyanoborhydrid zugegeben. Das Reaktionsgemisch wurde durch Zugabe von 0.1 N Salzsäure auf pH 3 eingestellt und für weitere 2 h auf 100°C erhitzt. Nach erneuter Zugabe gleicher Mengen an Bernsteinaldehydsäure-Lösung, Natriumcyanoborhydrid und Salzsäure wurde nochmals für 2 h auf 100°C erhitzt. Das Reaktionsgemisch wurde danach direkt mittels präparativer HPLC in seine Komponenten aufgetrennt. Es wurden so 18.5 mg (73% d. Th.) der Titelverbindung erhalten.

LC-MS (Methode 1): Rₜ = 1.17 min; m/z = 967 (M+H)⁺.

### Beispiel 4

### N-(3-Carboxypropyl)-N-methyl-L-valyl-N-[(3R,4S,5S)-1-{(2S-2-[(1R,2R)-3-{[(2S)-1-(benzyloxy)-3-phenylpropan-2-yl]amino}-1-methoxy-2-methyl-3-oxopropyl]pyrrolidin-1-yl -3-methoxy-5-methyl-1-oxoheptan-4-yl]-N-methyl-L-valinamid

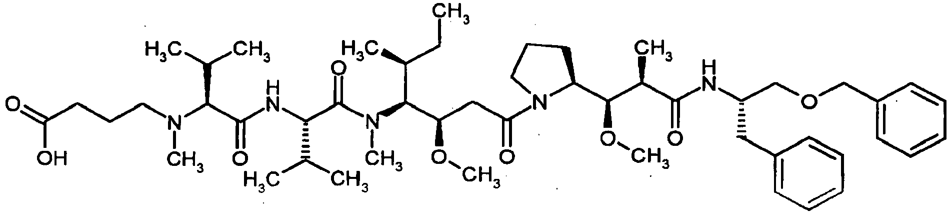

24 mg (26 µmol) *N*-Methyl-L-valyl-*N-*((3*R*,4*S*,5*S)*-1-{(2*S*)-2-[(1*R*,2*R*)-3-{[(2*S*)-1-(benzyloxy)-3-phenylpropan-2-yl]amino}-1-methoxy-2-methyl-3-oxopropyl]pyrrolidin-1-yl}-3-methoxy-5-methyl-1-oxoheptan-4-yl]-*N-*methyl-L-valinamid Trifluoressigsäure-Salz (Intermediat 33) und 33.7 µl einer 15%-igen wässrigen Bernsteinaldehydsäure-Lösung (52 µmol) wurden in 953 µl eines 1:1-Dioxan/Wasser-Gemisches gelöst und für 1 h auf 100°C erhitzt. Nach kurzem Abkühlen wurden 1.80 mg (29 µmol) Natriumcyanoborhydrid zugegeben. Das Reaktionsgemisch wurde durch Zugabe von 0.1 N Salzsäure auf pH 3 eingestellt und für weitere 2 h auf 100°C erhitzt. Nach erneuter Zugabe gleicher Mengen an Bernsteinaldehydsäure-Lösung, Natriumcyanoborhydrid und Salzsäure wurde nochmals für 2 h auf 100°C erhitzt. Das Reaktionsgemisch wurde danach direkt mittels präparativer HPLC in seine Komponenten aufgetrennt. Es wurden so 15.2 mg (65% d. Th.) der Titelverbindung erhalten.

LC-MS (Methode 1): Rₜ = 1.01 min; m/z = 895 (M+H)⁺

¹H-NMR (500 MHz, DMSO-d₆): δ [ppm] = 0.72-0.81 (m, 3H), 0.81-1.00 (m, 15H), 1.03 (dd, 3H), 1.12-1.56 (m, 3H), 1.62-2.45 (m, 10H), 2.61-2.73 (m, 1H), 2.78 (br. s, 2H), 2.84-3.07 (m, 3H), 3.11 (br. s, 2H), 3.17 (s, 1H), 3.21 (d, 3H), 3.26 (s, 3H), 3.30-3.35 (m, 2H), 3.51-3.94 (m, 4H), 4.00 (br. s, 1H), 4.08-4.35 (m, 1H), 4.50 (s, 1H), 4.53 (s, 1H), 4.55-4.78 (m, 2H), 7.12-7.16 (m, 1H), 7.16-7.25 (m, 4H), 7.30 (d, 1H), 7.33-7.42 (m, 4H), 7.76 und 8.01 (2d, 1H), 8.73-8.98 (m, 1H) [weitere Signale unter Lösungsmittel-Peaks verborgen].

### Beispiel 5

### N-(3-Carboxypropyl)-N-methyl-L-valyl-N-[(3R,4S,5S)-1-{(2S)-2-[(1R,2R)-3-{[(2S)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

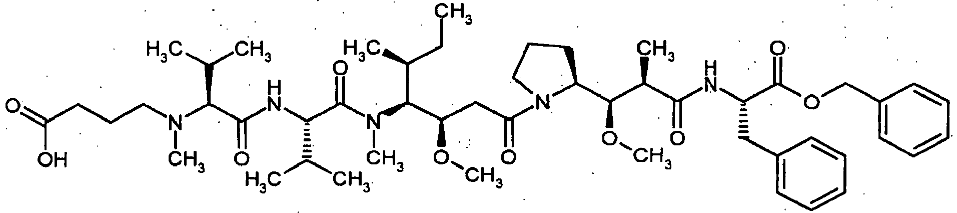

53 mg (84 µmol) *N*-[(9*H*-Fluoren-9-ylmethoxy)carbonyl]-*N*-methyl-L-valyl-[(2*R*,3*S*,4*S*)-1-carboxy-2-methoxy-4-methylhexan-3-yl]-*N-*methyl-L-valinamid (Intermediat 4) und 45 mg (84 µmol) Benzyl-*N*-{(2*R*,3*R*)-3-methoxy-2-methyl-3-[(2*S*)-pyrrolidin-2-yl]propanoyl}-L-phenylalaninat Trifluoressigsäure-Salz (Intermediat 8) wurden in 2 ml DMF aufgenommen, mit 19 µl *N*,*N*-Diisopropylethylamin, 14 mg (92 µmol) HOBt sowie 17.6 mg (92 µmol) EDC versetzt und dann über Nacht bei RT gerührt. Anschließend wurde das Reaktionsgemisch eingeengt und der Rückstand mittels präparativer HPLC gereinigt. Es wurden so 59 mg (68% d. Th.) des Fmoc-geschützten Intermediats *N-*[(9*H*-Fluoren-9-ylmethoxy)carbonyl]-*N-*methyl-L-valyl-*N-*[(3*R*,4*S*,5*S*)-1-{(2*S*)-2-[(1*R*,2*R*)-3-{[(2*S*)-1-(benzyloxy)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid erhalten.

LC-MS (Methode 1): Rₜ = 1.55 min; m/z = 1044 (M+H)⁺.

57 mg (0.055 mmol) dieses Intermediats wurden zur Abspaltung der Fmoc-Schutzgruppe mit 1.2 ml Piperidin in 5 ml DMF behandelt. Nach Einengen und Reinigung mittels präparativer HPLC wurden 39 mg (76% d. Th.) des freien Amin-Intermediats *N-*Methyl-L-valyl-*N-*[(3*R*,4*S*,5*S*)-1-{(2*S*)-2-[(1*R*,2*R*)-3-{[(2*S*)-1-(benzyloxy)-1-oxo-3-phenylpropan-2-yl]amimo}-1-methoxy-2-methyl-3-oxopropyl]pyrrolidin-1-yl}-3-methoxy-5-methyl-1-oxoheptan-4-yl]-*N*-methyl-L-valinamid als Trifluoressigsäure-Salz erhalten.

HPLC (Methode 5): Rₜ = 1.9 min;

LC-MS (Methode 1): Rₜ = 1.01 min; m/z = 822 (M+H)⁺.

37 mg (0.045 mmol) dieses Intermediats wurden in 5 ml Dioxan/Wasser (1:1) gelöst und analog zur Herstellung der Verbindung in Beispiel 6 mit einer 15%-igen wässrigen Lösung von 4-Oxobutansäure in Gegenwart von Natriumcyanoborhydrid umgesetzt. Es wurden 16 mg (39% d. Th.) der Titelverbindung in Form eines farblosen Schaums erhalten.

HPLC (Methode 5): Rₜ = 2.1 min;

LC-MS (Methode 1): Rₜ = 1.01 min; MS (ESIpos): m/z = 908 (M+H)⁺.

### Beispiel 6

### N-(3-Carboxypropyl)-N-methyl-L-valyl-N-[(3R,4S,5S)-1-{(2S)-2-[(1R,2R)-3-{[(2S,3S)-1-(benzyl-oxy)-1-oxo-3-phenylbutan-2-yl]amino}-1-methoxy-2-methyl-3-oxopropyl]pyrrolidin-1-yl}-3-methoxy-5-methyl-1-oxoheptan-4-yl]-N-methyl-L-valinamid

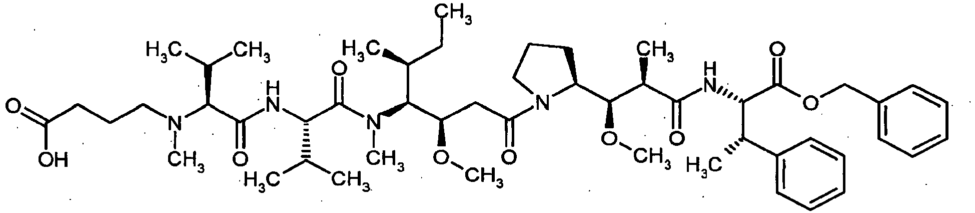

Zunächst wurde in Analogie zu der in Beispiel 5 beschriebenen Synthese ausgehend von den Intermediaten 4 und 7 die Amin-Verbindung *N*-Methyl-L-valyl-*N*-[(3*R*,4*S*,5*S*)-1-{(2*S*)-2-[(1*R*,2*R*)-3-{[(2*S*,3*S*)-1-(benzyloxy)-1-oxo-3-phenylbutan-2-yl]amino}-1-methoxy-2-methyl-3-oxopropyl]pyrrolidin-1-yl}-3-methoxy-5-methyl-1-oxoheptan-4-yl]-*N*-methyl-L-valinamid hergestellt.

30 mg (0.032 mmol) dieser Verbindung wurden in 6 ml Dioxan/Wasser (1:1) gelöst und mit 41 µl (0.063 mmol) einer 15%-igen wässrigen Lösung von 4-Oxobutansäure versetzt. Der Ansatz wurde anschließend 1 h bei 100°C gerührt. Nach Abkühlen auf RT wurden 2.2 mg (0.035 mmol) Natriumcyanoborhydrid zugegeben und die Mischung durch Zugabe von etwa 300 µl 0.1 N Salzsäure auf einen pH-Wert von 3 eingestellt. Der Ansatz wurde danach weitere 2 h bei 100°C gerührt. Nach Abkühlen wurden erneut 41 µl (0.063 mmol) der 15%-igen 4-Oxobutansäure-Lösung zugesetzt und der Ansatz wiederum 1 h bei 100°C gerührt. Dann wurden weitere 2.2 mg (0.035 mmol) Natriumcyanoborhydrid zugegeben und anschließend mit etwa 300 µl 0.1 N Salzsäure der pH-Wert wieder auf 3 eingestellt. Der Ansatz wurde danach erneut 2 h bei 100°C gerührt. Bei immer noch nicht vollständiger Umsetzung wurde diese Prozedur noch ein drittes Mal wiederholt. Der Ansatz wurde schließlich eingeengt und das Rohprodukt mittels präparativer HPLC gereinigt. Es wurden so 24 mg (82% d. Th.) der Titelverbindung in Form eines farblosen Schaums erhalten..

HPLC (Methode 5): Rₜ = 1.9 min;

LC-MS (Methode 9): Rₜ = 5.15 min; MS (ESIpos): m/z = 922 (M+H)⁺.

### Beispiel 7

### N-(3-Carboxypropyl)-N-methyl-L-valyl-N-[(3R,4S,5S)-3-methoxy-1-(2S)-2-[(1R,2R)-1-methoxy-3-{[(2S)-1-methoxy-1-oxo-3-phenylpropan-2-yl]amino}-2-methyl-3-oxopropyl]pyrrolidin-1-yl}-5-methyl-1-oxoheptan-4-yl]-N-methyl-L-valinamid

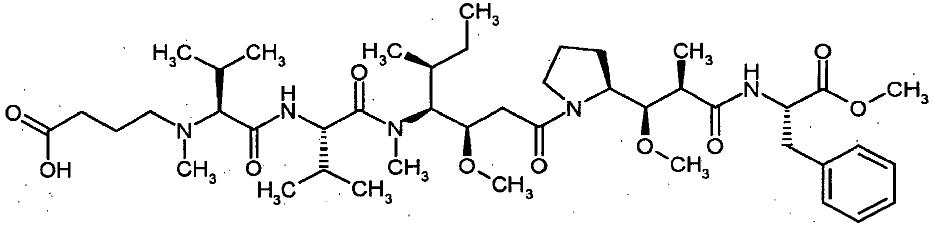

Zunächst wurde in Analogie zu der in Beispiel 5 beschriebenen Synthese ausgehend von den Intermediaten 4 und 11 die Amin-Verbindung *N-*Methyl-L-valyl-N-[(3*R*,4*S*,5*S*)-3-methoxy-1-{(2*S*)-2-[(1*R*,2*R*)-1-methoxy-3-{[(2*S*)-1-methoxy-1-oxo-3-phenylpropan-2-yl]amino}-2-methyl-3-oxo-propyl]pyrrolidin-1-yl}-5-methyl-1-oxoheptan-4-yl]-*N*-methyl-L-valinamid hergestellt. Aus 7 mg (0.009 mmol) dieser Verbindung wurden dann in Analogie zur Herstellung von Beispiel 6 durch Umsetzung mit 4-Oxobutansäure in Gegenwart von Natriumcyanoborhydrid 2 mg (22% d. Th.) der Titelverbindung in Form eines farblosen Schaums erhalten.

HPLC (Methode 5): Rₜ = 1.9 min;

LC-MS (Methode 2): Rₜ = 1.06 min; MS (ESIpos): m/z = 832 (M+H)⁺.

### Beispiel 8

### N-(3-Carboxypropyl)-N-methyl-L-valyl-N-[(3R,4S,5S)-1-{(2S)-2-[(1R,2R)-3-{[(2S)-1-(benzyloxy)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

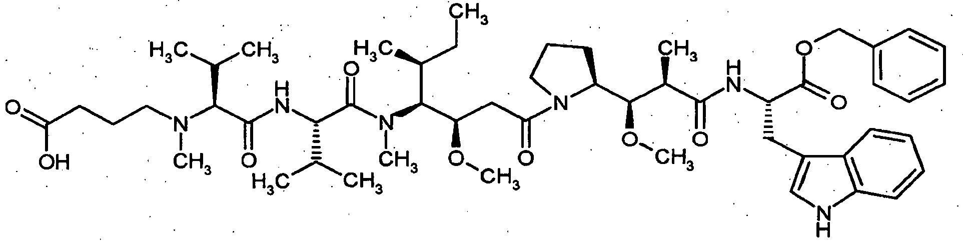

212 mg (411 µmol) *N-*(*tert*.-Butoxycarbonyl)-*N-*methyl-L-valyl-*N*-[(2*R*,3*S*,4*S*)-1-carboxy-2-methoxy-4-methylhexan-3-yl]-*N-*methyl-L-valinamid (Intermediat 5) und 237 mg (411 µmol) Benzyl-*N-*{(2*R*,3*R*)-3-methoxy-2-methyl-3-[(2*S*)-pyrrolidin-2-yl]propanoyl}-L-tryptophanat Trifluoressigsäure-Salz (Intermediat 12) wurden in 30 ml DMF aufgenommen und mit 188 mg (493 µmol) *O*-(7-Azabenzotriazol-1-yl)-*N,N,N',N*'-tetramethyluronium-hexafluorophosphat sowie 215 µl *N,N-*Diisopropylethylamin versetzt. Der Ansatz wurde 20 h bei RT gerührt, anschließend im Vakuum eingeengt und der Rückstand mittels präparativer HPLC aufgereinigt. Die Produktfraktionen wurden vereinigt, eingeengt und der Rückstand im Hochvakuum getrocknet. Es wurden so 315 mg (80% d. Th.) des Boc-geschützten Intermediats *N-*(*tert*.-Butoxycarbonyl)-*N-*methyl-L-valyl-*N-*[(3*R*,4*S*,5*S*)-1-{(2*S*)-2-[(1*R*,2*R*)-3-{[(2*S*)-1-(benzyloxy)-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als farbloser Schaum erhalten.

LC-MS (Methode 1): Rₜ = 1.45 min; m/z = 961 (M+H)⁺.

50 mg (52 µmol) dieses Intermediats wurden zur Abspaltung der Boc-Schutzgruppe mit 1 ml Trifluoressigsäure in 9 ml Dichlormethan behandelt. Nach Einengen und Reinigung mittels präparativer HPLC wurden 29 mg (57% d. Th.) des freien Amin-Intermediats *N*-Methyl-L-valyl-*N*-[(3*R*,4*S*,5*S*)-1-{(2*S*-2-[(1*R*,2*R*)-3-{[(2*S*)-1-(benzyloxy)-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoressigsäure-Salz erhalten.

LC-MS (Methode 1): Rₜ = 0.99 min; m/z = 861 (M+H)⁺.

29 mg (0.03 mmol) dieses Intermediats wurden in 6 ml Dioxan/Wasser (1:1) gelöst und mit 39 µl (0.059 mmol) einer 15%-igen wässrigen Lösung von 4-Oxobutansäure versetzt. Der Ansatz wurde anschließend 1 h bei 100°C gerührt. Nach Abkühlen auf RT wurden 2 mg (0.033 mmol) Natriumcyanoborhydrid zugegeben und die Mischung durch Zugabe von etwa 300 µl 0.1 N Salzsäure auf einen pH-Wert von 3 eingestellt. Der Ansatz wurde danach weitere 2 h bei 100°C gerührt. Nach Abkühlen wurden erneut 39 µl (0.059 mmol) der 15%-igen 4-Oxobutansäure-Lösung zugesetzt und der Ansatz wiederum 1 h bei 100°C gerührt. Dann wurden weitere 2 mg (0.033 mmol) Natriumcyanoborhydrid zugegeben und anschließend mit etwa 300 µl 0.1 N Salzsäure der pH-Wert wieder auf 3 eingestellt. Das Gemisch wurde erneut 2 h bei 100°C gerührt. Danach wurde der Ansatz auf ein 1:1-Gemisch aus halbgesättigter wässriger Ammoniumchlorid-Lösung und Ethylacetat gegossen. Die organische Phase wurde abgetrennt, mit gesättigter Natriumchlorid-Lösung gewaschen, über Natriumsulfat getrocknet und eingeengt. Der Rückstand wurde aus Wasser/ Acetonitril gefriergetrocknet. Es wurden so 27 mg (94% d. Th.) der Titelverbindung in Form eines farblosen Schaums erhalten.

HPLC (Methode 5): Rₜ = 2.2 min;

LC-MS (Methode 9): Rₜ = 5.04 min; MS (ESIpos): m/z = 947 (M+H)⁺.

### Beispiel 9

### N-(3-Carboxypropyl)-N-methyl-L-valyl-N-[(3R,4S,5S)-1-{(2S)-2-[(1R,2R)-3-({(2S)-1-[benzyl-(methyl)amino]-1-oxo-3-phenylpropan-2-yl}amino)-1-methoxy-2-methyl-3-oxopropyl]pyrrolidin-1-yl}-3-methoxy-5-methyl-1-oxoheptan-4-yl]-N-methyl-L-valinamid

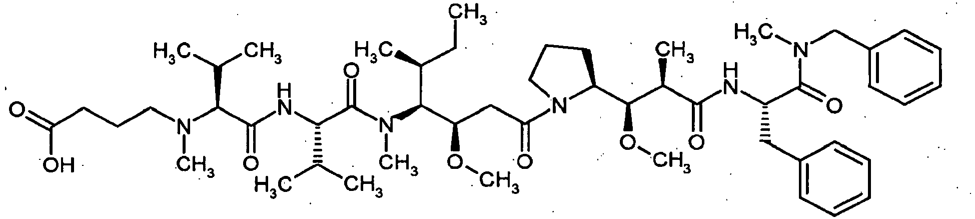

Zunächst wurde in Analogie zu der in Beispiel 5 beschriebenen Synthese ausgehend von den Intermediaten 4 und 10 die Amin-Verbindung *N*-Methyl-L-valyl-*N*-[(3*R*,4*S*,5*S*)-1-{(2*S*)-2-[(1*R,*2*R*)-3-({(2*S*)-1-[benzyl(methyl)amino]-1-oxo-3-phenylpropan-2-yl}amino)-1-methoxy-2-methyl-3-oxo-propyl]pyrrolidin-1-yl}-3-methoxy-5-methyl-1-oxoheptan-4-yl]-*N-*methyl-L-valinamid hergestellt. Aus 25 mg (0.026 mmol) dieser Verbindung wurden dann in Analogie zur Herstellung von Beispiel 6 durch Umsetzung mit 4-Oxobutansäure in Gegenwart von Natriumcyanoborhydrid 13 mg (54% d. Th.) der Titelverbindung in Form eines farblosen Schaums erhalten.

HPLC (Methode 5): Rₜ = 2.2 min;

LC-MS (Methode 9): Rₜ = 5.01 min; MS (ESIpos): m/z = 921 (M+H)⁺.

### Beispiel 10

### N(3-Carboxypropyl)-N-methyl-L-valyl-N-[(3R,4S,5S)-1-{(2S)-2-[(1R,2R)-3-({(1S,2R)-1-[(benzyl-oxy)carbonyl]-2-phenylcyclopropyl}amino)-1-methoxy-2-methyl-3-oxopropyl]pyrrolidin-1-yl}-3-methoxy-5-methyl-1-oxoheptan-4-yl]-N-methyl-L-valinamid

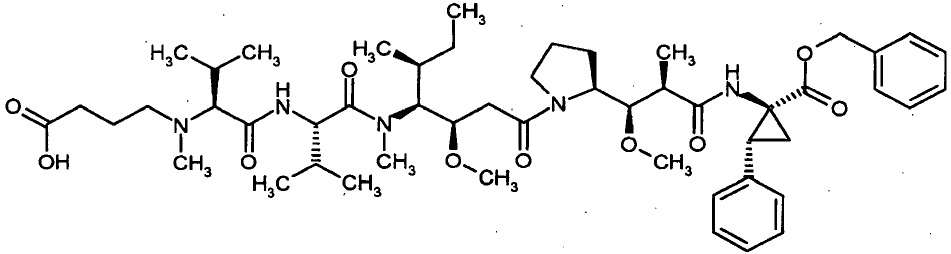

50 mg (73 µmol) *N-*(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) und 28 mg (73 µmol) Benzyl-(1*S*,2*R*)-1-amino-2-phenylcyclopropan-carboxylat Trifluoressigsäure-Salz (Intermediat 38) wurden in 5 ml DMF aufgenommen und mit 42 mg (110 µmol) *O*-(7-Azabenzotriazol-1-yl)-*N,N,N',N'*-tetramethyluronium-hexafluorophosphat und 38 µl *N,N-*Diisopropylethylamin versetzt. Der Ansatz wurde 5 h bei RT gerührt, anschließend im Vakuum eingeengt und der Rückstand mittels präparativer HPLC aufgereinigt. Die Produktfraktionen wurden vereinigt und eingeengt. Nach Lyophilisation aus Dioxan/Wasser wurden 35 mg (51% d. Th.) des Boc-geschützten Intermediats *N*-(tert.-Butoxycarbonyl)-*N*-methyl-L-valyl-*N-*[(3*R*,4*S*,5*S*)-1-{(2*S*)-2-[(1*R*,2*R*)-3-({(1*S*,2*R*)-1-[(benzyloxy)carbonyl]-2-phenylcyclopropyl}amino)-1-methoxy-2-methyl-3-oxopropyl]pyrrolidin-1-yl}-3-methoxy-5-methyl-1-oxoheptan-4-yl]-*N-*methyl-L-valinamid als farbloser Schaum erhalten.

LC-MS (Methode 1): Rₜ = 1.52 min; m/z = 934 (M+H)⁺.

35 mg dieses Intermediats wurden zur Abspaltung der Boc-Schutzgruppe mit 1 ml Trifluoressigsäure in 5 ml Dichlormethan behandelt. Nach Einengen und Lyophilisation aus Dioxan/Wasser wurden 34 mg (97% d. Th.) des freien Amin-Intermediats *N*-Methyl-L-valyl-*N*-[(3*R*,4*S*,5*S*)-1-{(2*S*)-2-[(1*R*,2*R*)-3-({(1*S*,2*R*)-1-[(benzyloxy)carbonyl]-2-phenylcyclopropyl}amino)-1-methoxy-2-methyl-3-oxopropyl]pyrrolidin-1-yl}-3-methoxy-5-methyl-1-oxoheptan-4-yl]-*N-*methyl-L-valinamid als Trifluoressigsäure-Salz erhalten.

LC-MS (Methode 1): Rₜ = 0.91 min; m/z = 834 (M+H)⁺.

Aus 1.1 mg (0.011 mmol) dieses Intermediats wurden dann in Analogie zur Herstellung von Beispiel 6 durch Umsetzung mit 4-Oxobutansäure in Gegenwart von Natriumcyanoborhydrid 2.5 mg (24% d. Th.) der Titelverbindung in Form eines farblosen Schaums erhalten.

HPLC (Methode 5): Rₜ = 2.2 min;

LC-MS (Methode 9): Rₜ = 5.1 min; MS (ESIpos): m/z = 920 (M+H)⁺.

### Beispiel 11

### N-(3-Carboxypropyl)-N-methyl-L-valyl-N-[(3R,4S,5S)-3-methoxy-1-{(2S)-2-[(1R,2R)-1-methoxy-2-methyl-3-oxo-3- {[(1S,2R)-2-phenyl-1-(propylcarbamoyl)cyclopropyl]amino}propyl]pyrrolidin-1-yl}-5-methyl-1-oxoheptan-4-yl]-N-methyl-L-valinamid

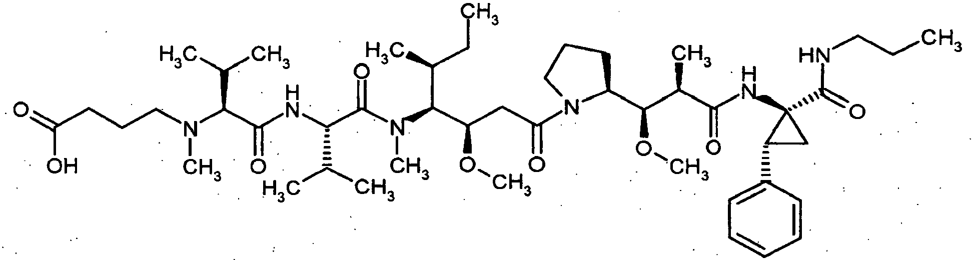

Zunächst wurde in Analogie zu der in Beispiel 10 beschriebenen Synthese durch Kupplung von *N-*(*tert*.-Butoxycarbonyl)-*N-*methyl-L-yalyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) und (1*S*,2*R*)-1-Amino-2-phenyl-*N-*propylcyclopropancarboxamid Trifluoressigsäure-Salz (Intermediat 16) in Gegenwart von *O*-(7-Azabenzotriazol-l-yl)-*N,N*,*N*',*N*',-tetramethyluronium-hexafluorophosphat und anschließende Abspaltung der Boc-Schutzgruppe mittels Trifluoressigsäure die Amin-Verbindung *N-*Methyl-L-valyl-*N-*[(3*R*,4*S*,5*S*)-3-methoxy-1-{(2*S*)-2-[(1*R*,2*R*)-1-methoxy-2-methyl-3-oxo-3-{[(1*S*,2*R*)-2-phenyl-1-(propylcarbamoyl)cyclopropyl]amino}propyl]pyrrolidin-1-yl}-5-methyl-1-oxoheptan-4-yl]-*N*-methyl-L-valinamid als Trifluoressigsäure-Salz hergestellt. Aus 14 mg (0.016 mmol) dieser Verbindung wurden dann in Analogie zur Herstellung von Beispiel 6 durch Umsetzung mit 4-Oxobutansäure in Gegenwart von Natriumcyanoborhydrid 11.3 mg (83%d. Th.) der Titelverbindung erhalten.

HPLC (Methode 5): Rₜ =1.9 min;

LC-MS (Methode 2): Rₜ = 1.27 min; MS (ESIpos): m/z = 871 (M+H)⁺.

### Beispiel 12

### N-(3-Carboxypropyl)-N-methyl-L-valyl-N-[(3R,4S,5S)-1-{(2S)-2-[(1R,2R)-3-{[(1S,2R)-1-(ethoxycarbonyl)-2-phenylcyclopropyl]amino}-1-methoxy-2-methyl-3-oxopropyl]pyrrolidin-1-yl}-3-methoxy-5-methyl-1-oxoheptan-4-yl]-N-methyl-L-valinamid

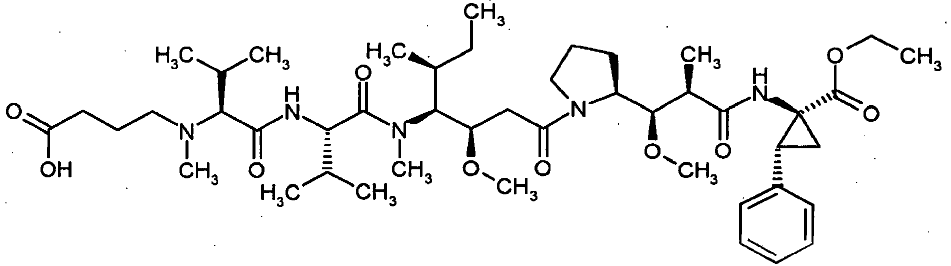

Zunächst wurde in Analogie zu der in Beispiel 10 beschriebenen Synthese durch Kupplung von *N-*(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) und Ethyl-(1*S*,2*R*)-1-amino-2-phenylcyclopropancarboxylat Trifluoressigsäure-Salz (Intermediat 17) in Gegenwart von O-(7-Azabenzotriazol-1-yl)-*N,N,N',N'-*tetramethyluronium-hexafluorophosphat und anschließende Abspaltung der Boc-Schutzgruppe mittels Trifluoressigsäure die Amin-Verbindung *N-*Methyl-L-valyl-*N-*[(3*R*,4*S*,5*S*)-1-{(2*S*)-2-[(1*R*,2*R*)-3-{[(1*S*,2*R*)-1-(ethoxycarbonyl)-2-phenylcyclopropyl]amino}-1-methoxy-2-methyl-3-oxopropyl]pyrrolidin-1-yl}-3-methoxy-5-methyl-1-oxoheptan-4-yl]-*N-*methyl-L-valinamid als Trifluoressigsäure-Salz hergestellt. Aus 70 mg (0.079 mmol) dieser Verbindung wurden dann in Analogie zur Herstellung von Beispiel 6 durch Umsetzung mit 4-Oxobutansäure in Gegenwart von Natriumcyanoborhydrid 46 mg (68% d. Th.) der Titelverbindung erhalten.

HPLC (Methode 5): Rₜ = 1.9 min;

LC-MS (Methode 2): Rₜ = 1.28 min; MS (ESIpos): m/z = 858 (M+H)⁺

¹H-NMR (500 MHz, DMSO-d₆): δ = 8.95 und 8.8 (2m, 1H), 8.85 und 8.7 (2s, 1H), 7.4-7.1 (m, 5H), 4.8 und 4.65 (2m, 1H), 4.55 (m, 1H), 4.12-3.95 (m, 2H), 3.9-3.8 (m, 1H), 3.8-3.4 (m, 5H), 3.35, 3.30, 3.20, 3.15, 3.10, 3.00, 2.81 und 2.79 (8s, 12H), 2.85-2.7 (m, 2H), 2.7-2.6 (m, 1H), 2.4-2.2 (m, 3H), 2.1-1.6 (m, 9H), 1.5-1.2 (m, 3H), 1.2-0.7 (m, 24H) [weitere Signale unter Lösungsmittel-Peaks verborgen].

### Beispiel 13

### N-(3-Carboxypropyl)-N-methyl-L-valyl-N-[(3R,4S,5S)-1-{(2S)-2-[(1R,2R)-3-{[(2S)-1-amino-1-oxo3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

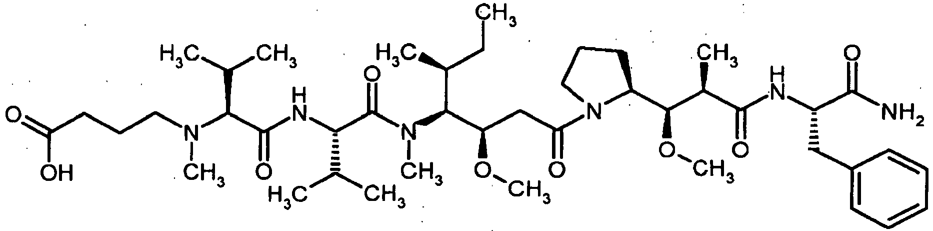

Zunächst wurde in Analogie zu der in Beispiel 10 beschriebenen Synthese durch Kupplung von *N-*(*tert*.-Butoxycarbonyl)-*N*-methyl-L-valyl-*N*-[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) und L-Phenylalaninamid-Hydrochlorid in Gegenwart von *O*-(7-Azabenzotriazol-1-yl)-*N*,*N,N'*-*N'-*tetramethyluronium-hexafluorophosphat und anschließende Abspaltung der Boc-Schutzgruppe mittels Trifluoressigsäure die Amin-Verbindung *N*-Methyl-L-valyl-*N*-[(3*R*,4*S*,5*S*)-1-{(2*S*)-2-[(1*R*,2*R*)-3-{[(2*S*)-1-amino-1-oxo-3-phenylpropan-2-yl]amino}-1-methoxy-2-methyl-3-oxopropyl]-Pyrrolidin-1-yl}-3-methoxy-5-methyl-1-oxoheptan-4-yl]-*N*-methyl-L-valinamid als Trifluoressigsäure-Salz hergestellt. Aus 47 mg (0.049 mmol) dieser Verbindung wurden dann in Analogie zur Herstellung von Beispiel 6 durch Umsetzung mit 4-Oxobutansäure in Gegenwart von Natriumcyanoborhydrid 39 mg (96% d. Th.) der Titelverbindung erhalten.

HPLC (Methode 5): Rₜ = 1.7 min;

LC-MS (Methode 9): Rₜ = 4.44 min; MS (ESIpos): m/z = 817 (M+H)⁺

¹H-NMR (500 MHz, DMSO-d₆): δ = 8.95 und 8.8 (2m, 1H), 8.25 und 8.0 (2d, 1H), 7.45, 7.35 und 7.0 (3s, breit, 2H), 7.3-7.1 (m, 5H), 4.8-4.4 (2m, 3H), 3.95 (m, 1H), 3.82 (m, 1H), 3.72 (d, 1H), 3.22, 3.18, 3.15, 3.05 und 3.00 (5s, 9H), 2.85-2.7 (m, 4H), 2.45-1.6 (m, 12H), 1.5-1.2 (m, 3H), 1.1-0.7 (m, 21H) [weitere Signale unter Lösungsmittel-Peaks verborgen].

### Beispiel 14

### (3R,4S,7S,10S)-3-(2-{(2S)-2-[(1R,2R)-3-{[(2S)-1-(2-Benzoylhydrazino)-1-oxo-3-phenylpropan-2-yl]amino}-1-methoxy-2-methyl-3-oxopropyl]pyrrolidin-1-yl}-2-oxoethyl)-4-[(2S)-butan-2-yl]-7,10-diisopropyl-5,11-dimethyl-6,9-dioxo-2-oxa-5,8,11-triazapentadecan-15-säure

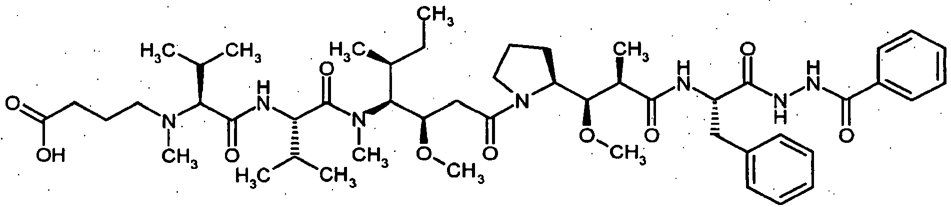

6.2 mg (6 µmol) (2*S*)-*N*-[(3*R*,4*S*,5*S*)-1-{(2*S*)-2-[(1*R*,2*R*)-3-{[(2*S*)-1-(2-Benzoylhydrazino)-1-oxo-3-phenylpropan-2-yl]amino}-1-methoxy-2-methyl-3-oxopropyl]pyrrolidin-1-yl}-3-methoxy-5-methyl-1-oroheptan-4-yl]-*N*,3-dimethyl-2-{[(2*S*)-3-methyl-2-(methylamino)butanoyl]amino}butanamid Trifluoressigsäure-Salz (Intermediat 37) und 8.3 µl einer 15%-igen wässrigen Bernsteinaldehydsäure-Lösung (13 µmol) wurden in 235 µl eines 1:1-Dioxan/Wasser-Gemisches gelöst und für 1 h auf 100°C erhitzt. Nach kurzem Abkühlen wurden 0.5 mg (7 µmol) Natriumcyanoborhydrid zugegeben. Das Reaktionsgemisch wurde durch Zugabe von 0.1 N Salzsäure auf pH 3 eingestellt und für weitere 2 h auf 100°C erhitzt. Nach erneuter Zugabe gleicher Mengen an Bernsteinaldehydsäure-Lösung, Natriumcyanoborhydrid und Salzsäure wurde nochmals für 2 h auf 100°C erhitzt. Das Reaktionsgemisch wurde danach direkt mittels präparativer HPLC in seine Komponenten aufgetrennt. Es wurden so 4.1 mg (68% d. Th.) der Titelverbindung erhalten.

LC-MS (Methode 1): Rₜ = 0.86 min; m/z = 936 (M+H)⁺

¹H-NMR (500 MHz, DMSO-d₆): δ [ppm] = 0.71-0.81 (m, 3H), 0.82-1.02 (m, 15H), 1.05 (d, 2H), 1.19-1.51 (m, 3H), 1.56-1.89 (m, 3H), 1.89-2.12 (m, 2H), 2.18-2,44 (m, 5H), 2.71-2.90 (m, 3H), 2.94-3.28 (m, 10H), 3.36-3.67 (m, 3H), 3.71-4.02 (m, 1H), 4.53-4.91 (m, 3H), 7.14-7.20 (m, 1H), 7.24 (dd, 2H), 7.31 (d, 1H), 7.35 (d, 1H), 7.52 (dd, 2H), 7.60 (dd, 1H), 7.92 (d, 2H), 8.24 und 8.46 (2d, 1H), 10.2-10.3 (m, 1H), 10.44-10.52 (m, 1H) [weitere Signale unter Lösungsmittel-Peaks verborgen].

**Beispiel 15**

### N-(3-Carboxypropyl)-N-methyl-L-valyl-N-[(3R,4S,5S)-1-{(2S-2-[(1R,2R)-3-{[(2S)-1-(benzyl-amino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

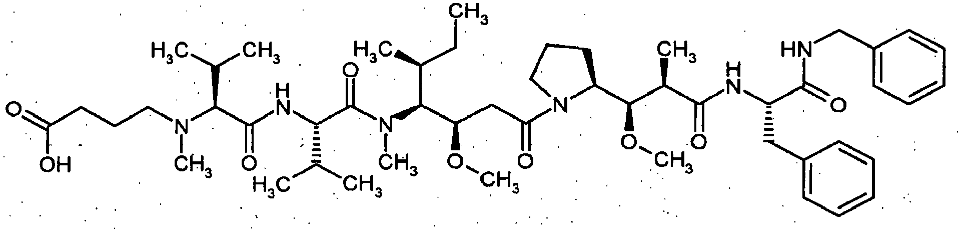

36 mg (43 µmol) *N*-(*tert*.-Butoxycarbonyl)-*N*-methyl-L-valyl-*N*-[(3*R*,4*S*,5*S*)-1-{(2*S*)-2-[(1*R*,2*R*)-3-{[(1*S*)*-*1-carboxy-2-phenylethyl]amino}-1-methoxy-2-methyl-3-oxopropyl]pyrrolidin-1-yl}-3-methoxy-5-methyl-1-oxoheptan-4-yl]-*N*-methyl-L-valinamid (Intermediat 39) und 4.6 mg (43 µmol) Benzylamin wurden in 5 ml DMF aufgenommen, mit 7.5 µl (88 µmol) *N*,*N*-Diisopropylethylamin, 10 mg (65 µmol) HOBt sowie 10 mg (52 µmol) EDC versetzt und dann über Nacht bei RT gerührt. Anschließend wurde das Reaktionsgemisch eingeengt und der Rückstand mittels präparativer HPLC gereinigt. Es wurden 29 mg (73% d. Th.) des Boc-geschützten Intermediats *N*-(*tert*.-Butoxycarbonyl)-*N-*methyl-L-valyl-*N-*[(3*R*,4*S*,5*S*)-1-{(2*S*)-2-[(1*R*,2*R*)-3-{[(2*S*)-1-(benzylamino)-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid erhalten.

LC-MS (Methode 1): Rₜ = 1.43 min; m/z = 921 (M+H)⁺.

29 mg dieses Intermediats wurden zur Abspaltung der Boc-Schutzgruppe mit 1 ml Trifluoressigsäure in 6 ml Dichlormethan behandelt. Nach Einengen und Lyophilisation aus Dioxan/Wasser wurden 30 mg (quant.) des freien Amin-Intermediats *N-*Methyl-L-valyl-*N-*[(3*R*,4*S*,5*S*)-1-{(2*S*)-2-[(1*R*,2*R*)-3-{[(2*S*)-1-(benzylamino)-1-oxo-3-phenylpropan-2-yl]amino}-1-methoxy-2-methyl-3-oxo-propyl]pyrrolidin-1-yl}-3-methoxy-5-methyl-1-oxoheptan-4-yl]-*N*-methyl-L-valinamid als Trifluoressigsäure-Salz erhalten.

LC-MS (Methode 1): Rₜ = 0.95 min; m/z = 821 (M+H)⁺.

Aus 17 mg (0.018 mmol) dieses Intermediats wurden dann in Analogie zur Herstellung von Beispiel 6 durch Umsetzung mit 4-Oxobutansäure in Gegenwart von Natriumcyanoborhydrid 13 mg (80% d. Th.) der Titelverbindung in Form eines farblosen Schaums erhalten.

HPLC (Methode 6): Rₜ = 1.7 min;

LC-MS (Methode 9): Rₜ = 4.97 min; MS (ESIpos): m/z = 907 (M+H)⁺.

### Beispiel 16

### N-(3-Carboxypropyl)-N-methyl-L-valyl-N-[(3R,4S,5S)-1-{(2S)-2-[(1R,2R)-3-{[(2S)-1-(benzyl-amino)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

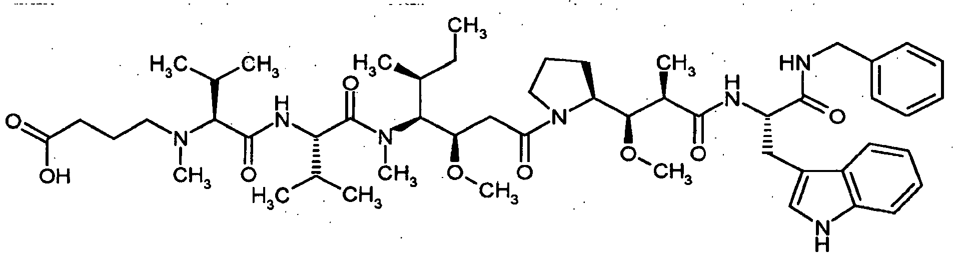

Zunächst wurde in Analogie zu der in Beispiel 10 beschriebenen Synthese durch Kupplung von N-(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) und *N-*Benzyl-L-tryptophanamid Trifluoressigsäure-Salz (Intermediat 40) in Gegenwart von *O-*(7-Azabenzotriazol-1-yl)-*N*,*N*,*N*'*N*',-tetramethyluronium-hexafluorophosphat und anschließende Abspaltung der Boc-Schutzgruppe mittels Trifluoressigsäure die Amin-Verbindung *N-*Methyl-L-valyl-*N*-[(3*R*,4*S*,5*S*)-1-{(2*S*)-2-[(1*R*,2*R*)-3-{[(2S)-1-(benzylamino)-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoressigsäure-Salz hergestellt. Aus 10 mg (0.01 mmol) dieser Verbindung wurden dann in Analogie zur Herstellung von Beispiel 6 durch Umsetzung mit 4-Oxobutansäure in Gegenwart von Natriumcyanoborhydrid 2.5 mg (26% d. Th.) der Titelverbindung erhalten.

HPLC (Methode 6): Rₜ = 1.7 min;

LC-MS (Methode 2): Rₜ = 1.13 min; MS (ESIpos): m/z = 946 (M+H)⁺.

### Beispiel 17

### N-(3-Carboxypropyl)-N-methyl-L-valyl-N-[(3R,4S,5S)-1-{(2S)-2-[(1R,2R)-3-{[(1S,2R)-1-carbamoyl-2-phenylcyclopropyl]amino}-1-methoxy-2-methyl-3-oxopropyl]pyrrolidin-1-yl}-3-methoxy-5-methyl-1-o_{YO}heptan-4-yl]-N methyl-L-valinamid

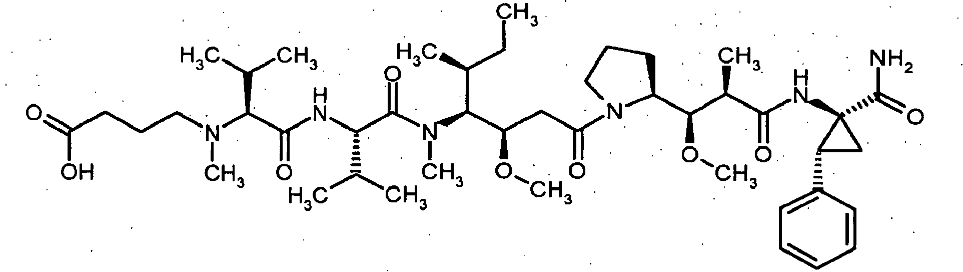

Zunächst wurde in Analogie zu der in Beispiel 10 beschriebenen Synthese durch Kupplung von *N-*(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) und (1*S*,2*R*)-1-Amino-2-phenylcyclopropancarboxamid Trifluoressigsäure-Salz (Intermediat 41) in Gegenwart von O-(7-Azabenzotriazol-1-yl)-*N,N,N',N*'-tetramethyluronium-hexafluorophosphat und anschließende Abspaltung der Boc-Schutzgruppe mittels Trifluoressigsäure die Amin-Verbindung *N*-Methyl-L-valyl-*N*-[(3*R*,4*S*,5*S*)-1-{(2*S*)-2-[1*R*,2*R*)-3-{[(1*R*,2*R*)-1-carbamoyl-2-phenyl-cyclopropyl]amino}-1-methoxy-2-methyl-3-oxopropyl]pyrrolidin-1-yl}-3-methoxy-5-methyl-1-oxoheptan-4-yl]-*N-*methyl-L-valinamid als Trifluoressigsäure-Salz hergestellt. Aus 15 mg (0.0175 mmol) dieser Verbindung wurden dann in Analogie zur Herstellung von Beispiel 6 durch Umsetzung mit 4-Oxobutansäure in Gegenwart von Natriumcyanoborhydrid 11 mg (76% d. Th.) der Titelverbindung erhalten.

HPLC (Methode 5): Rₜ = 1.7 min;

LC-MS (Methode 9): Rₜ = 4.66 min; MS (ESIpos): m/z = 829 (M+H)⁺.

### Beispiel 18

### N-(3-Carboxypropyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

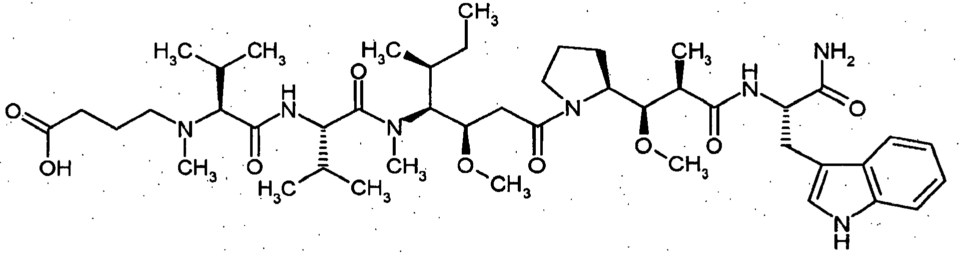

Zunächst wurde in Analogie zu den in den Beispielen 5 und 10 beschriebenen Synthesen durch Kupplung von *N*-[(9*H*-Fluoren-9-ylmethoxy)carbonyl)-*N*-methyl-L-valyl-*N*-[(2*R*,3*S*,4*S*)-1-carboxy-2-methoxy-4-methylhexan-3-yl]-*N*-methyl-L-valinamid (Intermediat 4) und N^{α}-{(2*R*,3*R*)-3-Methoxy-2-methyl-3-[(2*S*)-pyrrolidin-2-yl]propanoyl}-L-tryptophanamid Trifluoressigsäure-Salz (Intermediats 42) in Gegenwart von *O*-(7-Azabenzotriazol-1-yl)-N,N,N',N'-tetramethyluronium-hexafluorophosphat und anschließende Abspaltung der Fmoc-Schutzgruppe mittels Piperidin die Amin-Verbindung *N-*Methyl-L-valyl-*N-*[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoressigsäure-Salz hergestellt. Aus 78 mg (0.088 mmol) dieser Verbindung wurden dann in Analogie zur Herstellung von Beispiel 6 durch Umsetzung mit 4-Oxobutansäure in Gegenwart von Natriumcyanoborhydrid 68 mg (90% d. Th.) der Titelverbindung erhalten.

HPLC (Methode 6): Rₜ = 1.8 min;

LC-MS (Methode 9): Rₜ = 4.49 min; MS (ESIpos): m/z = 856 (M+H)⁺.

### Beispiel 19

### N-(5-Carboxypentyl)-N-methyl-L-valyl-N-[(3R,4S,5S)-1-{(2S)-2-[(1R,2R)-3-{[(2S)-1-amino-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

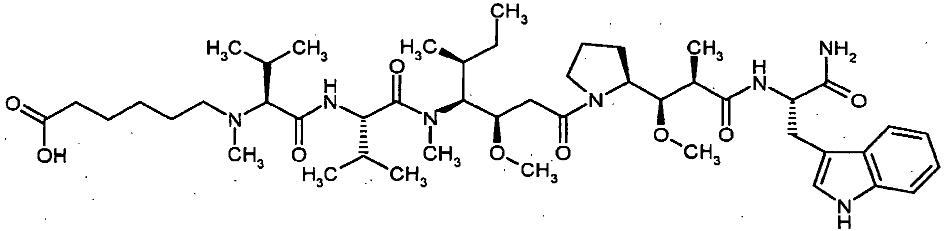

Die Titelverbindung wurde in Analogie zur Synthese von Beispiel 18 durch Umsetzung von 20 mg (26 µmol) *N*-Methyl-L-valyl-*N*-[(3*R*,4*S*,5*S*)-1-{(2*S*)-2-[(1*R*,2*R*)-3-{[(2*S*)-1-amino-3-(1*H*-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid Trifluoressigsäure-Salz mit 6-Oxohexansäure in Gegenwart von Natriumcyanoborhydrid hergestellt.

Ausbeute: 5 mg (25% d. Th.)

HPLC (Methode 6): Rₜ =1.6 min;

LC-MS (Methode 11): Rₜ = 0.72 min; MS (ESIpos): m/z = 884 (M+H)⁺.

### Beispiel 20

### N-(3-Carboxypropyl)-N-methyl-L-valyl-N [(3R,4S,5S)-1-{(2S)-2-[(1R,2R)-3-{[(2S)-1-tert.-butoxy-1-oxo-3-phenylpropan-2-yl]amino}-1-methoxy-2-methyl-3-oxopropyl]pyrrolidin-1-yl}-3-methoxy-5-methyl-1-oxoheptan-4-yl]-N-methyl-L-valinamid

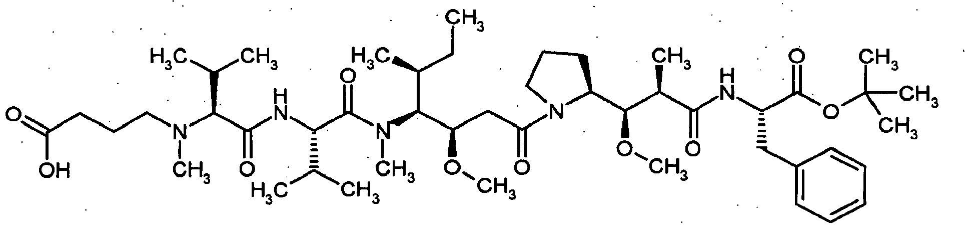

Zunächst wurde in Analogie zu der in Beispiel 16 beschriebenen Synthese durch Kupplung von *N-*(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxy-propyl]pyrrolidin-1-yl}-3-methoxy-5-methyl-1-oxoheptan-4-yl]-*N*-methyl-L-valinamid (Intermediat 15) und *tert*.-Butyl-L-phenylalaninat-Hydrochlorid in Gegenwart von *O*-(7-Azabenzotriazol-1-yl)-*N*,*N*,*N',N*'-tetramethyluronium-hexafluorophosphat und anschließende schonende Abspaltung der Boc-Schutzgruppe mittels Trifluoressigsäure unter Bewahrung der *tert*.-Butylester-Gruppe (40 min Rühren bei RT mit 10% Trifluoressigsäure in Dichlormethan) die Amin-Verbindung *N-*Methyl-L-valyl*-N-*[(3*R,*4*S,*5*S*)*-*1-{(2*S*)*-*2*-*[(1*R,*2*R)*-3-{[(2*S*)-1-*tert*.-butoxy-1-oxo-3-phenylpropan-2-yl]amino}-1-methoxy-2-methyl-3-oxopropyl]pyrrolidin-1-yl}-3-methoxy-5-methyl-1-oxoheptan-4-yl]-*N-*methyl-L-valinamid als Trifluoressigsäure-Salz hergestellt. Aus 22 mg (0.02 mmol, 80% Reinheit) dieser Verbindung wurden dann in Analogie zur Herstellung von Beispiel 1 durch Umsetzung mit 4-Oxobutansäure in Gegenwart von Natriumcyanoborhydrid 16 mg (94% d. Th.) der Titelverbindung erhalten.

HPLC (Methode 5): Rₜ = 2.0 min;

LC-MS (Methode 9): Rₜ = 5.05 min; MS (ESIpos): m/z = 874 (M+H)⁺.

### Beispiel 21

### N-(3-Carboxypropyl)-N-methyl-L-valyl-N-[(3R,4S,5S)-1-{(2S)-2-[(1R,2R)-3-{[(2S)-1-tert.-butoxy-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

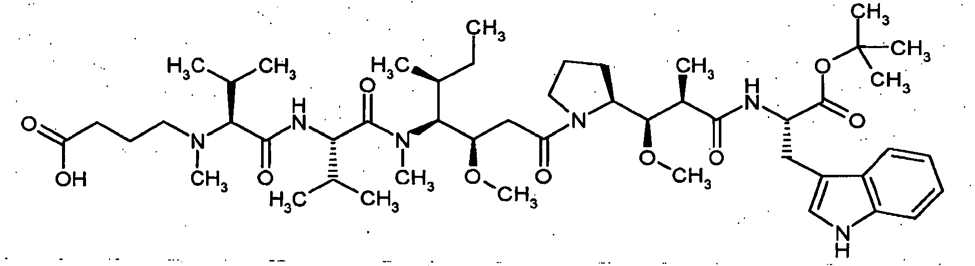

Die Herstellung der Titelverbindung erfolgte in Analogie zur in Beispiel 20 beschriebenen Synthese über drei Stufen ausgehend von 230 mg (336 µmol) *N*-(*tert*.-Butoxycarbonyl)-*N*-methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinaniid (Intermediat 15) und *tert*.-Butyl-L-tryptophanat-Hydrochlorid.

Ausbeute: 95 mg (31% d. Th. über drei Stufen)

HPLC (Methode 5): Rₜ = 2.0 min;

LC-MS (Methode 9): Rₜ = 5.05 min; MS (ESIpos): m/z = 913 (M+H)⁺.

### Beispiel 22

### N-(3-Carboxypropyl)-N-methyl-L-valyl-N-[(3R,4S,5S)-1-{(2S)-1-2-[(1R,2R)-3-{[(1S)-1-carboxy-2-(1H-indol-3-yl)ethyl]amino}-1-methoxy-2-methyl-3-oxopropyl]pyrrolidin-1-yl}-3-methoxy-5-methyl-1-oxoheptan-4-yl]-N-methyl-L-valinamid

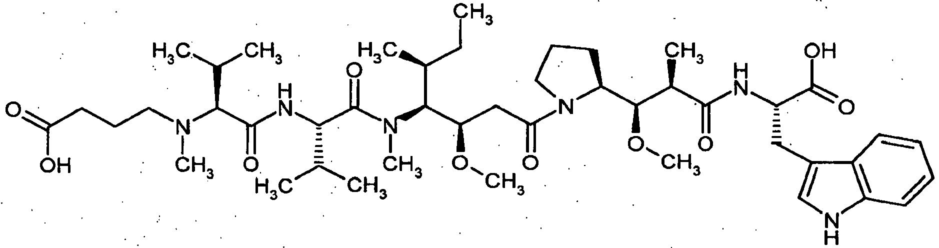

8 mg (9 µmol) der Verbindung aus Beispiel 21 wurden mit 1 ml Trifluoressigsäure in 3 ml Dichlormethan 4 h bei RT gerührt. Nach Einengen des Ansatzes im Vakuum wurde das Rohprodukt mittels präparativer HPLC gereinigt.

Ausbeute: 3 mg (37% d. Th.)

LC-MS (Methode 1): Rₜ = 0.77 min; MS (ESIpos): m/z = 857 (M+H)⁺.

### Beispiel 23

### N-(3-Carboxypropyl)-N-methyl-L-valyl-N-[(3R,4S,5S)-3-methoxy-1-{(2S)-2-[(1R,2R)-1-methoxy-2-methyl-3-{[(25)-1-(morpholin-4-yl)-1-oxo-3-phenylpropan-2-yl]amino}-3-oxopropyl]pyrrolidin-1-yl}-5-methyl-1-oxoheptan-4-yl]-N-methyl-L-valinamid

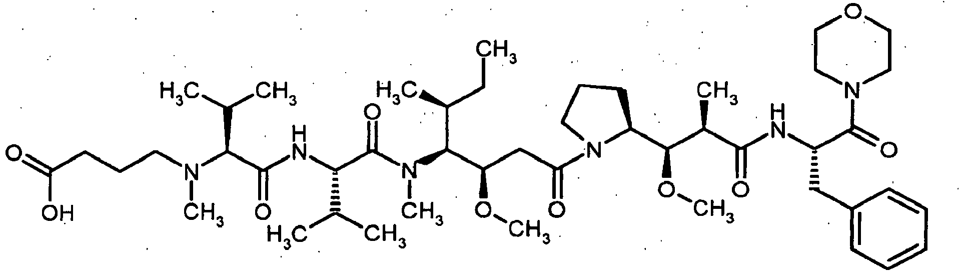

Zunächst wurde in Analogie zu der in Beispiel 15 beschriebenen Synthese durch Kupplung von *N-*(*tert*.-Butoxycarbonyl)-*N-*methyl-L-valyl-*N-*[(3*R*,4*S*,5*S*)-1-{(2*S*)-2-[(1*R*,2*R*)-3-{[(1*S*)-1-carboxy-2-phenylethyl]aminol-1-methoxy-2-methyl-3-oxopropyl]pyrrolidin-1-yl}-3-methoxy-5-methyl-1-oxo-heptan-4-yl]-*N-*methyl-L-valinamid (Intermediat 39) und Morpholin in Gegenwart von EDC und HOBt sowie anschließende Abspaltung der Boc-Schutzgruppe mittels Trifluoressigsäure die Amin-Verbindung *N*-Methyl-L-valyl-*N*-[(3*R*,4*S*,5*S*)-3-methoxy-1-{(2*S*)-2-[(1*R*,2*R*)-1-methoxy-2-methyl-3-{[(2*S*)-1-(morpholin-4-yl)-1-oxo-3-phenylpropan-2-yl]amino}-3-oxopropyl]pyrrolidin-1-yl}-5-methyl-1-oxoheptan-4-yl]-*N-*methyl-L-valinamid als Trifluoressigsäure-Salz hergestellt. Aus 30 mg (0.033 mmol) dieser Verbindung wurden dann in Analogie zur Herstellung von Beispiel 1 durch Umsetzung mit 4-Oxobutansäure in Gegenwart von Natriumcyanoborhydrid 22 mg (76% d. Th.) der Titelverbindung erhalten.

HPLC (Methode 6): Rₜ = 1.6 min;

LC-MS-(Methode 9): Rₜ = 4.58 min; MS (ESIpos): m/z = 887 (M+H)⁺.

### Beispiel 24

### N-(3-Carboxypropyl)-N-methyl-L-valyl-N-[(3R,4S,5S)-1-{(2S)-2-[(1R,2R)-3-{[(1S)-1-carboxy-2-phenylethyl]amino}-1-methoxy-2-methyl-3-oxopropyl]pyrrolidin-1-yl}-3-methoxy-5-methyl-1-oxo-heptan-4-yl]-N-methyl-L-valinamid

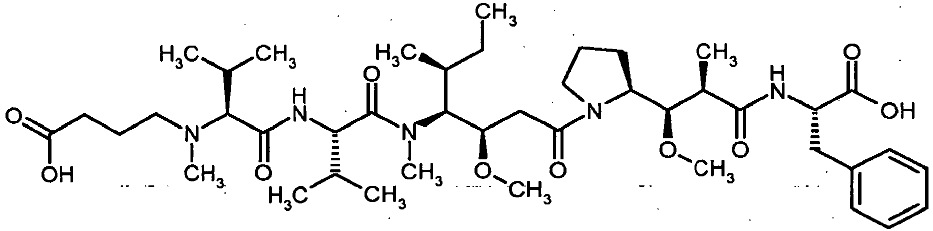

0.4 mg (0.5 µmol) der Verbindung aus Beispiel 20 wurden in 1 ml Dichlormethan aufgenommen und mit 1 ml Trifluoressigsäure versetzt. Nach 1 h Rühren bei RT wurde das Gemisch eingeengt und der Rückstand aus Dioxan/Wasser lyophilisiert. Es wurden 0.37 mg (99% d. Th.) der Titelverbindung als farbloser Schaum erhalten.

HPLC (Methode 5): Rₜ = 1.6 min;

LC-MS (Methode 1): Rₜ = 0.8 min; MS (ESIpos): m/z = 818 (M+H)⁺.

### Beispiel 25

### N-(3-Carboxypropyl)-N-methyl-L-valyl-N-[(3R,4S,5S)-1-{(2S)-2-[(1R,2R)-3-{[(2S,3R)-1-(benzyl-amino)-3-hydroxy-1-oxobutan-2-yl]amino}-1-methoxy-2-methyl-3-oxopropyl]pyrrolidin-1-yl}-3-methoxy-5-methyl-1-oxoheptan-4-yl]-N-methyl-L-valinamid

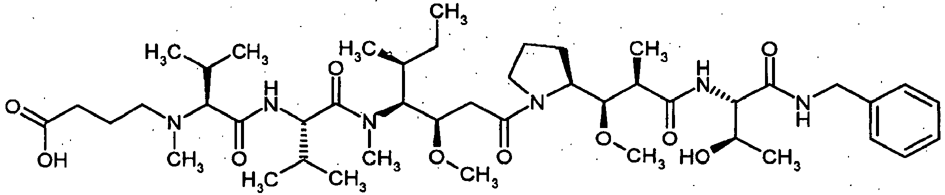

Zunächst wurde in Analogie zu der in Beispiel 16 beschriebenen Synthese durch Kupplung von *N-*(*tert*.-Butoxycarbonyl)-*N-*methyl-L-valyl-*N-*[(3*R*,4*S*,5*S*)-1-{(2*S*)-2-[(1*R*,2*R*)-2-carboxy-1-methoxypropyl]pyrrolidin-1-yl}-3-methoxy-5-methyl-1-oxoheptan-4-yl]-*N*-methyl-L-valinamid (Intermediat 15) und *N-*Benzyl-L-threoninamid Trifluoressigsäure-Salz in Gegenwart von *O*-(7-Azabenzotriazol-1-yl)-*N,N,N',N*'-tetramethyluronium-hexafluorophosphat und anschließende Abspaltung der Boc-Schutzgruppe mittels Trifluoressigsäure die Amin-Verbindung *N*-Methyl-L-valyl-*N*-[(3*R*,4*S*,5*S*)-1-{(2*S*)-2-[(1*R*,2*R*)-3-{[(2*S*,3*R*)-1-(benzylamino)-3-hydroxy-1-oxobutan-2-yl]amino}-1-methoxy-2-methyl-3-oxopropyl]pyrrolidin-1-yl}-3-methoxy-5-methyl-1-oxoheptan-4-yl]-*N*-methyl-L-valinamid als Trifluoressigsäure-Salz hergestellt. Aus 21 mg (0.024 mmol) dieser Verbindung wurden dann in Analogie zur Herstellung von Beispiel 1 durch Umsetzung mit 4-Oxobutansäure in Gegenwart von Natriumcyanoborhydrid 20 mg (97% d. Th.) der Titelverbindung erhalten.

HPLC (Methode 5): Rₜ = 1.5 min;

LC-MS (Methode 9): Rₜ = 4.49 min; MS (ESIpos): m/z = 861 (M+H)⁺.

### Beispiel 26

### N-(3-Carboxypropyl)-N-methyl-L-valyl-N-[(3R,4S,5S-3-methoxy-1-{(2S)-2-[(1R,2R)-1-methoxy-3-{[(2S)-1-{[4-(methoxycarbonyl)benzyl]oxy}-3-phenylpropan-2-yl]amino}-2-methyl-3-oxopropyl]-pyrrolidin-1-yl}-5-methyl-1-oxoheptan-4-yl]-N-methyl-L-valinamid

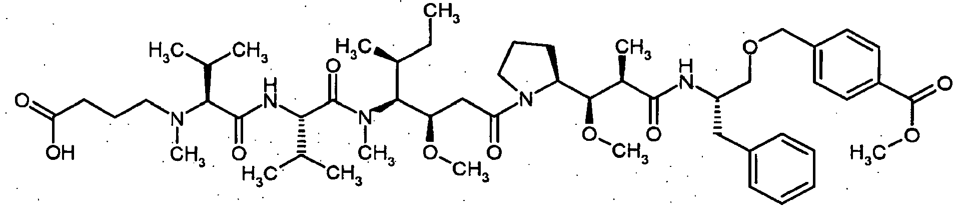

11.5 mg (12 µmol) *N-*Methyl-L-valyl-*N*-[(3*R*,4*S*,5*S*)-3-methoxy-1-{(2*S*)-2-[(1*R*,2*R*)-1-methoxy-3-{[(2*S*)-1-{[4-(methoxycarbonyl)benzyl]oxy}-3-phenylpropan-2-yl]amino}-2-methyl-3-oxopropyl]-pyrrolidin-1-yl}-5-methyl-1-oxoheptan-4-yl]-*N*-methyl-L-valinamid Trifluoressigsäure-Salz (Intermediat 46) wurden in 0.7 ml Dioxan/Wasser (1:1) gelöst und analog zur Herstellung von Beispiel 1 mit 15%-iger wässriger Lösung von 4-Oxobutansäure in Gegenwart von Natriumcyanoborhydrid umgesetzt. Nach Lyophilisation aus Dioxan wurden 8.3 mg (74% d. Th.) der Titelverbindung in Form eines weißen Feststoffs erhalten.

LC-MS (Methode 1): Rₜ = 0.95 min; MS (ESIpos): m/z = 953 (M+H)⁺.

### Beispiel 27

### N-(3-Carboxypropyl)-N-methyl-L-valyl-N-[(3R,4S,5S)-1-{(2S)-2-[(1R,2R)-3-({(2S)-1-[(4-carboxy-benzyl)oxy]-3-phenylpropan-2-yl}amino)-1-methoxy-2-methyl-3-oxopropyl]pyrrolidin-1-yl}-3-methoxy-5-methyl-1-oxoheptan-4-yl]-N-methyl-L-valinamid

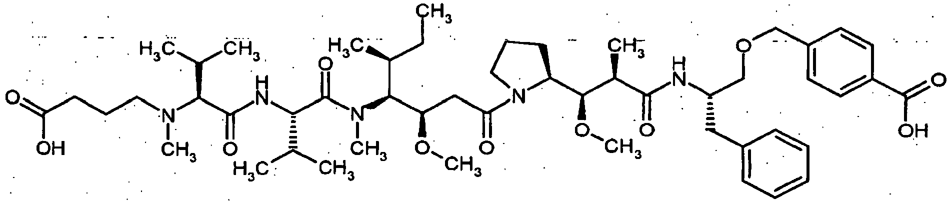

7.5 mg (8 µmol) *N*-(3-Carboxypropyl)-*N*-methyl-L-valyl-*N*-[(3*R*,4*S*,5*S*)-3-methoxy-1-{(2*S*)-2-[(1*R*,2*R*)-1-methoxy-3-{[(2*S*)-1-{[4-(methoxycarbonyl)benzyl]oxy}-3-phenylpropan-2-yl]amino}-2-methyl-3-oxopropyl]pyrrolidin-1-yl}-5-methyl-1-oxoheptan-4-yl]-*N*-methyl-L-valinamid (Beispiel 26) wurden in 0.25 ml THF/Wasser (1:1) gelöst. Es wurden 0.8 mg (32 µmol) Lithiumhydroxid zugegeben und der Ansatz 3 h bei RT gerührt. Anschließend wurde die Reaktionsmischung mit 1 N Salzsäure sauer gestellt und dreimal mit je 5 ml Ethylacetat extrahiert. Die vereinigten organischen Phasen wurden über Magnesiumsulfat getrocknet und im Vakuum eingeengt. Nach Lyophilisation aus Dioxan wurden 2.3 mg (71% Reinheit, 22% d. Th.) der Titelverbindung in Form eines weißen Feststoffs erhalten.

LC-MS (Methode 1): Rₜ = 0.91 min; MS (ESIpos): m/z = 939 (M+H)⁺.

### Beispiel 28

### N-(5-Carboxypentyl)-N-methyl-L-valyl-N-[(3R,4S,5S)-1-{(2S)-2-[(1R,2R)-3-{[(2S)-1-(benzyl-amino)-3-(1H-indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-N-methyl-L-valinamid

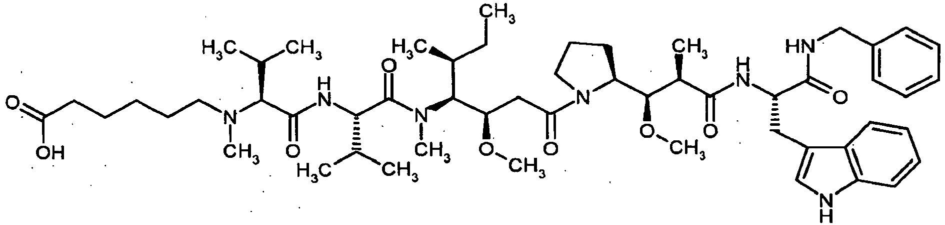

Die Titelverbindung wurde in Analogie zur Synthese von Beispiel 16 durch Umsetzung von 100 mg (103 µmol) *N*-Methyl-L-valyl-*N*-[(3*R*,4*S*,5*S*)-1-{(2*S*)-2-[(1*R*,2*R*)-3-{[(2*S*)-1-(benzylamino)-3-(1*H-*indol-3-yl)-1-oxopropan-2-yl]amino}-1-methoxy-2-methyl-3-oxopropyl]pyrrolidin-1-yl}-3-methoxy-5-methyl-1-oxoheptan-4-yl]-*N*-methyl-L-valinamid Trifluoressigsäure-Salz mit 6-Oxohexansäure in Gegenwart von Natriumcyanoborhydrid hergestellt.

Ausbeute: 40 mg (40% d. Th.)

HPLC (Methode 5): Rₜ = 1.9 min;

LC-MS (Methode 1): Rₜ = 0.92 min; MS (ESIpos): m/z = 974 (M+H)⁺.

### B. Bewertung der biologischen Wirksamkeit

Die biologische Aktivität der erfindungsgemäßen Verbindungen kann durch *in vitro-* und *in vivo-*Untersuchungen, wie sie dem Fachmann bekannt sind, nachgewiesen werden. Beispielsweise können die pharmakologischen und pharmakokinetischen Eigenschaften der erfindungsgemäßen Verbindungen mit Hilfe der nachstehend beschriebenen Assays bestimmt werden:

### B-1. Bestimmung der antiproliferativen Wirkung an der 786-O RCC-Zelllinie:

Eine definierte Zellzahl der humanen renalen Krebszelllinie 786-O wurde in einer 96-well-Mikrotiterplatte im Vollmedium ausgesät (2 500 oder 7 000 Zellen/well) und über Nacht bei 37°C / 5% CO₂ inkubiert. Nach 18 h wurde das Aussaatmedium durch serumfreies Medium oder Medium mit 2% FCS ersetzt. Die Behandlung startete mit Zugabe der jeweiligen Testsubstanz in variierender Konzentration (10⁻⁵ M bis 10⁻¹⁴ M): Es wurden Inkubationszeiten von 48 h bis 96 h gewählt. Die Proliferation wurde mit Hilfe des MTT-Assays (ATCC, Manassas, Virginia, USA, Katalog-Nr. 30-1010K) bestimmt. Nach Ablauf der Inkubationszeit wurde das MTT-Reagens für 4 h mit den Zellen inkubiert, bevor durch Zugabe des Detergens die Lyse der Zellen über Nacht erfolgte. Die Detektion des gebildeten Farbstoffs erfolgte bei 570nm. Die Proliferation nicht mit Testsubstanz, aber ansonsten identisch behandelter Zellen wurde als 100%-Wert definiert. Die aus diesem Test gewonnenen Daten repräsentieren Dreifachbestimmungen, und es wurden mindestens zwei unabhängige Experimente durchgeführt.

In der folgenden Tabelle 1 sind die IC₅₀-Werte repräsentativer Ausführungsbeispiele aus diesem Assay aufgeführt:

**Tabelle 1**

| **Ausführungsbeispiel** | **IC₅₀ [nM]** |
|---|---|
| 1 | 1.1 |
| 2 | 2.2 |
| 3 | 0.7 |
| 4 | 0.8 |
| 5 | 0.2 |
| 6 | 1.1 |
| 8 | 5.2 |
| 9 | 53 |
| 10 | 81 |
| 11 | 6.7 |
| 12 | 13 |
| 13 | 12.5 |
| 14 | 59 |
| 15 | 0.9 |
| 16 | 12 |
| 18 | 173 |
| 20 | 0.3 |
| 25 | 164 |
| 26 | 10 |

Im Vergleich hierzu weist Monomethylauristatin F (MMAF) in diesem Test einen IC₅₀-Wert von 260 nM auf.

### B-2. Bestimmung der antiproliferativen Wirkung an der HT29wt-Zelllinie:

Eine definierte Zellzahl der humanen Colonkarzinom-Zelllinie HT29wt (Wildtyp) wurde in einer 96-well-Mikrotiterplatte im Vollmedium (10% FCS-RPMI) ausgesät (2 500 Zellen/well) und über Nacht bei 37°C / 5% CO₂ inkubiert. Nach 18 h wurde das Aussaatmedium durch frisches Medium mit 10% FCS ersetzt. Die Behandlung startete mit Zugabe der jeweiligen Testsubstanz. Von den zu untersuchenden Substanzen wurden Dosis-Wirkungskurven in einem Konzentrationsbereich von 10⁻⁵ M bis 10⁻¹⁴ M (1:10-Verdünnungsreihe) bestimmt. Es wurden Inkubationszeiten von 48 h bis 96 h gewählt. Die Detektion der Proliferation erfolgte mit Hilfe des MTT-Assays (ATCC, Manassas, Virginia, USA, Katalog-Nr. 30-1010K). Nach Ablauf der gewählten Inkubationszeit wurde das MTT-Reagens für 4 h mit den Zellen inkubiert, bevor durch Zugabe des Detergens die Lyse der Zellen über Nacht erfolgte. Die Detektion des gebildeten Farbstoffs erfolgte bei 570 nm. Die Proliferation nicht mit Testsubstanz, aber ansonsten identisch behandelter Zellen wurde als 100%-Wert definiert. Die aus diesem Test gewonnenen Daten repräsentieren Dreifachbestimmungen, und es wurden mindestens zwei unabhängige Experimente durchgeführt.

In der folgenden Tabelle 2 sind die IC₅₀-Werte repräsentativer Ausführungsbeispiele aus diesem Assay aufgeführt:

**Tabelle 2**

| **Ausführungsbeispiel** | **IC₅₀ [nM]** |
|---|---|
| 1 | 0.1 |
| 4 | 0.1 |
| 6 | 0.4 |
| 8 | 0.5 |
| 9 | 4.4 |
| 11 | 0.8 |
| 13 | 1.0 |
| 16 | 0.1 |
| 18 | 16 |
| 25 | 1.5 |
| 26 | 1.5 |
| 28 | 1.3 |

Im Vergleich hierzu weist Monomethylauristatin F (MMAF) in diesem Test einen IC₅₀-Wert von 10 nM auf.

### B-3. Bestimmung des Einflusses auf die Tubulinpolymerisation:

Krebszellen sind entartete Zellen, die häufig auch durch eine gesteigerte Zellteilung zu einer Tumorbildung führen. Microtubuli bilden die Spindelfasern des Spindelapparates und sind ein essentieller

Bestandteil des Zellzyklus. Der geregelte Auf- und Abbau von Microtubuli ermöglicht die genaue Aufteilung der Chromosomen auf die Tochterzellen und stellt einen kontinuierlich dynamischen Prozess dar. Eine Störung dieser Dynamik führt zu einer fehlerhaften Zellteilung und letztlich zum Zelltod. Die gesteigerte Zellteilung von Krebszellen macht diese jedoch auch besonders empfänglich gegenüber Spindelfasergiften, die einen festen Bestandteil der Chemotherapie darstellen. Spindelfasergifte wie Paclitaxel oder Epothilon führen zu einer stark erhöhten Polymerisationsgeschwindigkeit der Microtubuli, während Vinca-Alkaloide oder auch Monomethylauristatin E (MMAE) zu einer stark reduzierten Polymerisationsgeschwindigkeit der Microtubuli führen. In beiden Fällen ist die notwendige Dynamik des Zellzyklus empfindlich gestört. Die im Rahmen der vorliegenden Erfindung untersuchten Verbindungen führen zu einer reduzierten Polymerisations-geschwindigkeit der Microtubuli.

Zur Untersuchung der Tubulinpolymerisation wurde das "Fluorescence-based Microtubule Polymerisation Assay Kit" der Firma Cytoskeleton (Denver, Colorado, USA; Bestellnummer: BK011) verwendet. Bei diesem Assay wird unpolymerisiertem Tubulin GTP zugesetzt, womit die Polymerisation spontan stattfinden kann. Der Assay beruht auf der Bindung des Fluorophors 4',6-Diamidino-2-phenylindol (DAPI) an Tubulin. Freies und gebundenes DAPI können aufgrund unterschiedlicher Emissionsspektra differenziert werden. Da DAPI eine deutlich höhere Affinität gegenüber polymerisiertem im Vergleich zu nicht-polymerisiertem Tubulin zeigt, läßt sich die Tubulinpolymerisation über die Zunahme der Fluoreszenz gebundener DAPI-Fluorophore verfolgen.

Zur Durchführung dieses Assays wurden die in DMSO gelösten Testsubstanzen von ihrer Ausgangskonzentration von 10 mM auf 1 µM in Wasser verdünnt. Neben der Puffer-Kontrolle wurde als Assay-Kontrolle auch polymerisationssteigernd Paclitaxel und andererseits polymerisationshemmend Vinblastin mitgeführt. Für die Messung wurden 96-well-Lochplatten mit halber Bodenfläche verwendet. Die Kinetik der Tubulinpolymerisation würde 1 h lang bei 37°C in einem Fluorimeter verfolgt. Die Anregungswellenlänge betrug 355 nm, die Emission wurde bei 460 nm verfolgt. Für den Bereich des linearen Anstiegs innerhalb der ersten 10 Minuten wurde die Fluoreszenzänderung pro Minute (ΔF/min) berechnet, welche die Polymerisationsgeschwindigkeit der Microtubuli darstellt. Die Potenz der Testsubstanzen wurde anhand der jeweiligen Reduktion der Polymerisationsgeschwindigkeit quantifiziert.

### B-4. Bestimmung der Plasma-Stabilität in vitro:

### Methode A:

1 mg der jeweiligen Testsubstanz wurde in 0.5 ml Acetonitril/DMSO (9:1) gelöst. Von dieser Lösung wurden 20 µl abgenommen und zu 1 ml 37°C warmen Ratten- bzw. Humanplasma gegeben (Plasma von männlichen Wistar-Ratten mit Li-Heparin, Fa. Harlan & Winkelmann bzw. humanes Leukozyten-depletiertes Frischplasma aus Vollblutentnahme). Aus dieser kräftig geschüttelten Plasmalösung wurden sofort nach Zugabe der Probe (Anfangswert als Bezugsgröße) und dann nach 5, 10, 30, 60, 120, 180 und 240 Minuten sowie gegebenenfalls nach 24 Stunden jeweils 100 µl-Aliquote entnommen und in 300 µl Acetonitril gegeben. Die ausgefällten Plasmaproteine wurden für 10 Minuten bei 5000 U/min abzentrifugiert und 30 µl des Überstandes per HPLC auf seinen Gehalt an unveränderter Testsubstanz analysiert. Quantifiziert wurde über die Flächenprozente der entsprechenden Peaks.

### HPLC-Methode bei Rattenplasma:

Instrument: Agilent 1200 mit DAD, binärer Pumpe, Autosampler, Säulenofen und Thermostat; Säule: Kromasil 100 C18, 250 mm x 4 mm, 5 µm; Säulentemperatur: 45°C; Eluent A: 5 ml Perchlorsäure / L Wasser, Eluent B: Acetonitril; Gradient: 0-8 min 98% A, 2% B; 8-15 min 56% A, 44% B; 15-20 min 10% A, 90% B; 20-21 min 10% A, 90% B; 21-23 min 98% A, 2% B; 23-25 min 98% A, 2% B; Flussrate: 2 ml/min; UV-Detektion; 220 nm.

### HPLC-Methode bei Humanplasma:

Instrument: Agilent 1100 mit DAD, binärer Pumpe, Autosampler, Säulenofen und Thermostat; Säule: Kromasil 100 C18, 250 mm x 4 mm, 5 µm; Säulentemperatur: 45°C; Eluent A: 5 ml Perchlorsäure / L Wasser, Eluent B: Acetonitril; Gradient: 0-3 min 98% A, 2% B; 3-10 min 65% A, 35% B; 10-15 min 40% A, 60% B; 15-21 min 10% A, 90% B; 21-22 min 10% A, 90% B; 22-24 min 98% A, 2% B; 24-26 min 98% A, 2% B; Flussrate: 2 ml/min; UV-Detektion: 220 nm.

### Methode B:

Die jeweilige Testsubstanz wurde in Ratten- bzw. Humanplasma bei 37°C über einen Zeitraum von 5 h unter leichtem Rühren inkubiert. Zu verschiedenen Zeitpunkten (0, 2, 5, 10, 20, 30, 60, 120, 180 und 300 Minuten) wurde jeweils ein 100 µl-Aliquot entnommen. Nach Zugabe von internem Standard (10 µl) wurden die Proteine durch Zugabe von 200 µl Acetonitril gefällt und das Gemisch in einer Eppendorf-Zentrifuge für 5 Minuten zentrifugiert. Nach Zugabe von 150 µl Ammoniumacetat-Puffer pH 3 zu 150 µl des Überstandes wurde der Gehalt an unveränderter Testsubstanz mittels LC/MSMS analysiert.

### B-5. Bestimmung der Zell-Permeabilität:

Die Zell-Permeabilität einer Substanz kann mittels *in vitro*-Testung in einem Flux-Assay unter Verwendung von Caco-2-Zellen untersucht werden [M.D. Troutman und D.R. Thakker, *Pharm. Res.* 20 *(8)*, 1210-1224 (2003)]. Hierzu wurden die Zellen auf 24-Loch-Filterplatten für 15-16 Tage kultiviert. Zur Bestimmung der Permeation wurde die jeweilige Testsubstanz in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als aktiv transportiert klassifiziert, wenn das Verhältnis von Pₐₚₚ (B-A) zu Pₐₚₚ (A-B) >2 oder <0.5 war.

Von entscheidender Bedeutung für Toxophore, die intrazellulär freigesetzt werden, ist die Permeabilität von B nach A [Pₐₚₚ (B-A)]: Je niedriger diese Permeabilität ist, desto länger ist die Verweilzeit der Substanz in der Zelle nach intrazellulärer Freisetzung und damit auch die Zeit, die für eine Interaktion mit dem biochemischen Target (hier: Tubulin) zur Verfügung steht.

In der folgenden Tabelle 3 sind Permeabilitätsdaten repräsentativer Ausführungsbeispiele aus diesem Assay aufgeführt:

**Tabelle 3**

| **Ausführungsbeispiel** | **Pₐₚₚ (B-A) [nm/s]** |
|---|---|
| 7 | 18 |
| 8 | 22 |
| 11 | 11 |
| 12 | 15 |
| 13 | 10 |
| 14 | 3 |
| 17 | 2 |
| 18 | 2 |
| 19 | 2 |
| 23 | 2 |

Im Vergleich hierzu weisen Monomethylauristatin E (MMAE) und Monomethylauristatin F (MMAF) in diesem Test einen Pₐₚₚ (B-A)-Wert von 89 nm/s beziehungsweise 73 nm/s auf.

### B-6. Bestimmung der Substrateigenschaften für P-Glycoprotein (P-gp):

Viele Tumorzellen exprimieren Transporterproteine für Wirkstoffe, was häufig mit einer Resistenzentwicklung gegenüber Cytostatika einhergeht. Substanzen, die keine Substrate von solchen Transporterproteinen wie beispielsweise P-Glycoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.H. Schinkel et al., J. Clin. Invest. 96, 1698-1705 (1995)]. Hierzu wurden die LLC-PK1- oder L-MDR1-Zellen auf 96-Loch-Filterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als P-gp-Substrat klassifiziert, wenn das Efflux-Verhältnis Pₐₚₚ (B-A) zu Pₐₚₚ (A-B) >2 war.

Als weitere Kriterien zur Bewertung der P-gp-Substrateigenschaften können die Efflux-Verhältnisse in L-MDR1- und LLC-PK1-Zellen oder das Efflux-Verhältnis in An- oder Abwesenheit eines Inhibitors miteinander verglichen werden. Wenn sich diese Werte um mehr als einen Faktor 2 unterscheiden, so handelt es sich bei der betreffenden Substanz um ein P-gp-Substrat.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 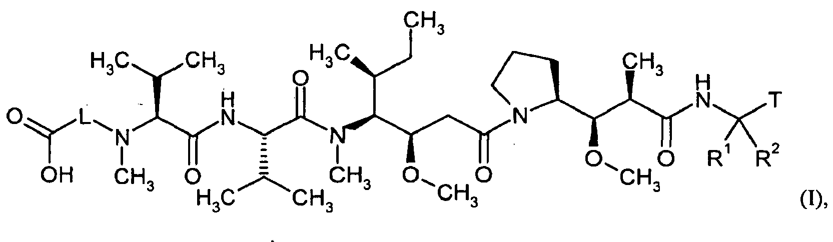 in welcher
L für geradkettiges (C₁-C₁₂)-Alkandiyl steht, das bis zu vierfach mit Methyl substituiert sein kann und in dem (*a*) zwei Kohlenstoffatome in 1,2-, 1,3- oder 1,4-Relation zueinander unter Einbezug der gegebenenfalls zwischen ihnen liegenden Kohlenstoffatome zu einem (C₃-C₆)-Cycloalkyl-Ring oder einem Phenyl-Ring verbrückt sein können oder (b) bis zu drei zueinander nicht benachbarte CH₂-Gruppen gegen -O- ausgetauscht sein können,
R¹ für Wasserstoff oder Methyl steht,
R² für Isopropyl, Isobutyl, *sec*.-Butyl, *tert.*-Butyl, 1-Hydroxyethyl, Phenyl, Benzyl, 4-Hydroxybenzyl, 1-Phenylethyl, Diphenylmethyl, 1*H*-Imidazol-4-ylmethyl oder 1*H*-Indol-3-ylmethyl steht,
oder
R¹ und R² zusammen mit dem Kohlenstoffatom, an das sie beide gebunden sind, eine 2-Phenylcyclopropan-1,1-diyl-Gruppe der Formel 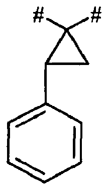 bilden, worin
# die Verknüpfungsstellen mit den übrigen Teilen des Moleküls kennzeichnet,
und
T für eine Gruppe der Formel -C(=O)-OR³, -C(=O)-NR⁴R⁵, -C(=O)-NH-NH-R⁶ oder -CH₂-O-R⁷ steht, worin
R³ Wasserstoff, (C₁-C₆)-Alkyl oder (C₃-C₁₀)-Cycloalkyl bedeutet,
wobei (C₁-C₆)-Alkyl mit Phenyl, Naphthyl oder (C₃-C₁₀)-Cycloalkyl substituiert sein kann,
R⁴ Wasserstoff oder (C₁-C₆)-Alkyl bedeutet,
R⁵ Wasserstoff, (C₁-C₆)-Alkyl oder (C₃-C₁₀)-Cycloalkyl bedeutet,
wobei (C₁-C₆)-Alkyl mit Phenyl substituiert sein kann,
oder
R⁴ und R⁵ miteinander verknüpft sind und zusammen mit dem Stickstoffatom, an das sie gebunden sind, einen 5- bis 7-gliedrigen, gesättigten Aza-Heterocyclus bilden, der ein weiteres Ring-Heteroatom aus der Reihe >N-H, >N-CH₃ oder -O- enthalten kann, das sich in 1,3- oder gegebenenfalls 1,4-Stellung in Relation zum erstgenannten Stickstoffatom befindet,
R⁶ (C₁-C₆)-Alkyl, (C₁-C₆)-Alkylcarbonyl, Phenyl oder Benzoyl bedeutet,
und
R⁷ (C₁-C₆)-Alkyl, das mit Phenyl substituiert sein kann, bedeutet,
wobei Phenyl seinerseits mit (C₁-C₆)-Alkoxycarbonyl oder Carboxyl substituiert sein kann,
sowie ihre Salze, Solvate und Solvate der Salze.

2. Verbindung der Formel (I) nach Anspruch 1, in welcher
L für geradkettiges (C₁-C₈)-Alkandiyl steht, in dem (*a*) zwei Kohlenstoffatome in 1,3- oder 1,4-Relation zueinander unter Einbezug des einen beziehungsweise der beiden zwischen ihnen liegenden Kohlenstoffatome zu einem Phenyl-Ring verbrückt sein können oder (*b*) bis zu zwei zueinander nicht benachbarte CH₂-Gruppen gegen -O- ausgetauscht sein können,
R¹ für Wasserstoff steht,
R² für Benzyl, 4-Hydroxybenzyl, 1-Phenylethyl oder 1*H*-Indol-3-ylmethyl steht,
oder
R¹ und R² zusammen mit dem Kohlenstoffatom, an das sie beide gebunden sind, eine 2-Phenylcyclopropan-1,1-diyl-Gruppe der Formel 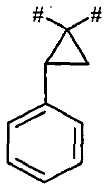 bilden, worin
# die Verknüpfungsstellen mit den übrigen Teilen des Moleküls kennzeichnet,
und
T für eine Gruppe der Formel -C(=O)-OR³, -C(=O)-NR⁴R⁵, -C(=O)-NH-NH-R⁶ oder -CH₂-O-R⁷ steht, worin
R³ Wasserstoff oder (C₁-C₄)-Alkyl, das mit Phenyl, Naphthyl oder (C₃-C₁₀)-Cycloalkyl substituiert sein kann, bedeutet,
R⁴ Wasserstoff oder Methyl bedeutet,
R⁵ Wasserstoff oder (C₁-C₄)-Alkyl, das mit Phenyl substituiert sein kann, bedeutet,
oder
R⁴ und R⁵ miteinander verknüpft sind und zusammen mit dem Stickstoffatom, an das sie gebunden sind, einen Piperidin- oder Morpholin-Ring bilden,
R⁶ (C₁-C₄)-Alkylcarbonyl oder Benzoyl bedeutet,
und
R⁷ (C₁-C₄)-Alkyl oder Benzyl, das in der Phenylgruppe mit (C₁-C₄)-Alkoxy-carbonyl oder Carboxyl substituiert sein kann, bedeutet,
sowie ihre Salze, Solvate und Solvate der Salze.

3. Verbindung der Formel (I) nach Anspruch 1 oder 2, in welcher
L für geradkettiges (C₁-C₆)-Alkandiyl steht,
R¹ für Wasserstoff steht,
R² für Benzyl, 1-Phenylethyl oder 1*H*-Indol-3-ylmethyl steht,
oder
R¹ und R² zusammen mit dem Kohlenstoffatom, an das sie beide gebunden sind, eine (1*S*,2*R*)-2-Phenylcyclopropan-1,1-diyl-Gruppe der Formel 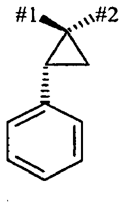 bilden, worin
#1 die Verknüpfungsstelle mit dem angrenzenden Stickstoffatom
und
#2 die Verknüpfungsstelle mit der Gruppe T kennzeichnen,
und
T für eine Gruppe der Formel -C(=O)-OR³, -C(=O)-NR⁴R⁵, -C(=O)-NH-NH-R⁶ oder -CH₂-O-R⁷ steht, worin
R³ Wasserstoff, Methyl, Ethyl, *n*-Propyl, Benzyl oder Adamantylmethyl bedeutet,
R⁴ Wasserstoff oder Methyl bedeutet,
R⁵ Wasserstoff, Methyl, Ethyl, *n*-Propyl oder Benzyl bedeutet,
R⁶ Benzoyl bedeutet,
und
R⁷ Benzyl, das in der Phenylgruppe mit Methoxycarbonyl oder Carboxyl substituiert sein kann, bedeutet,
sowie ihre Salze, Solvate und Solvate der Salze.

4. Verbindung gemäß Anspruch 1, 2 oder 3 mit der Formel (I-A) 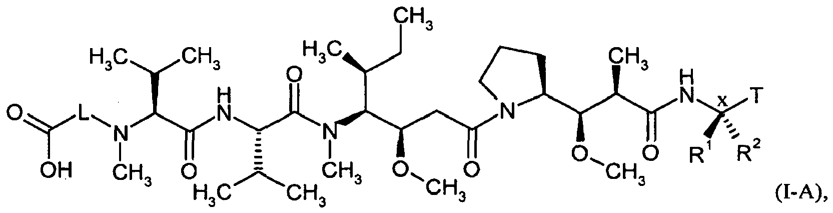
in welcher L, R¹, R² und T die in Anspruch 1, 2 oder 3 definierten Bedeutungen haben und das die Reste R¹ und R² tragende C^{X}-Kohlenstoffatom die abgebildete Absolutkonfiguration aufweist,
sowie ihre Salze, Solvate und Solvate der Salze.

5. Die Verbindung nach Anspruch 1, wobei diese Verbindung ausgewählt wird aus der Gruppe bestehend aus: 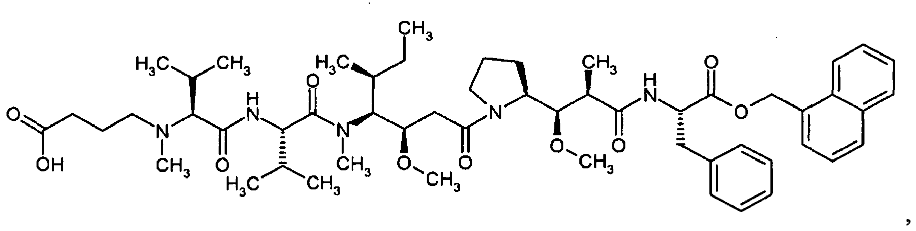 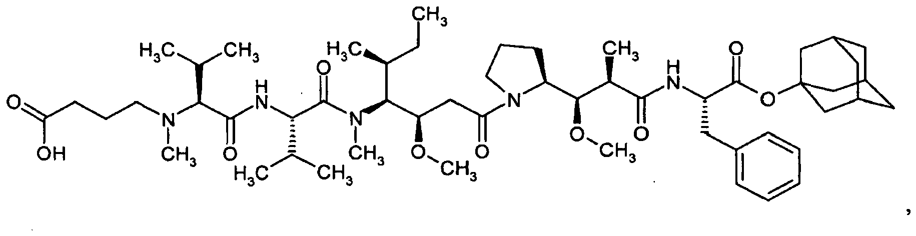 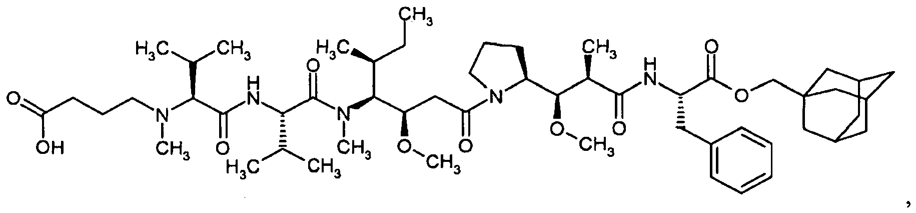 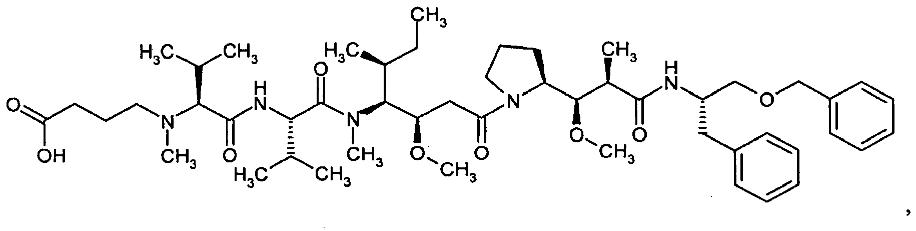 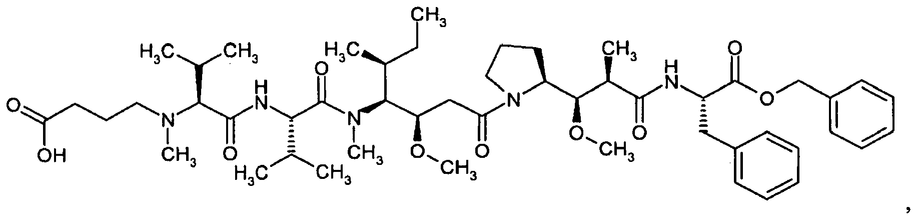 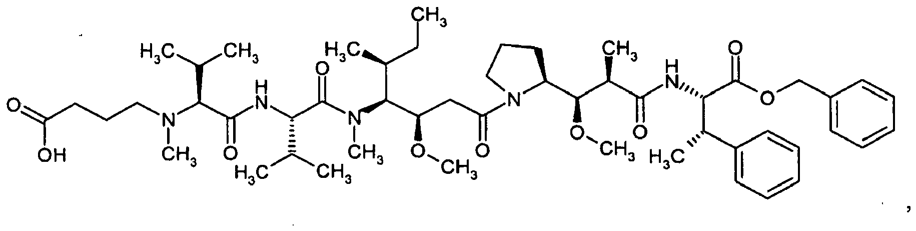 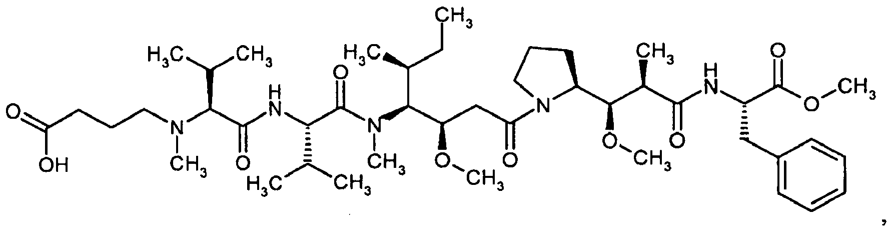 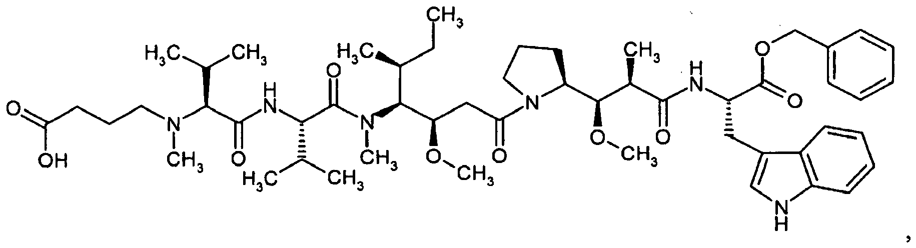 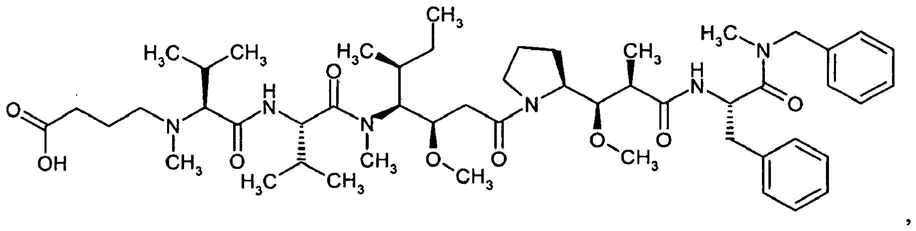 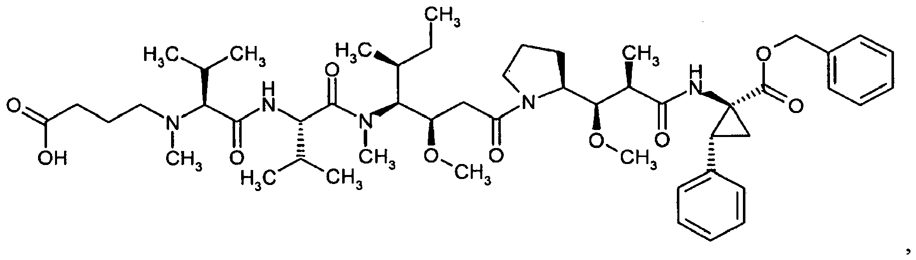 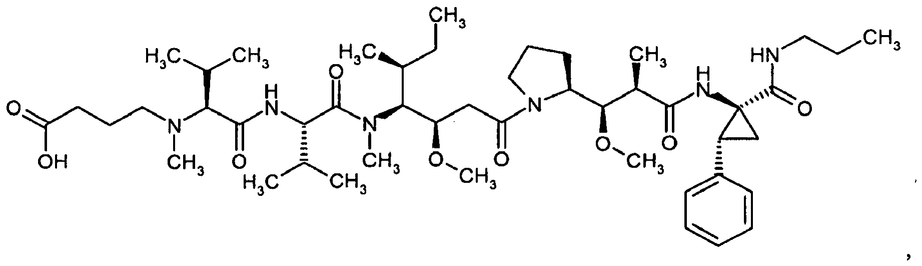 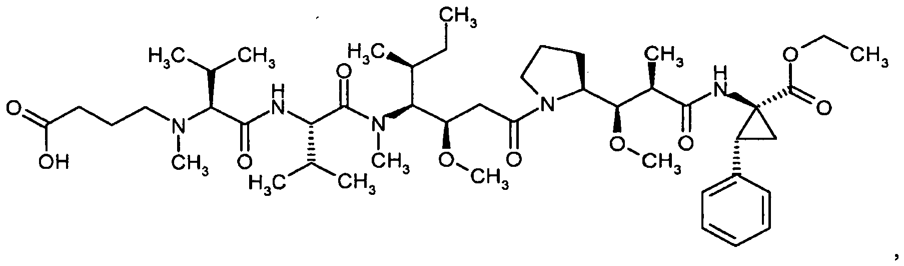 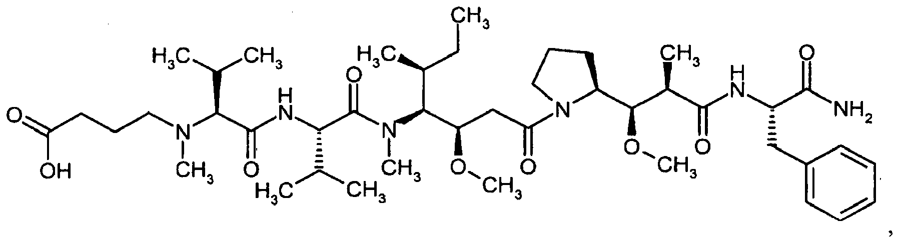 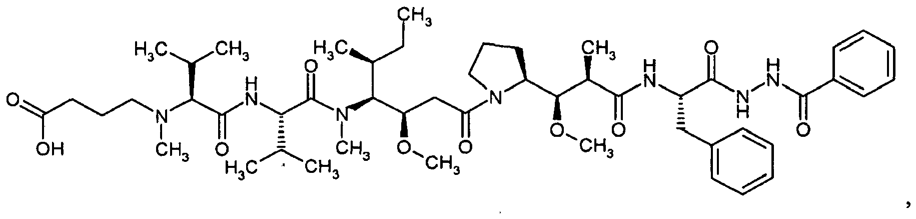 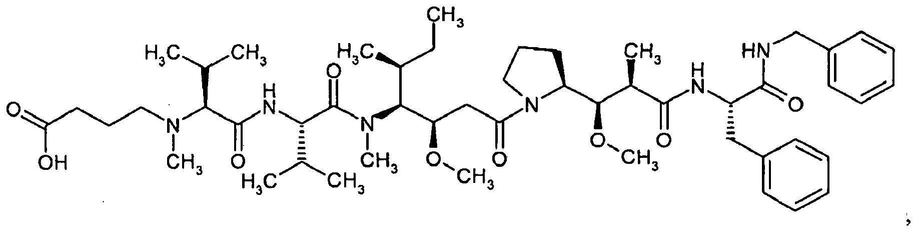 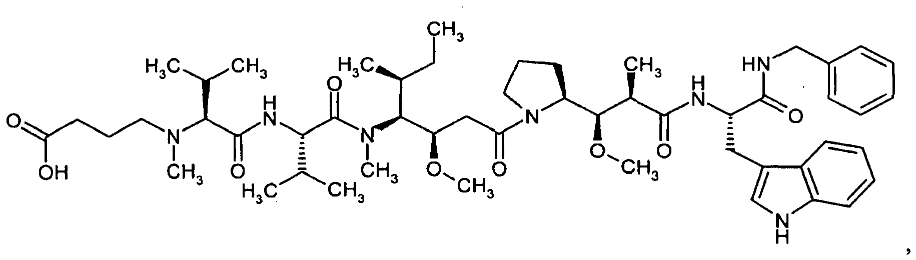 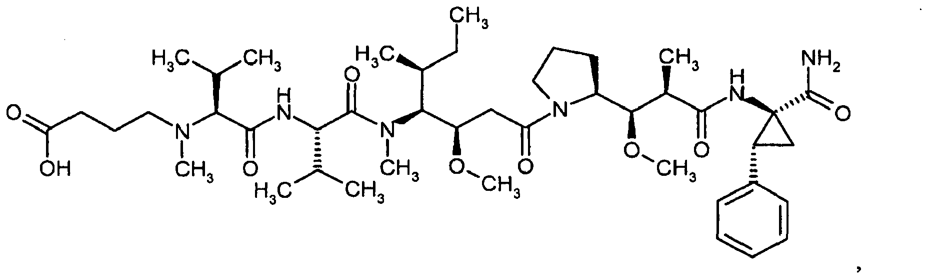 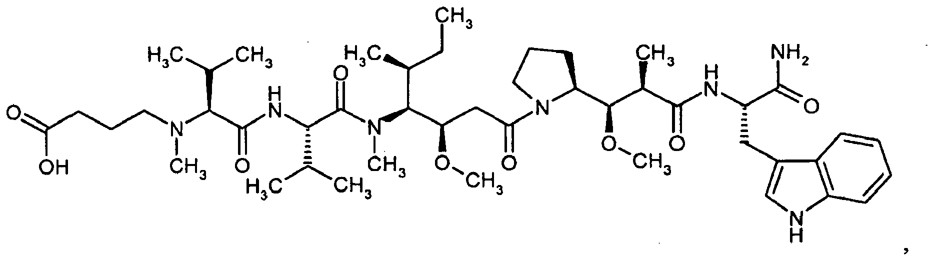 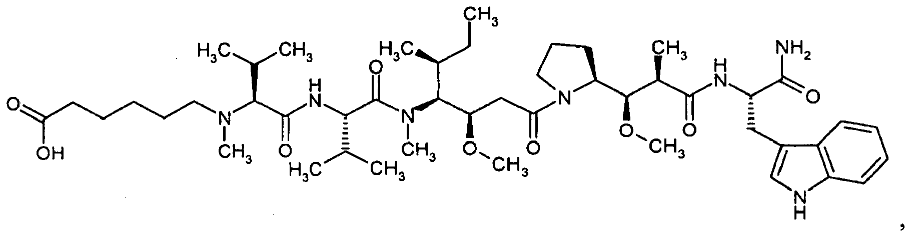 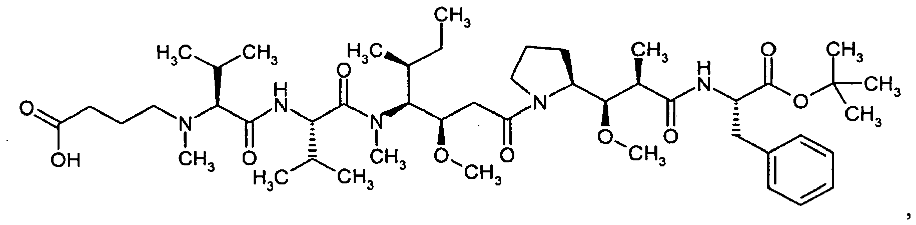 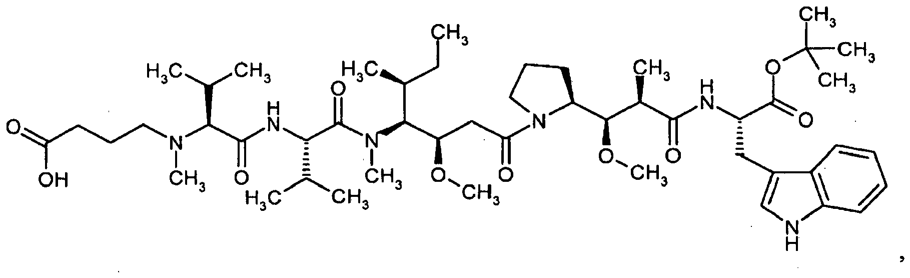 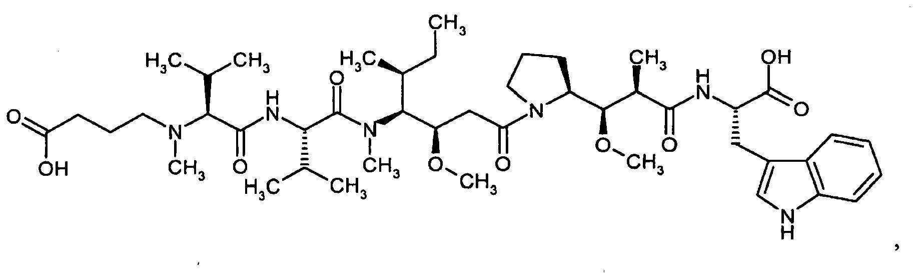 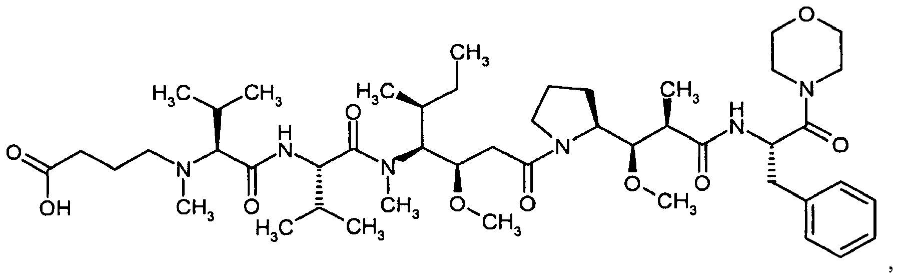 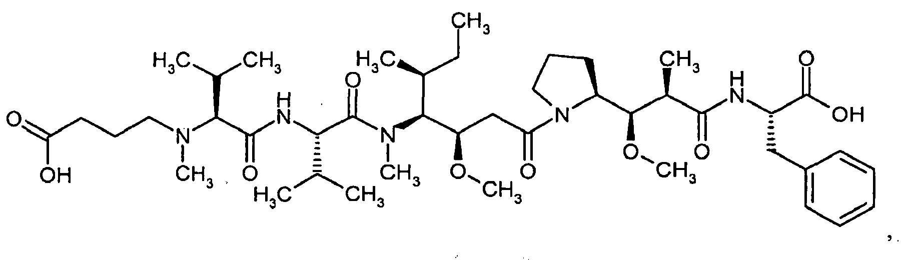 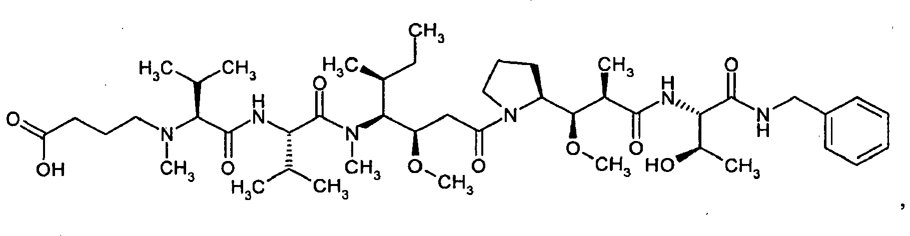 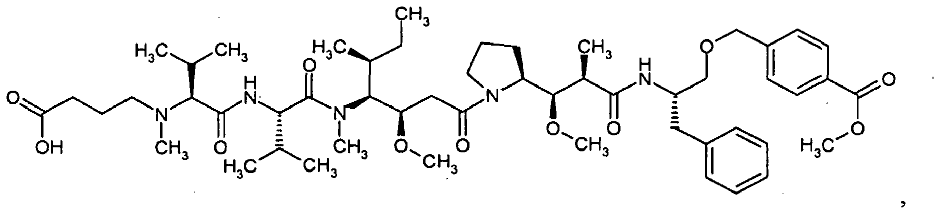 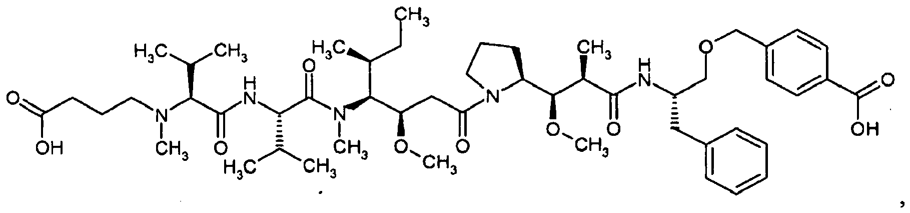 and 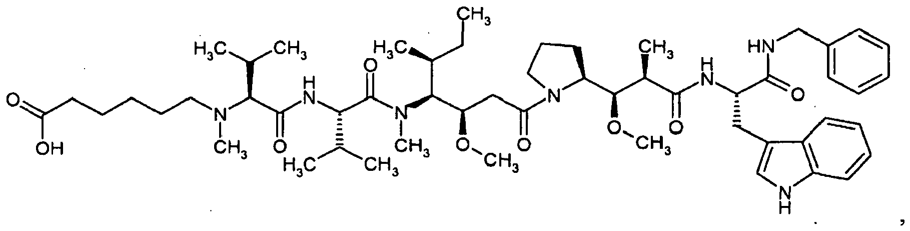 und deren Salze, Solvate und Solvate der Salze.

6. Verfahren zur Herstellung einer Verbindung der Formel (I), wie in den Ansprüchen 1 bis 5 definiert, **dadurch gekennzeichnet, dass** man eine Verbindung der Formel (II) 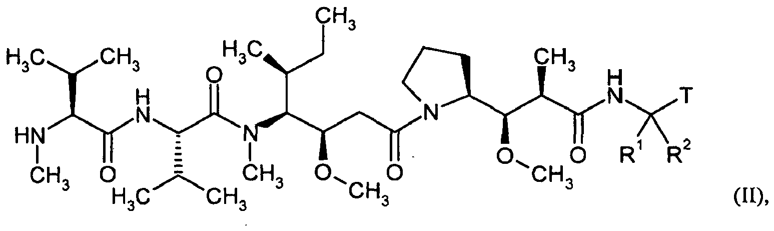 in welcher R¹, R² und T die in den Ansprüchen 1 bis 4 angegebenen Bedeutungen haben, in einem inerten Lösungsmittel entweder
[A] durch baseninduzierte Alkylierung mit einer Verbindung der Formel (III) 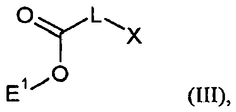 in welcher L die in den Ansprüchen 1 bis 4 angegebene Bedeutung hat,
E¹ für Wasserstoff, (C₁-C₄)-Alkyl oder Benzyl steht,
und
X für eine Fluchtgruppe wie beispielsweise Chlorid, Bromid, Iodid, Mesylat, Triflat oder Tosylat steht,
zu einer Verbindung der Formel (IV) 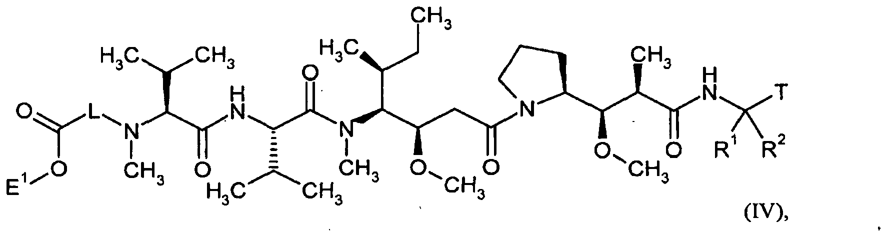 in welcher E¹, L, R¹, R² und T die oben angegebenen Bedeutungen haben,
umsetzt und anschließend im Fall, dass E¹ für (C₁-C₄)-Alkyl oder Benzyl steht, diesen Ester-Rest nach üblichen Methoden abspaltet, so dass ebenso wie im Fall, dass E¹ in (III) für Wasserstoff steht, die erfindungsgemäße Carbonsäure der Formel (I) 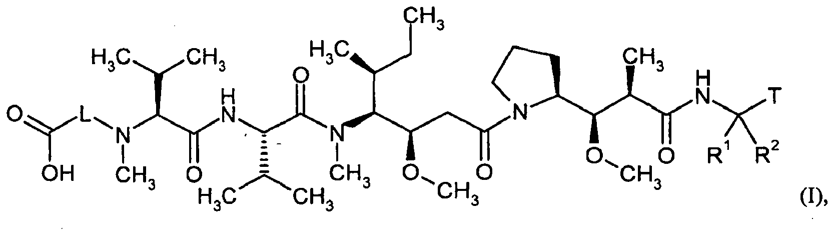 in welcher L, R¹, R² und T die oben angegebenen Bedeutungen haben, erhalten wird,
oder
[B] durch Umsetzung mit einer Verbindung der Formel (V) 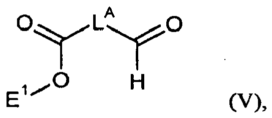 in welcher
E¹ für Wasserstoff, (C₁-C₄)-Alkyl oder Benzyl steht,
und
L^{A} die in den Ansprüchen 1 bis 4 definierte Bedeutung von L hat, jedoch in der AlkylKettenlänge um eine CH₂-Einheit verkürzt ist,
in Gegenwart eines geeigneten Reduktionsmittels in eine Verbindung der Formel (VI) 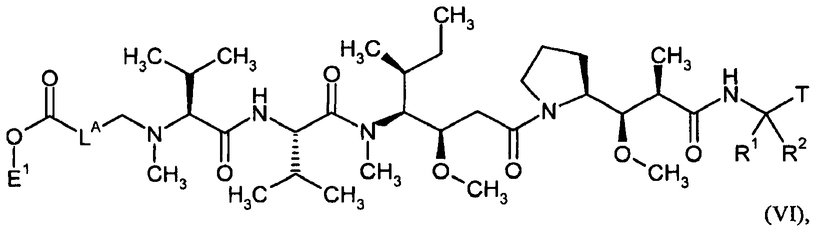 in welcher E¹, L^{A}, R¹, R² und T die oben angegebenen Bedeutungen haben,
überführt und anschließend im Fall, dass E¹ für (C₁-C₄)-Alkyl oder Benzyl steht, diesen Ester-Rest nach üblichen Methoden abspaltet, so dass ebenso wie im Fall, dass E¹ in (V) für Wasserstoff steht, die erfindungsgemäße Carbonsäure der Formel (I-B) 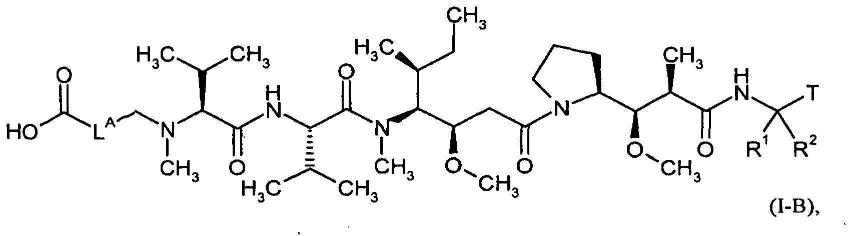 in welcher L^{A}, R¹, R² und T die oben angegebenen Bedeutungen haben,
erhalten wird,
und die resultierenden Verbindungen der Formel (I) bzw. (I-B) gegebenenfalls in ihre Enantiomere und/oder Diastereomere trennt und/oder mit den entsprechenden (i) Lösungsmitteln und/oder (*ii*) Basen oder Säuren zu ihren Solvaten, Salzen und/oder Solvaten der Salze umsetzt.

7. Verbindung, wie in einem der Ansprüche 1 bis 5 definiert, zur Verwendung in einem Verfahren zur Behandlung und/oder Prävention von Krankheiten.

8. Verbindung, wie in einem der Ansprüche 1 bis 5 definiert, zur Verwendung in einem Verfahren zur Behandlung und/oder Prävention von Krebs- und Tumorerkrankungen.

9. Verwendung einer Verbindung, wie in einem der Ansprüche 1 bis 5 definiert, zur Herstellung eines Arzneimittels zur Behandlung und/oder Prävention von Krebs- und Tumorerkrankungen.

10. Arzneimittel enthaltend eine Verbindung, wie in einem der Ansprüche 1 bis 5 definiert, in Kombination mit einem oder mehreren inerten, nicht-toxischen, pharmazeutisch geeigneten Hilfsstoffen.

11. Arzneimittel enthaltend eine Verbindung, wie in einem der Ansprüche 1 bis 5 definiert, in Kombination mit einem oder mehreren weiteren Wirkstoffen.

12. Arzneimittel nach Anspruch 10 oder 11 zur Verwendung in einem Verfahren zur Behandlung und/oder Prävention von Krebs-und Tumorerkrankungen.

13. Antiproliferativ wirkendes Konjugat, bei dem die Verbindung nach einem oder mehreren der Ansprüche 1-5 mit einem Protein verknüpft ist.

14. Antiproliferativ wirkendes Konjugat nach Anspruch 13, wobei das Protein ein Antikörper ist.

15. Antiproliferativ wirkendes Konjugat nach Anspruch 14, bei dem die Verbindung nach einem oder mehreren der Ansprüche 1-5 über die N-terminale Carboxyalkyl-Gruppierung mit dem Antikörper verbunden ist.

## Claims

1. Compound of the formula (I) 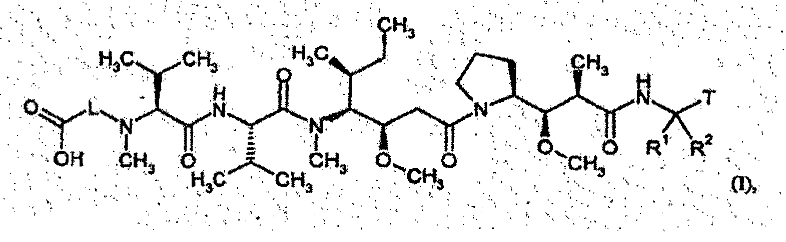 in which
L represents straight-chain (C₁-C₁₂) alkanediyl which may be substituted up to four times with methyl and in which (*a*) two carbon atoms in 1,2 relation, 1,3 relation or 1,4 relation to one another including the carbon atoms optionally lying between them may be bridged to form a (C₃-C₆) cycloalkyl ring or a phenyl ring or (*b*) up to three CH₂ groups not adjacent to one another may be exchanged for -O-,
R¹ represents hydrogen or methyl,
R² represents isopropyl, isobutyl, *sec*.-butyl, *tert.*-butyl, 1-hydroxyethyl, phenyl, benzyl, 4-hydroxybenzyl, 1-phenylethyl, diphenylmethyl, 1*H*-imidazol-4-ylmethyl or 1*H*-indol-3-ylmethyl,
or
R¹ and R² together with the carbon atom to which they are both bound, form a 2-phenylcyclopropan-1,1-diyl group of the formula 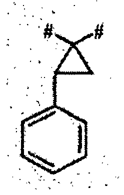 wherein
# indicates the points of attachment with the remaining parts of the molecule,
and
T represents a group of the formula -C(=O)-OR³, -C(=O)-NR⁴R⁵, -C(=O)-NH-NH-R⁶ or -CH₂-O-R⁷, wherein
R³ denotes hydrogen, (C₁-C₆) alkyl or (C₃-C₁₀) cycloalkyl,
wherein (C₁-C₆) alkyl may be substituted with phenyl, naphthyl or (C₃-C₁₀) cycloalkyl,
R⁴ denotes hydrogen or (C₁-C₆) alkyl,
R⁵ denotes hydrogen, (C₁-C₆) alkyl or (C₃-C₁₀) cycloalkyl,
wherein (C₁-C₆) alkyl may be substituted with phenyl,
or
R⁴ and R⁵ are linked to one another and together with the nitrogen atom to which they are bound, form a 5-membered to 7-membered, saturated aza-heterocycle, which may contain a further ring heteroatom from the series >N-H, >N-CH₃ or -O- which is located in 1,3-position or optionally 1,4-position in relation to the first-mentioned nitrogen atom,
R⁶ denotes (C₁-C₆) alkyl, (C₁-C₆) alkylcarbonyl, phenyl or benzoyl,
and
R⁷ denotes (C₁-C₆) alkyl which may be substituted with phenyl,
wherein phenyl in turn may be substituted with (C₁-C₆) alkoxycarbonyl or carboxyl,
as well as its salts, solvates and solvates of the salts.

2. Compound of the formula (I) according to claim 1, in which
L represents straight-chain (C₁-C₆) alkanediyl in which (*a*) two carbon atoms in 1,3 relation or 1,4 relation to one another including one or both carbon atoms lying between them may be bridged to form a phenyl ring or (*b*) up to two CH₂ groups not adjacent to one another may be exchanged for -O-,
R¹ represents hydrogen,
R² represents benzyl, 4-hydroxybenzyl, 1-phenylethyl or 1*H*-indol-3-ylmethyl,
or
R¹ and R² together with the carbon atom to which they are both bound, form a 2-phenylcyclopropan-1,1-diyl group of the formula 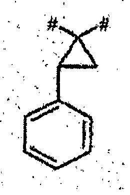 wherein
# indicates the points of attachment with the remaining parts of the molecule,
and
T represents a group of the formula -C(=O)-OR³, -C(=O)-NR⁴R⁵, -C(=O)-NH-NH-R⁶ or -CH₂-O-R⁷, wherein
R³ denotes hydrogen or (C₁-C₄) alkyl which may be substituted with phenyl, naphthyl or (C₃-C₁₀) cycloalkyl,
R⁴ denotes hydrogen or methyl,
R⁵ denotes hydrogen or (C₁-C₄) alkyl which may be substituted with phenyl,
or
R⁴ and R⁵ are linked to one another and together with the nitrogen atom to which they are bound, form a piperidine ring or morpholine ring,
R⁶ denotes (C₁-C₄) alkylcarbonyl or benzoyl,
and
R⁷ denotes (C₁-C₄) alkyl or benzyl which may be substituted in the phenyl group with (C₁-C₄) alkoxycarbonyl or carboxyl,
as well as its salts, solvates and solvates of the salts.

3. Compound of the formula (I) according to claim 1 or 2, in which
L represents straight-chain (C₁-C₆) alkanediyl,
R¹ represents hydrogen,
R² represents benzyl, 1-phenylethyl or 1*H*-indol-3-ylmethyl,
or
R¹ and R² together with the carbon atom to which they are both bound, form a (1*S*, 2*R*)-2-phenylcyclopropan-1,1-diyl group of the formula 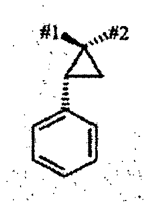 wherein
#1 indicates the point of attachment with the adjoining nitrogen atom
and
#2 indicates the point of attachment with the group T,
and
T represents a group of the formula -C(=O)-OR³, -C(=O)-NR⁴R⁵, -C(=O)-NH-NH-R⁶ or -CH₂-O-R⁷, wherein
R³ denotes hydrogen, methyl, ethyl, *n*-propyl, benzyl or adamantylmethyl,
R⁴ denotes hydrogen or methyl,
R⁵ denotes hydrogen, methyl, ethyl, *n*-propyl or benzyl,
R⁶ denotes benzoyl,
and
R⁷ denotes benzyl which may be substituted in the phenyl group with methoxycarbonyl or carboxyl,
as well as its salts, solvates and solvates of the salts.

4. Compound according to claim 1, 2 or 3 with the formula (I-A) 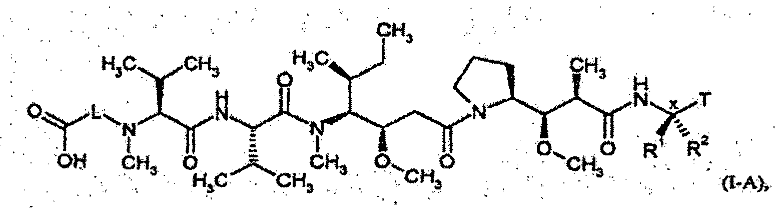 in which L, R¹, R² and T have the meanings defined in claim 1, 2 or 3 and the C^{x} carbon atom carrying the radicals R¹ and R² has the absolute configuration shown,
as well as its salts, solvates and solvates of the salts.

5. The compound according to claim 1, wherein this compound is selected from the group consisting of: 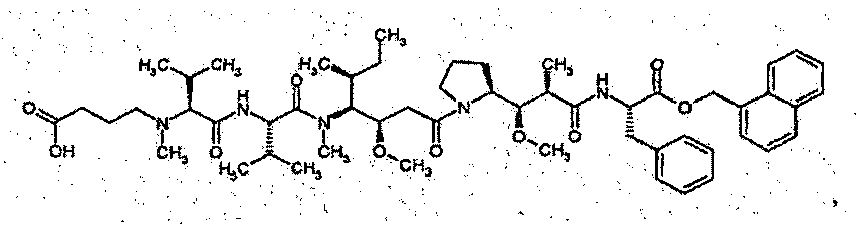 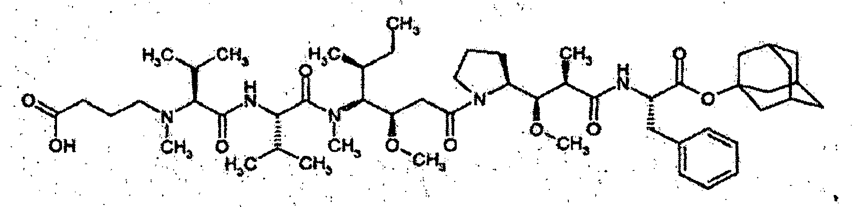 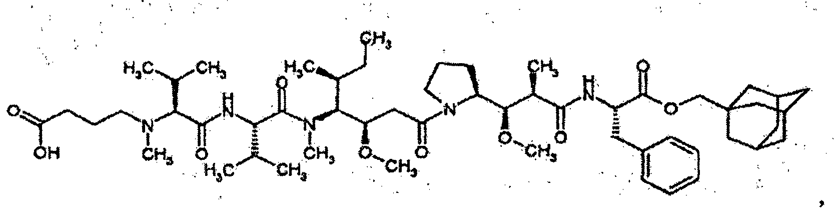 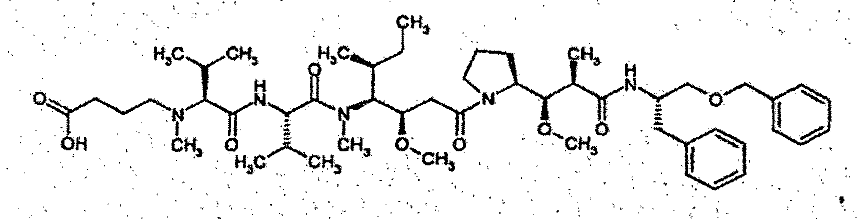 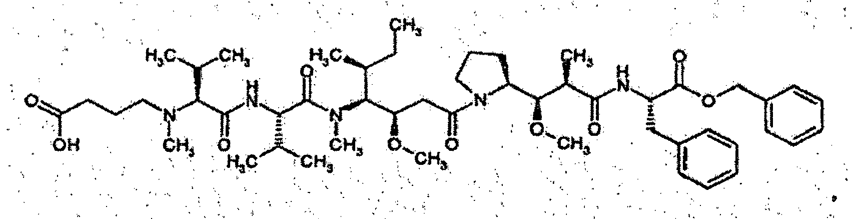 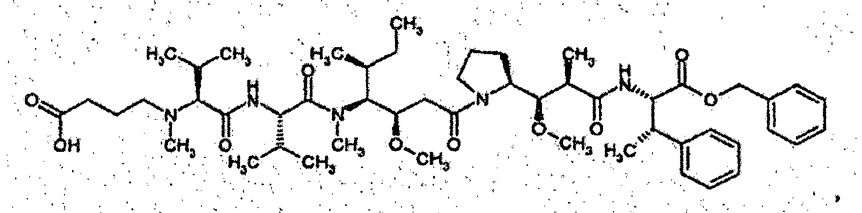 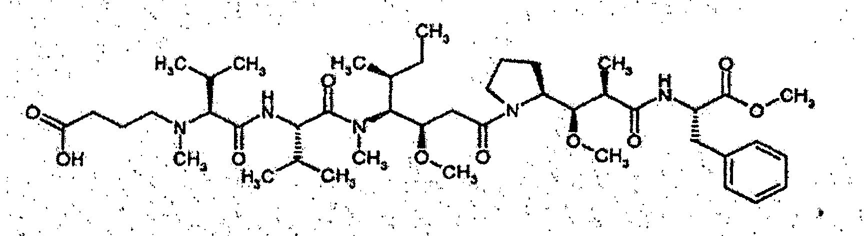 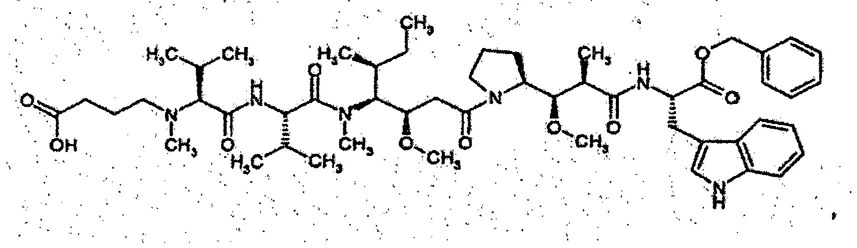 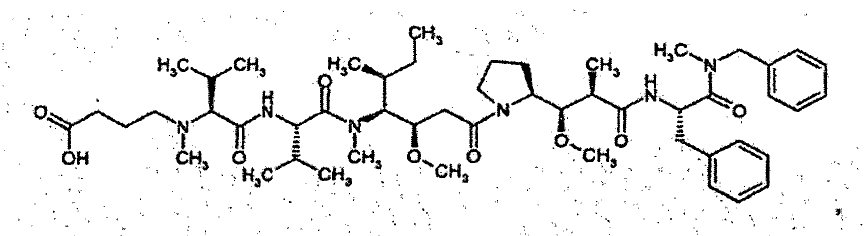 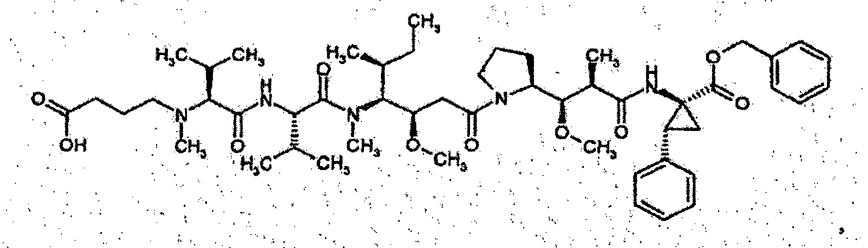 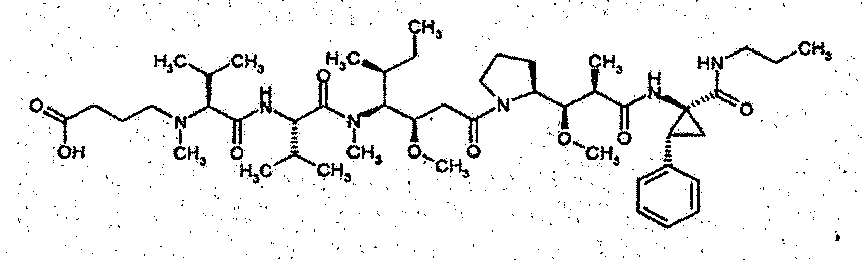 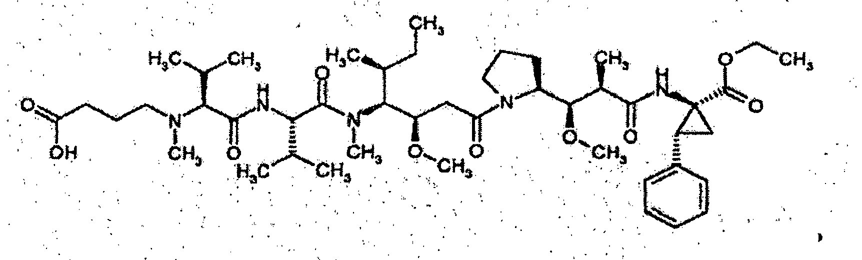 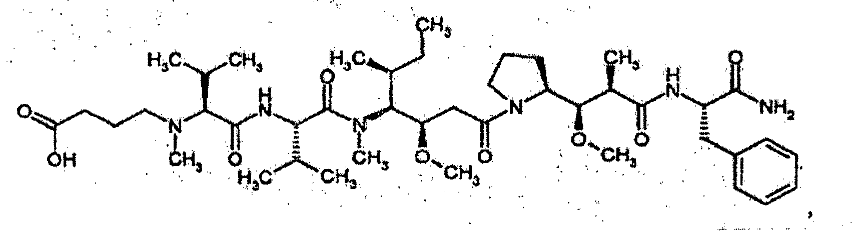 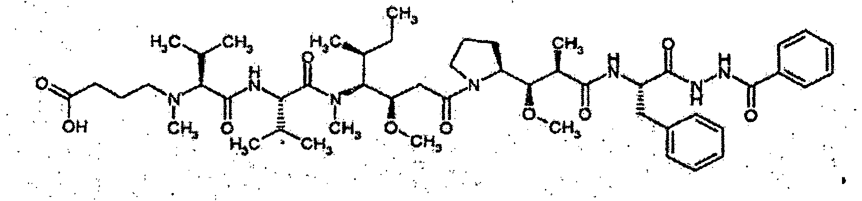 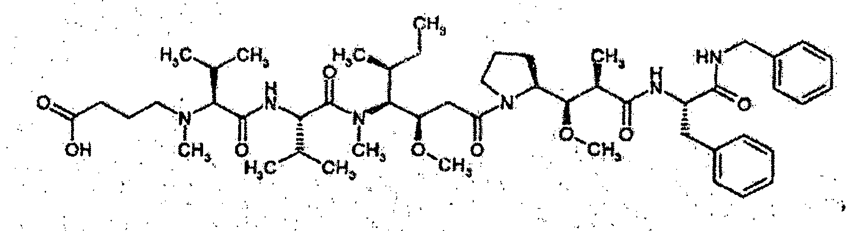 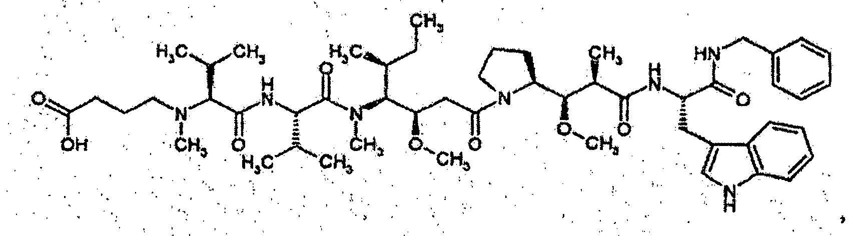 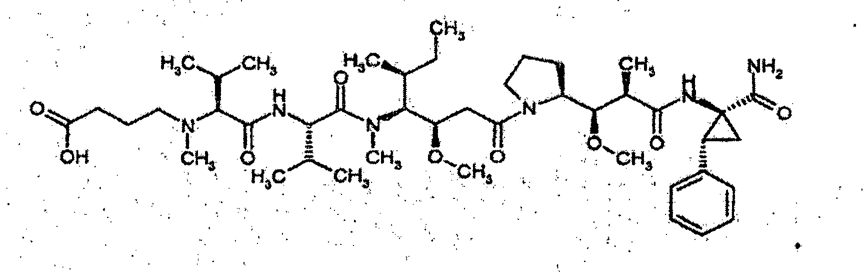 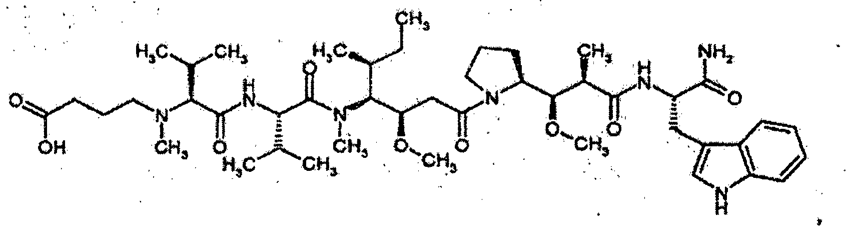 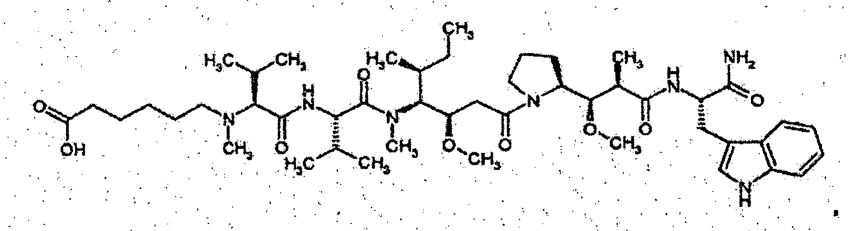 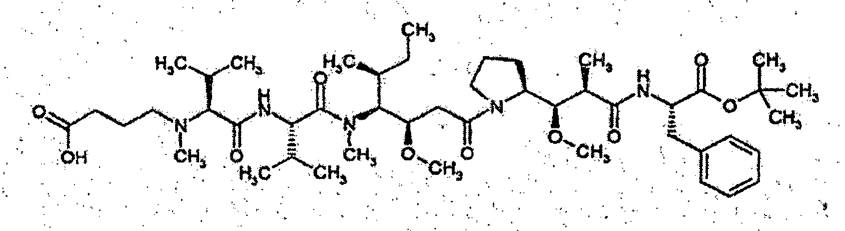 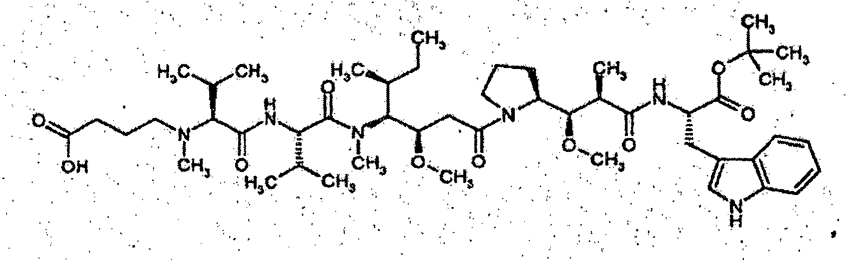 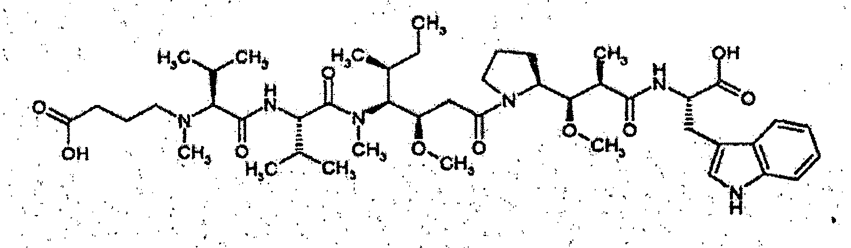 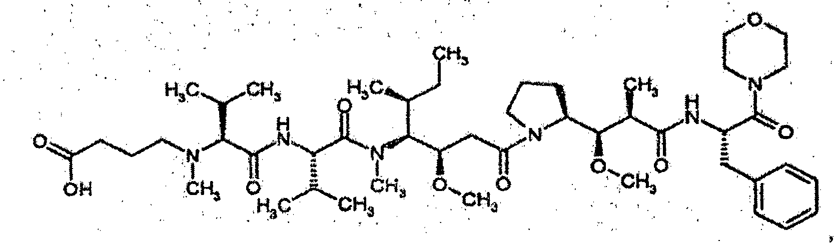 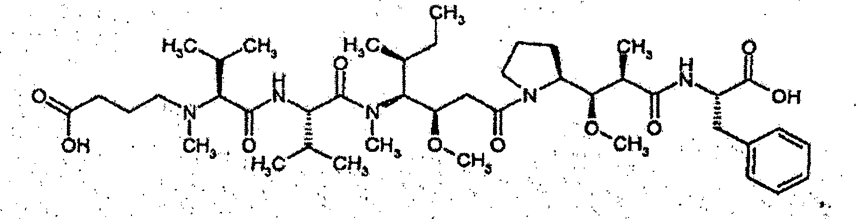 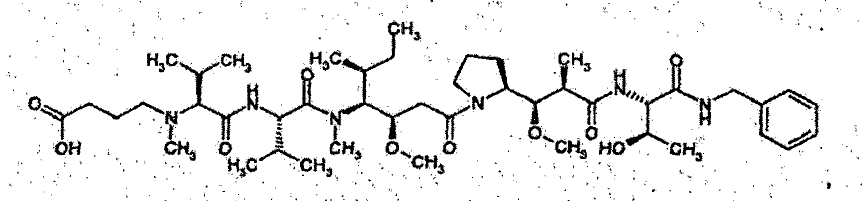 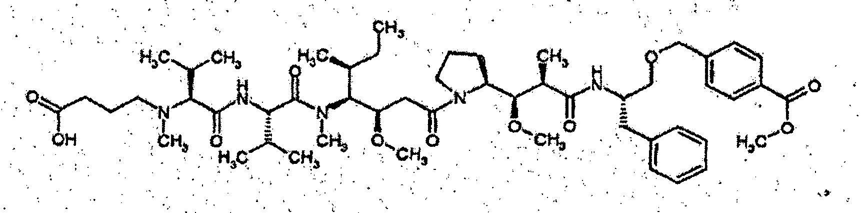 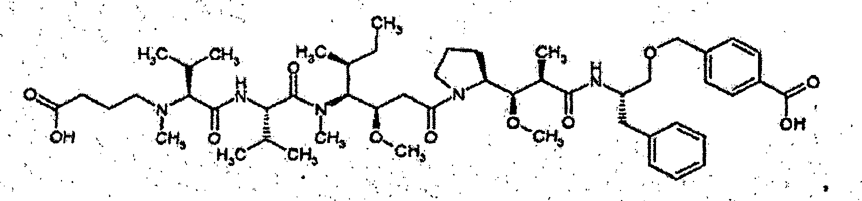 and 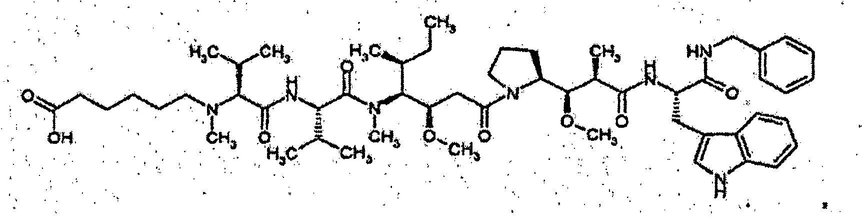 and salts thereof, solvates and solvates of the salts.

6. Method for producing a compound of the formula (I), as defined in claims 1 to 5, **characterised in that** a compound of the formula (II) 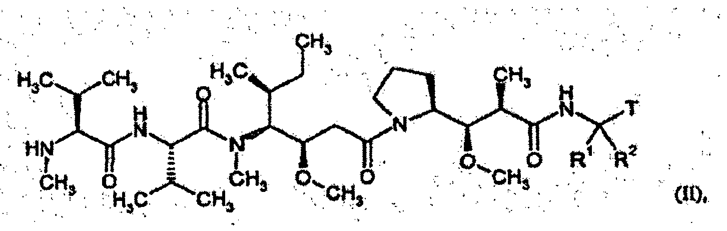 in which R¹, R² and T have the meanings indicated in claims 1 to 4, is reacted in an inert solvent either
[A] by base-induced alkylation with a compound of the formula (III) 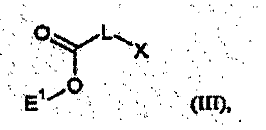 in which L has the meaning indicated in claims 1 to 4,
E¹ represents hydrogen, (C₁-C₄) alkyl or benzyl,
and
X represents a volatile group, such as for example chloride, bromide, iodide, mesylate, triflate or tosylate,
to form a compound of the formula (IV) 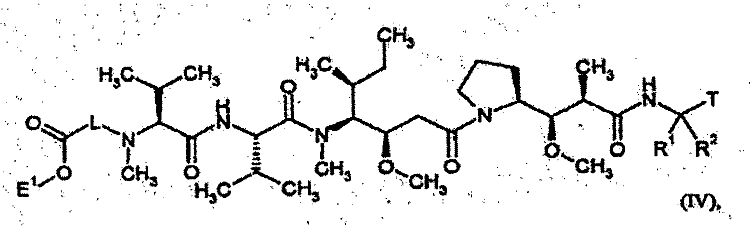 in which E¹, L, R¹, R² and T have the meanings indicated above,
and then in the case that E¹ represents (C₁-C₄) alkyl or benzyl, this ester radical is cleaved according to conventional methods so that just as in the case that E¹ in (III) represents hydrogen, the carboxylic acid of the invention of formula (I) 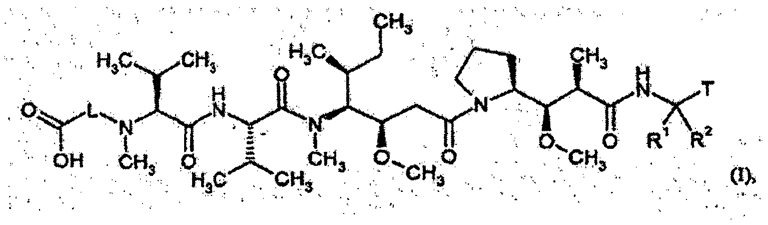 in which L, R¹, R² and T have the meanings indicated above, is obtained
or
[B] is converted by reacting with a compound of the formula (V) 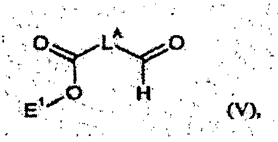 in which
E¹ represents hydrogen, (C₁-C₄) alkyl or benzyl,
and
L^{A} has the meaning of L defined in claims 1 to 4, but is shortened in alkyl chain length by one CH₂ unit,
in the presence of a suitable reducing agent to a compound of the formula (VI) 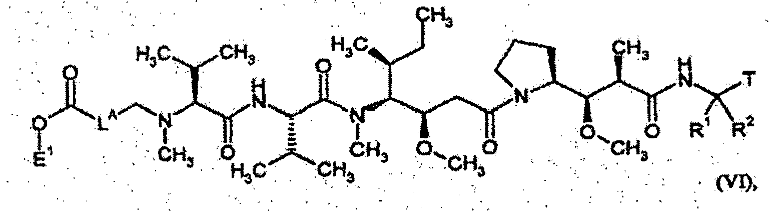 in which E¹, L^{A}, R¹, R² and T have the meanings indicated above,
and then in the case that E¹ represents (C₁-C₄) alkyl or benzyl, this ester radical is cleaved according to conventional methods so that just as in the case that E¹ in (V) represents hydrogen, the carboxylic acid of the invention of formula (I-B) 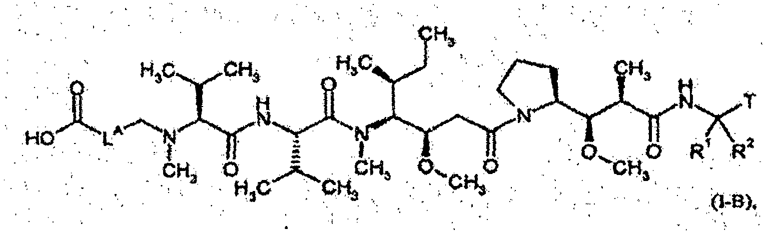 in which L^{A}, R¹, R² and T have the meanings indicated above, is obtained,
and the resulting compounds of formula (I) or (I-B) are separated optionally into their enantiomers and/or diastereomers and/or reacted with the corresponding (i) solvents and/or (*ii*) bases or acids to form their solvates, salts and/or solvates of the salts.

7. Compound, as defined in one of claims 1 to 5, for use in a method for treating and/or preventing diseases.

8. Compound, as defined in one of claims 1 to 5, for use in a method for treating and/or preventing cancer and tumour-related diseases.

9. Use of a compound, as defined in one of claims 1 to 5, for producing a medicament for treating and/or preventing cancer and tumour-related diseases.

10. Medicament containing a compound, as defined in one of claims 1 to 5, in combination with one or more inert, non-toxic, pharmaceutically suitable adjuvants.

11. Medicament containing a compound, as defined in one of claims 1 to 5, in combination with one or more further active ingredients.

12. Medicament according to claim 10 or 11 for use in a method for treating and/or preventing cancer and tumour-related diseases.

13. Antiproliferative conjugate in which a compound according to one or more of claims 1-5 is linked to a protein.

14. Antiproliferative conjugate according to claim 13, wherein the protein is an antibody.

15. Antiproliferative conjugate according to claim 14, in which the compound according to one or more of claims 1-5 is joined to the antibody via the N-terminal carboxyalkyl grouping.

## Revendications

1. Composé de formule (I) 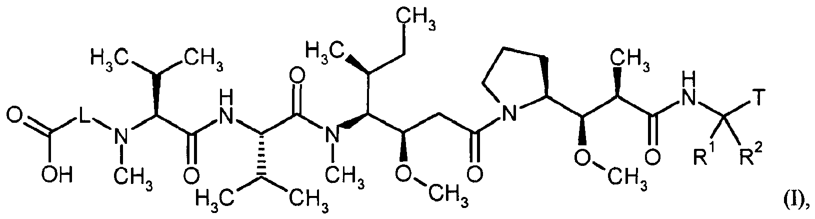 dans laquelle
L représente un alcanediyle (en C₁-C₁₂) linéaire qui peut être substitué jusqu'à 4 fois par le groupe méthyle et dans lequel (a) deux atomes de carbone en position 1,2, 1,3 ou 1,4 les uns par rapport aux autres, en intégrant les atomes de carbone se trouvant le cas échéant entre eux, peuvent être liés à un radical cycloalkyle (en C₃-C₆) ou un radical phényle ou (b) jusqu'à trois groupes CH₂ non adjacents peuvent être remplacés par -O-,
R¹ représente un hydrogène ou un groupe méthyle,
R² représente un groupe isopropyle, isobutyle, *sec*.-butyle, *tert*.-butyle, 1-hydroxyéthyle, phényle, benzyle, 4-hydroxybenzyle, 1-phényléthyle, diphénylméthyle, 1*H*-imidazol-4-ylméthyle ou 1*H*-indol-3-ylméthyle,
ou
R¹ et R² forment, conjointement avec l'atome de carbone auquel ils sont tous deux reliés, un groupe 2-phénylcyclopropane-1,1-diyle de formule 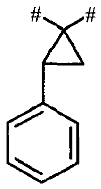 dans laquelle
# caractérise les sites de liaison avec les autres parties de la molécule,
et
T représente un groupe de formules -C(=O)-OR³, -C(=O)-NR⁴R⁵, -C(=O)-NH-NH-R⁶, ou -CH₂-O-R⁷, dans lesquelles
R³ représente un hydrogène, un groupe alkyle (en C₁-C₆) ou cycloalkyle (en C₃-C₁₀),
le groupe alkyle (en C₁-C₆) pouvant être substitué par un groupe phényle, naphtyle ou cycloalkyle (en C₃-C₁₀),
R⁴ représente un hydrogène ou un groupe alkyle (en C₁-C₆),
R⁵ représente un hydrogène, un groupe alkyle (en C₁-C₆) ou cycloalkyle (en C₃-C₁₀),
le groupe alkyle (en C₁-C₆) pouvant être substitué par un groupe phényle,
ou
R⁴ et R⁵ sont liés l'un à l'autre et forment, conjointement avec l'atome d'azote auquel ils sont reliés, un aza-hétérocycle saturé, de 5 à 7 membres, qui peut comporter un autre hétéroatome de cycle de la série >N-H, >N-CH₃, ou -O-, qui se trouve en position 1,3 ou le cas échéant 1,4 par rapport à l'atome d'azote cité en premier,
R⁶ représente un groupe alkyle (en C₁-C₆), alkyl-carbonyle (en C₁-C₆), phényle ou benzoyle,
et
R⁷ représente un groupe alkyle (en C₁-C₆) qui peut être substitué par un groupe phényle,
le groupe phényle pouvant être pour sa part substitué par un groupe alcoxycarbonyle (en C₁-C₆) ou carboxyle,
ainsi que ses sels, solvates et solvates de ses sels.

2. Composé de formule (I) selon la revendication 1, dans laquelle
L représente un alcanediyle (en C₁-C₈) linéaire, dans lequel (a) deux atomes de carbone en position 1,3 ou 1,4 les uns par rapport aux autres, en intégrant l'un et/ou les deux atomes de carbone se trouvant entre eux, peuvent être liés à un radical phényle ou (b) jusqu'à deux groupes CH₂ non adjacents peuvent être remplacés par -O-,
R¹ représente un hydrogène,
R² représente un groupe benzyle, 4-hydroxybenzyle, 1-phényléthyle ou 1*H*-indol-3-ylméthyle,
ou
R¹ et R² forment, conjointement avec l'atome de carbone auquel ils sont tous deux reliés, un groupe 2-phénylcyclopropane-1,1-diyle de formule 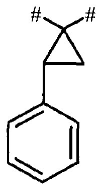 dans laquelle
# caractérise les sites de liaison avec les autres parties de la molécule,
et
T représente un groupe de formules -C(=O)-OR³, -C(=O)-NR⁴R⁵, -C(=O)-NH-NH-R⁶, ou -CH₂-O-R⁷, dans lesquelles
R³ représente un hydrogène ou un groupe alkyle (en C₁-C₄) qui peut être substitué par un groupe phényle, naphtyle ou cycloalkyle (en C₃-C₁₀),
R⁴ représente un hydrogène ou un groupe méthyle,
R⁵ représente un hydrogène ou un groupe alkyle (en C₁-C₄), qui peut être substitué par un groupe phényle,
ou
R⁴ et R⁵ sont liés l'un à l'autre et forment, conjointement avec l'atome d'azote auquel ils sont reliés, un radical pipéridine ou morpholine,
R⁶ représente un groupe alkylcarbonyle (en C₁-C₄) ou benzoyle,
et
R⁷ représente un groupe alkyle (en C₁-C₄) ou benzyle, qui peut être substitué dans le groupe phényle par un groupe alcoxycarbonyle (en C₁-C₄) ou carboxyle,
ainsi que ses sels, solvates et solvates de ses sels.

3. Composé de formule (I) selon la revendication 1 ou 2, dans laquelle
L représente un alcanediyle (en C₁-C₆) linéaire,
R¹ représente un hydrogène,
R² représente un groupe benzyle, 1-phényléthyle ou 1H-indol-3-ylméthyle,
ou
R¹ et R² forment, conjointement avec l'atome de carbone auquel ils sont tous deux reliés, un groupe (1*S*,2*R*)-2-phénylcyclopropane-1,1-diyle de formule 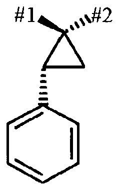 dans laquelle
#1 caractérise le site de liaison avec l'atome d'azote adjacent,
et et
#2 caractérise le site de liaison avec le groupe T,
T représente un groupe de formules -C(=O)-OR³, -C(=O)-NR⁴R⁵, -C(=O)-NH-NH-R⁶, ou -CH₂-O-R⁷, dans lesquelles
R³ représente un hydrogène, un groupe méthyle, éthyle, *n*-propyle, benzyle ou adamantylméthyle,
R⁴ représente un hydrogène ou un groupe méthyle,
R⁵ représente un hydrogène, un groupe méthyle, éthyle, *n*-propyle ou benzyle,
R⁶ représente un groupe benzoyle,
et
R⁷ représente un groupe benzyle, qui peut être substitué dans le groupe phényle par un groupe méthoxycarbonyle ou carboxyle,
ainsi que ses sels, solvates et solvates de ses sels.

4. Composé selon la revendication 1, 2 ou 3, ayant pour formule (I-A) 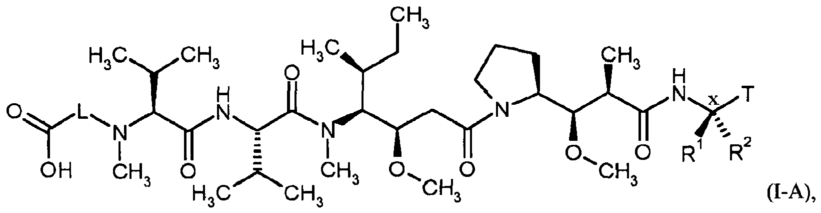 dans laquelle L, R¹, R² et T ont les significations indiquées dans la revendication 1, 2 ou 3 et l'atome de carbone C^{x} portant les résidus R¹ et R² présente la configuration absolue reproduite,
ainsi que ses sels, solvates et solvates de ses sels.

5. Composé selon la revendication 1, ledit composé étant choisi dans le groupe constitué par: 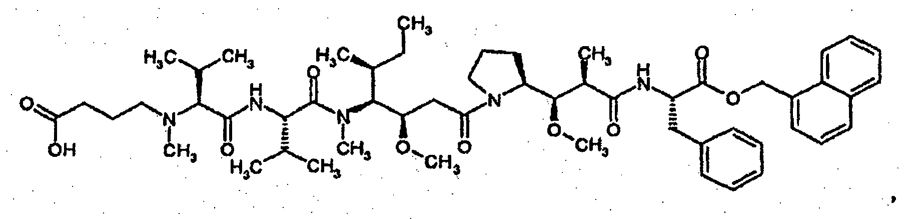 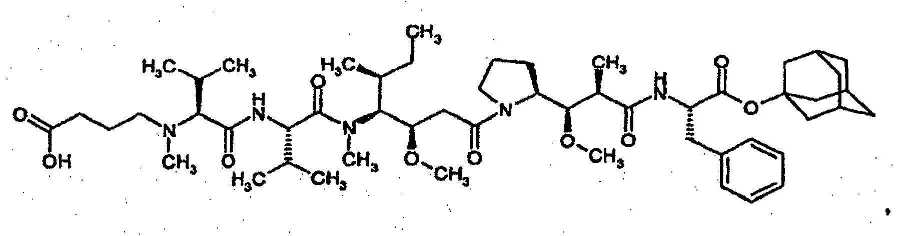 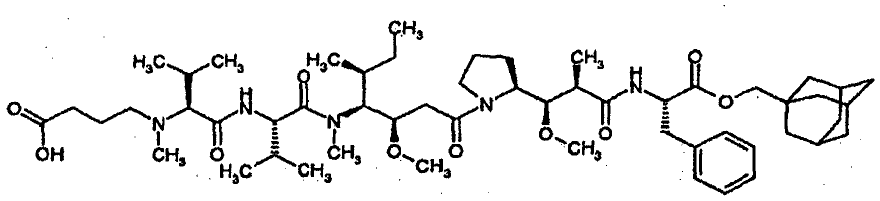 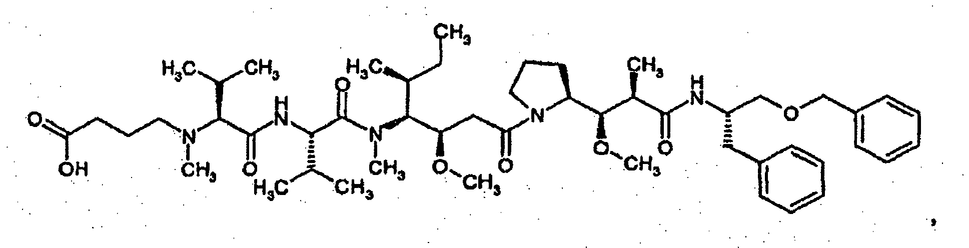 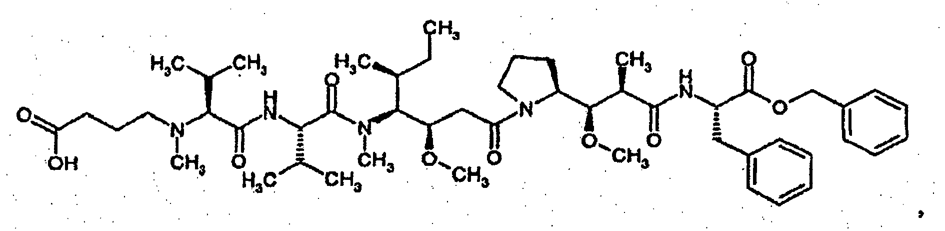 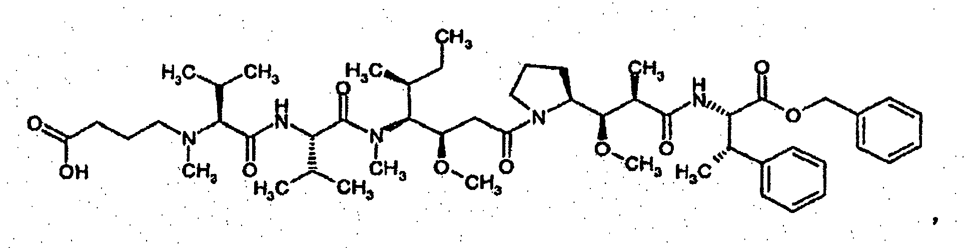 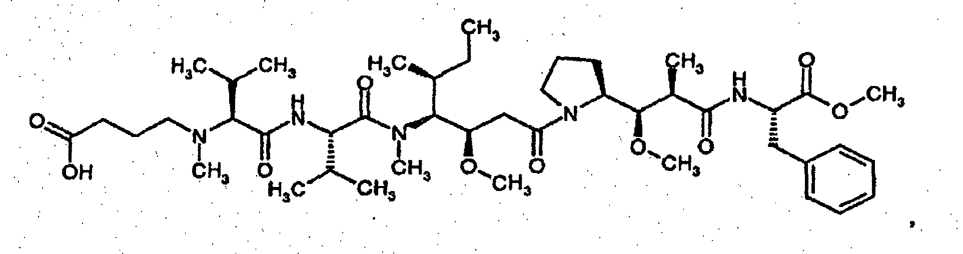 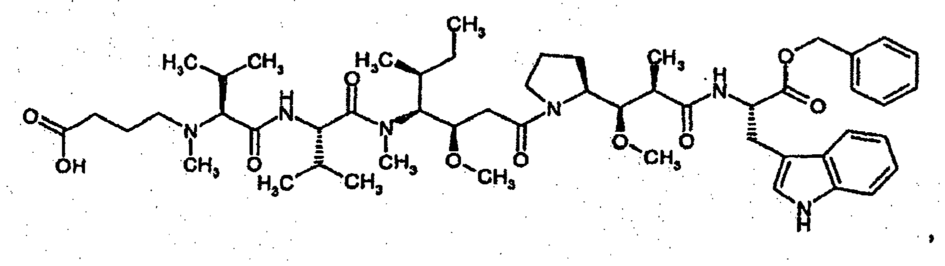 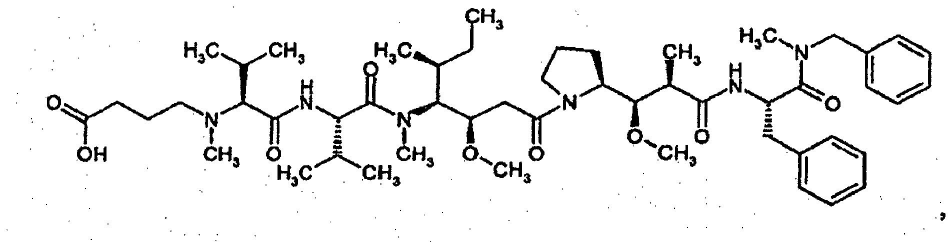 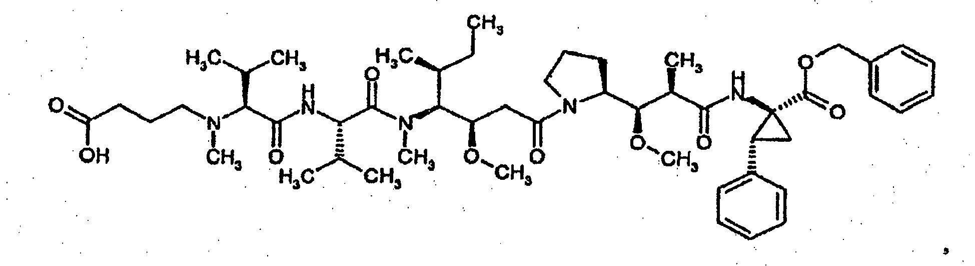 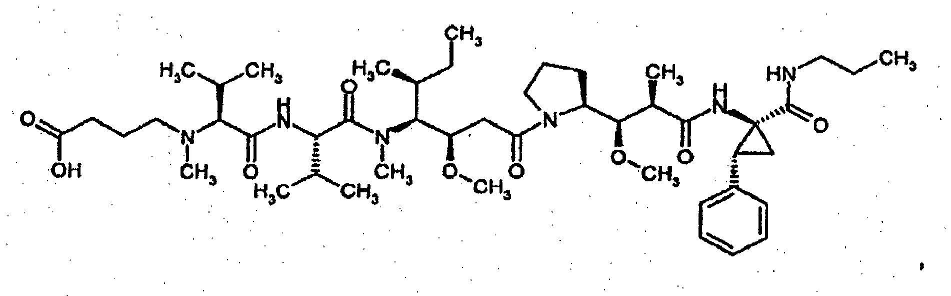 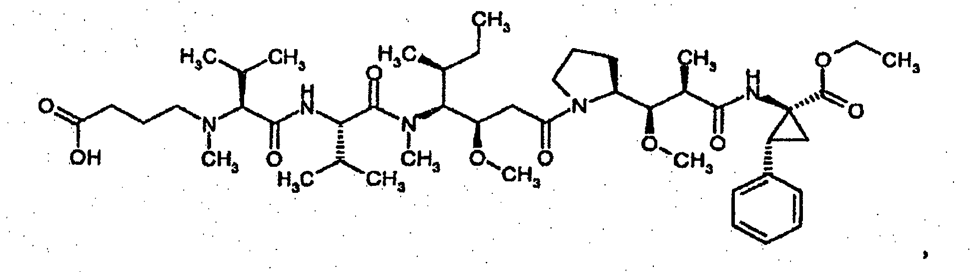 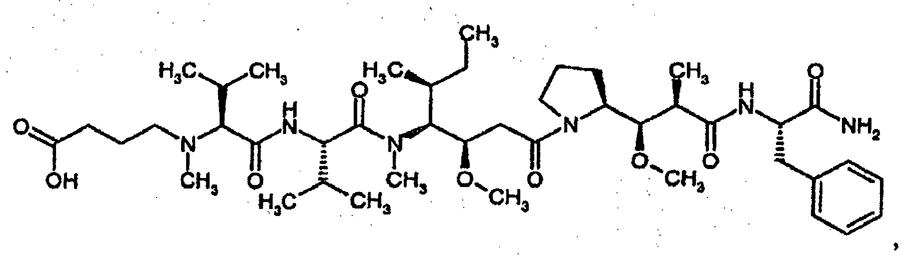 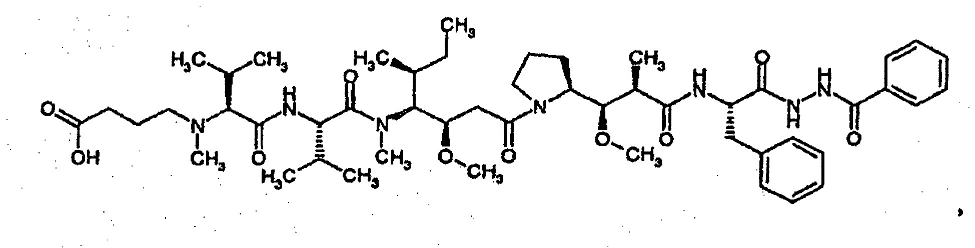 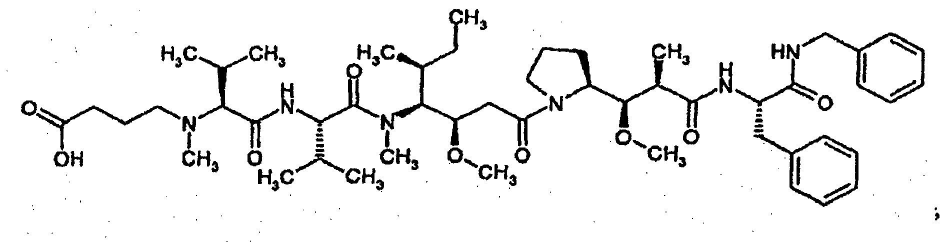 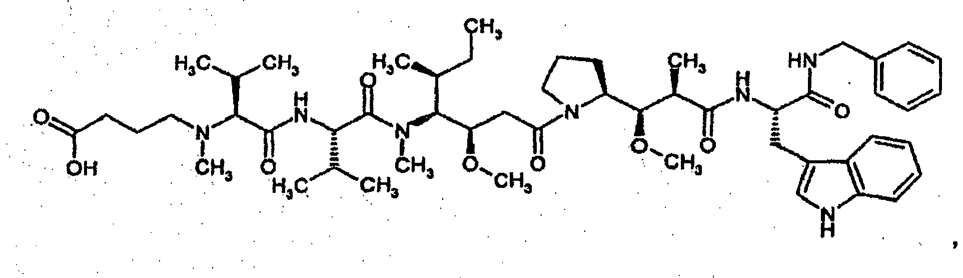 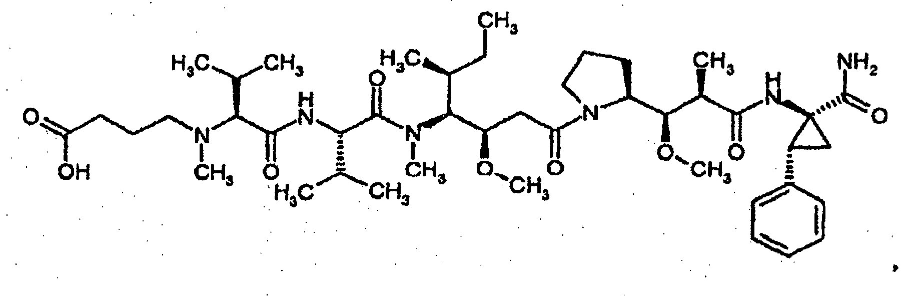 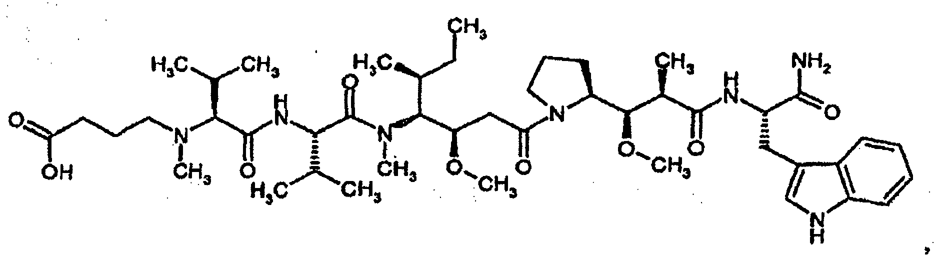 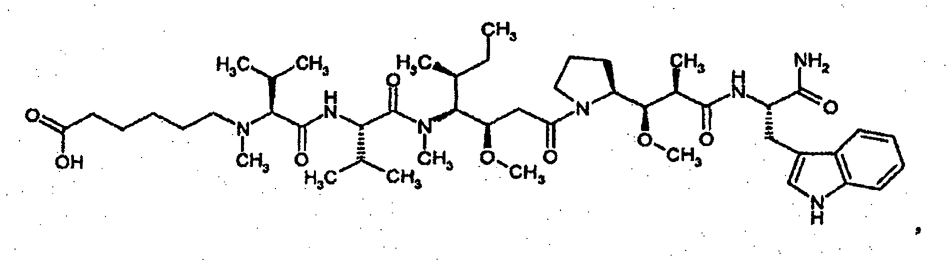 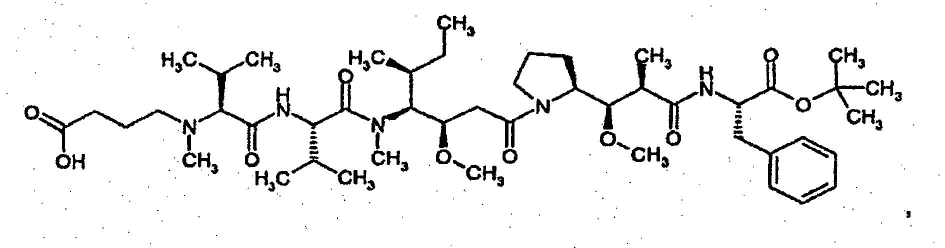 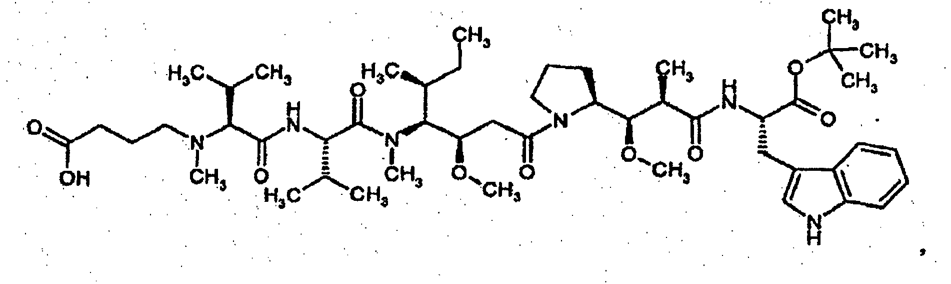 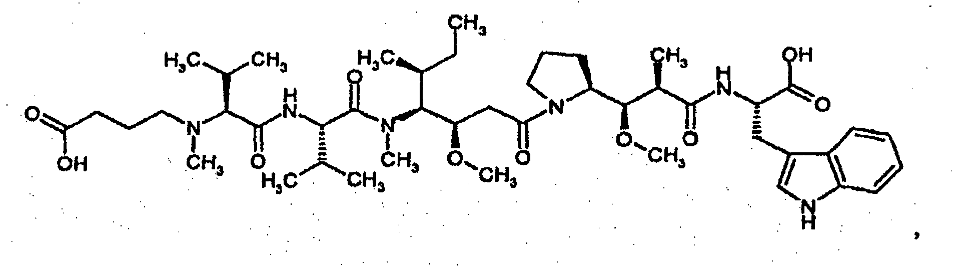 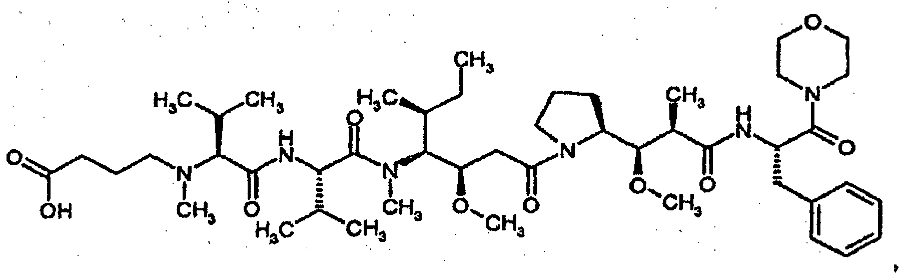 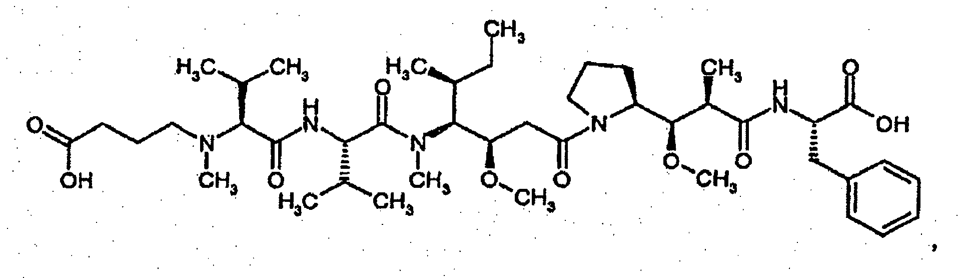 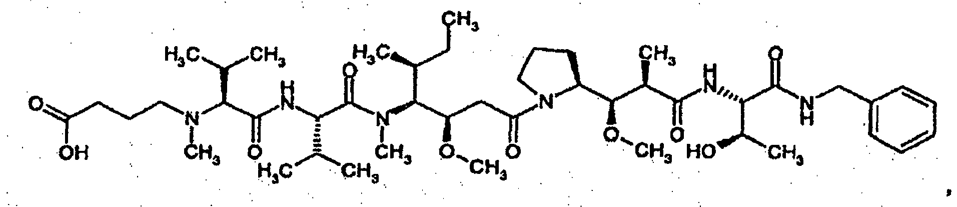 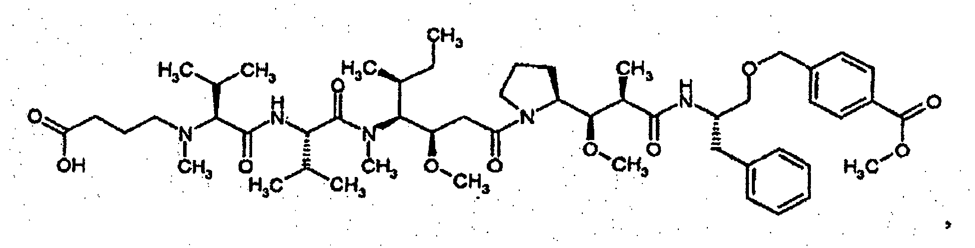 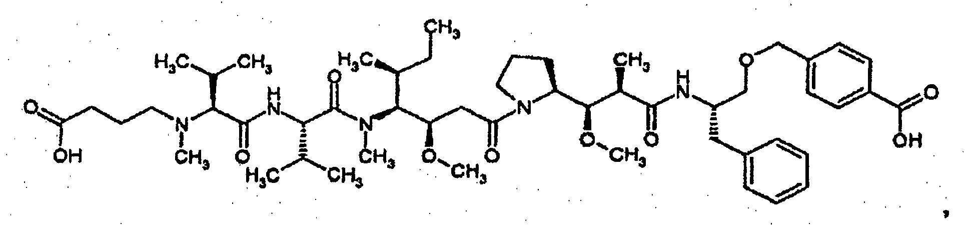 et 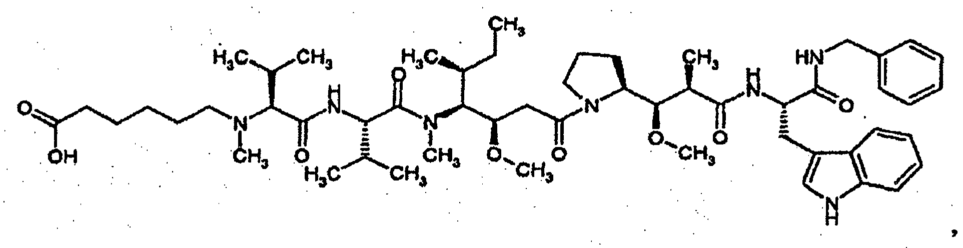 ainsi que leurs sels, solvates et solvates de leurs sels.

6. Procédé de préparation d'un composé de formule (I), tel que défini dans les revendications 1 à 5, **caractérisé en ce que** l'on fait réagir un composé de formule (II) 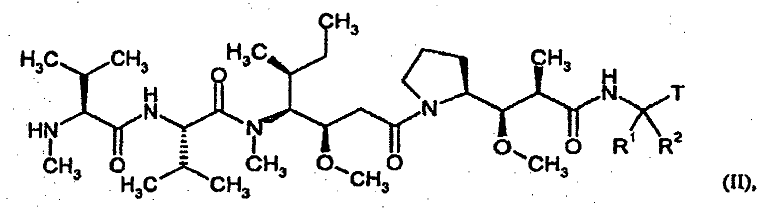 dans laquelle R¹, R² et T ont les significations indiquées dans les revendications 1 à 4,
dans un solvant inerte soit
[A] par alkylation induite par des bases avec un composé de formule (III) 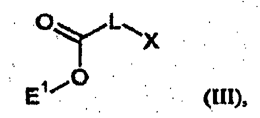 dans laquelle L a les significations indiquées dans les revendications 1 à 4,
E¹ représente un hydrogène, un groupe alkyle (en C₁-C₄) ou benzyle,
et
X représente un groupe sortant, par exemple chlorure, bromure, iodure, mésylate, triflate ou tosylate,
pour donner un composé de formule (IV) 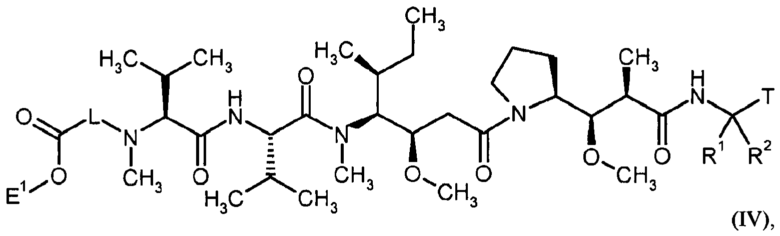 dans laquelle E¹, L, R¹, R² et T ont les significations indiquées ci-dessus,
et ensuite, dans le cas où E¹ représente un groupe alkyle (en C₁-C₄) ou benzyle, on clive ce résidu ester d'après des méthodes usuelles de manière à obtenir, tout comme dans le cas où E¹ représente un hydrogène dans la formule (III), l'acide carboxylique selon l'invention de formule (I) 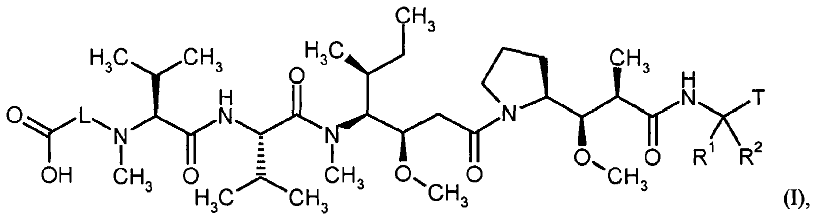 dans laquelle L, R¹, R² et T ont les significations indiquées ci-dessus,
soit
[B] par conversion avec un composé de formule (V) 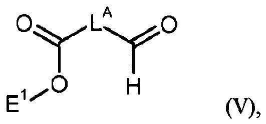 dans laquelle
E¹ représente un hydrogène, un groupe alkyle (en C₁-C₄) ou benzyle,
et
L^{A} a la signification de L définie dans les revendications 1 à 4, mais est cependant raccourci d'une unité CH₂ dans la longueur de chaîne alkyle,
en présence d'un agent réducteur approprié pour donner un composé de formule (VI) 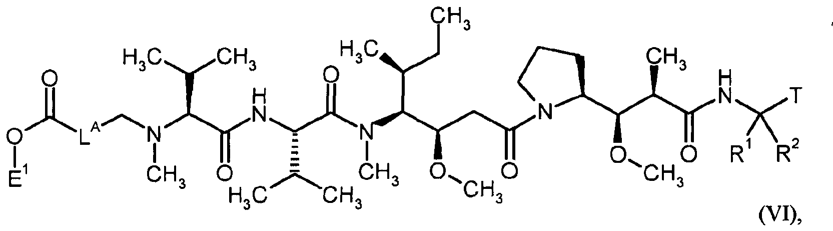 dans laquelle E¹, L^{A}, R¹, R² et T ont les significations indiquées ci-dessus,
et ensuite, dans le cas où E¹ représente un groupe alkyle (en C₁-C₄) ou benzyle, on clive ce résidu ester d'après des méthodes usuelles de manière à obtenir, tout comme dans le cas où E¹ représente un hydrogène dans la formule (V), l'acide carboxylique selon l'invention de formule (I-B) 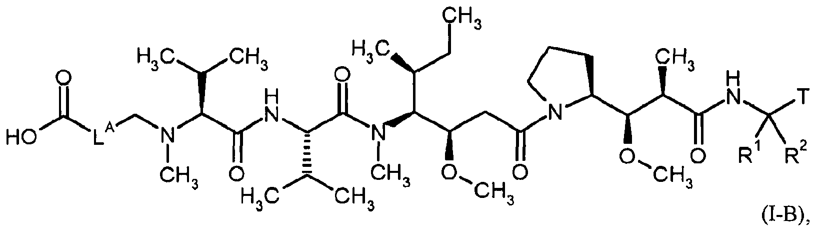 dans laquelle L^{A}, R¹, R² et T ont les significations indiquées ci-dessus,
et on sépare les composés résultants de formule (I) ou (I-B) le cas échéant en leurs énantiomères et/ou diastéréomères et/ou on les convertit avec les (i) solvants et/ou (ii) bases ou acides correspondants en leurs solvates, sels et/ou solvates de leurs sels.

7. Composé tel que défini dans l'une des revendications 1 à 5, pour l'utilisation dans un procédé destiné au traitement et/ou à la prévention des maladies.

8. Composé tel que défini dans l'une des revendications 1 à 5, pour l'utilisation dans un procédé destiné au traitement et/ou à la prévention des affections cancéreuses et tumorales.

9. Utilisation d'un composé tel que défini dans l'une des revendications 1 à 5, pour la préparation d'un médicament destiné au traitement et/ou à la prévention des affections cancéreuses et tumorales.

10. Médicament contenant un composé tel que défini dans l'une des revendications 1 à 5, en combinaison avec un ou plusieurs auxiliaires inertes non toxiques pharmaceutiquement acceptables.

11. Médicament contenant un composé tel que défini dans l'une des revendications 1 à 5, en combinaison avec une ou plusieurs autres substances actives.

12. Médicament selon la revendication 10 ou 11 pour l'utilisation dans un procédé destiné au traitement et/ou à la prévention des affections cancéreuses et tumorales.

13. Conjugué ayant une action antiproliférative, dans lequel le composé selon l'une ou plusieurs des revendications 1 à 5 est lié à une protéine.

14. Conjugué ayant une action antiproliférative selon la revendication 13, dans lequel la protéine est un anticorps.

15. Conjugué ayant une action antiproliférative selon la revendication 14, dans lequel le composé selon l'une ou plusieurs des revendications 1 à 5 est lié à l'anticorps par le biais du groupement carboxyalkyle N-terminal.
